# EUROPÄISCHE PATENTSCHRIFT

(11) **EP 1 049 666 B1**
(45) Veröffentlichungstag und Bekanntmachung des Hinweises auf die Patenterteilung: **04.06.2003**
(21) Anmeldenummer: 99903617.1
(22) Anmeldetag: 09.01.1999
(51) Int. Cl.: C07C 311/44, C07C 311/46, C07D 233/26, C07D 319/06, A61K 31/18

(54) **NEUE NAPHTHYL- UND ANILID-SUBSTITUIERTE SULFONAMIDE**
NOVEL NAPHTHYL-SUBSTITUTED AND ANILIDE-SUBSTITUTED SULFONAMIDES
NOUVEAUX SULFAMIDES A SUBSTITUTION PAR NAPHTYLE ET ANILIDE

(30) Priorität: 23.01.1998 DE 19802439
(43) Veröffentlichungstag der Anmeldung: 08.11.2000
(73) Patentinhaber: Bayer Aktiengesellschaft, 51368 Leverkusen (DE)
(72) Erfinder: BENDER, Wolfgang, D-42113 Wuppertal (DE); REEFSCHLÄGER, Jürgen, D-42111 Wuppertal (DE); ECKENBERG, Peter, D-42115 Wuppertal (DE); GOLDMANN, Siegfried, D-42327 Wuppertal (DE); HÄRTER, Michael, D-42489 Wülfrath (DE); HALLENBERGER, Sabine, D-42109 Wuppertal (DE); TRAPPE, Jörg, D-42115 Wuppertal (DE); WEBER, Olaf, D-42489 Wülfrath (DE)
(86) Internationale Anmeldenummer: EP9900099
(87) Internationale Veröffentlichungsnummer: WO99037609

(56) Entgegenhaltungen:
- WO-A-90/09787
- DE-A- 4 331 134
- S.-W. JIN, ET AL.: "A new sensitive Edman-type reagent: 4-(N-1-dimethylamino- naphthalene-5-sulphonylamino)phenyl isothiocyanate" FEBS LETTERS, Bd. 198, Nr. 1, 17. März 1986, Seiten 150-154, XP002104472 Amsterdam, NL

## Beschreibung

Die vorliegende Erfindung betrifft neue Naphthyl- und Anilid-substituierte Sulfonamide, Verfahren zu ihrer Herstellung und ihre Verwendung als antivirale Mittel, insbesondere gegen Cytomegalieviren.

α,β-Naphthyl verknüpfte Phenylsulfonamine sind überwiegend aus phototechnischen Publikationen bekannt [vgl. hierzu JP-06 122 669-A2, EP-684 515-A1; JP-59 174 836-A2, DE-2 902 074, US-3 925 347, US-4 035 401, US-3 622 603, US-3 482 971, EP-284 130, DE-4331134].

FEBS LETTERS, Bd. 198, Nr. 1 offenbart 4-(N-1-dimethylamino-naphtalene-5-sulphonylamino)phenyl isothiocyanate als Edman-type reagent.

Die WO 90/09 787 offenbart Sulfonamide als Radio- oder Chemosensibilisierungsmittel und ihre Verwendung bei der Behandlung von Tumoren.

Außerdem ist die Verbindung N-[4-[[[5-(Dimethylamino)-1-naphthalenyl]sulfonyl]-amino]phenyl]-acetamid bekannt (J. Inst. Chem. (India) (1976), 48, Pt 6, 280-5).

Die vorliegende Erfindung betrifft jetzt neue Naphthyl- und Anilid-substituierte Sulfonamide der allgemeinen Formel (I), 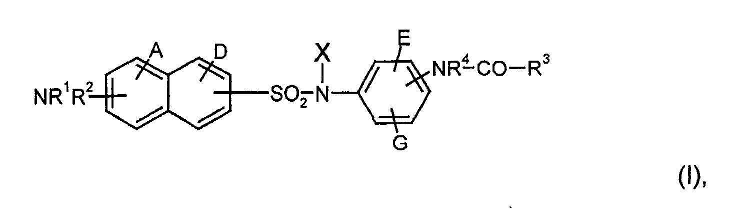 in welcher
- R¹ und R²: gleich oder verschieden sind und für Wasserstoff, Formyl, gegebenenfalls durch ein bis drei Halogenatomen substituiertes Phenyl oder Benzyl, oder geradkettiges oder verzweigtes Alkyl oder Acyl mit jeweils bis zu 6 Kohlenstoffatomen stehen, wobei Alkyl oder Acyl gegebenenfalls durch ein bis drei Substituenten ausgewählt aus Halogen und Hydroxy substituiert sein können,
- A, D, E und G: gleich oder verschieden sind und für Wasserstoff, Halogen, Nitro, Cyano, Hydroxy, Carboxyl, Trifluormethyl, Trifluormethoxy oder für geradkettiges oder verzweigtes Alkyl, Alkoxy oder Alkoxycarbonyl mit jeweils bis zu 5 Kohlenstoffatomen stehen,
- R³: für geradkettiges oder verzweigtes Alkenyl mit bis zu 6 Kohlenstoffatomen steht, oder
für geradkettiges oder verzweigtes Alkyl mit bis zu 8 Kohlenstoffatomen steht, das gegebenenfalls eine Aminogruppe trägt, die durch Alkyl mit bis zu 4 Kohlenstoffatomen oder durch eine Aminoschutzgruppe substituiert sein kann, oder das Alkyl gegebenenfalls ein- bis 3-fach gleich oder verschieden durch Hydroxy, Cyano, Halogen, Azido, Nitro, Trifluormethyl, Carboxyl oder Phenyl substituiert ist, das seinerseits bis zu 2-fach, gleich oder verschieden durch Nitro, Halogen, Hydroxy oder durch geradkettiges oder verzweigtes Alkyl oder Alkoxy mit bis zu 4 Kohlenstoffatomen substituiert sein kann, oder
- R³: für Reste der Formeln 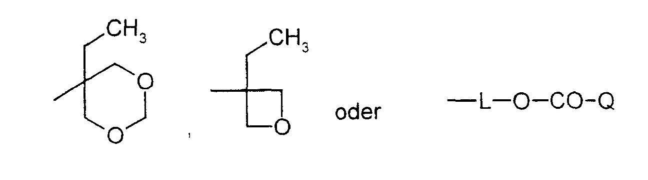 steht,
worin
- L: für eine geradkettige oder verzweigte Alkandiylgruppe mit bis zu 6 Kohlenstoffatomen steht,
- Q: für Alkyl mit bis zu 6 Kohlenstoffatomen steht, das gegebenenfalls durch Carboxyl substituiert ist, oder für Reste der Formeln 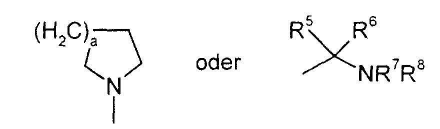 steht,
worin
a die Zahl 1 oder 2 bedeutet,
R⁵ Wasserstoff bedeutet,
R⁶ Cycloalkyl mit 3 bis 8 Kohlenstoffatomen oder Aryl mit 6 bis 10 Kohlenstoffatomen oder Wasserstoff bedeutet, oder geradkettiges oder verzweigtes Alkyl mit bis zu 8 Kohlenstoffatomen bedeutet,
wobei das Alkyl gegebenenfalls durch Cyano, Methylthio, Hydroxy, Mercapto, Guanidyl oder durch eine Gruppe der Formel -NR⁹R¹⁰ oder R¹¹-OC- substituiert ist,
worin
R⁹ und R¹⁰ unabhängig voneinander Wasserstoff, geradkettiges oder verzweigtes Alkyl mit bis zu 8 Kohlenstoffatomen oder Phenyl bedeuten,
und
R¹¹ Hydroxy, Benzyloxy, Alkoxy mit bis zu 6 Kohlenstoffatomen oder die oben aufgeführte Gruppe -NR⁹R¹⁰ bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe -NR⁹R¹⁰ substituiert ist.
worin
R⁹ und R¹⁰ die oben angegebene Bedeutung haben,
oder das Alkyl gegebenenfalls durch einen 5- bis 6-gliedrigen stickstoffhaltigen Heterocyclus oder durch Indolyl substituiert ist, worin die entsprechenden -NH-Funktionen gegebenenfalls durch Alkyl mit bis zu 6 Kohlenstoffatomen substituiert oder durch eine Aminoschutzgruppe geschützt sind,
R⁷ und R⁸ gleich oder verschieden sind und Wasserstoff oder eine Aminoschutzgruppe bedeuten,
- R⁴: für Wasserstoff oder für einen Rest der Formel 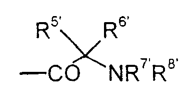 steht,
worin
R^{5'}, R^{6'}, R^{7'} und R^{8'} die oben angegebene Bedeutung von R⁵, R⁶, R⁷ und R⁸ haben und mit dieser gleich oder verschieden sind,
- X: die oben angegebene Bedeutung von R⁴ besitzt und von dieser Bedeutung gleich oder verschieden sein kann,
und deren Stereoisomere, stereoisomeren Gemische und Salze,
mit Ausnahme von N-[4-[[[5-(Dimethylamino)-1-naphthalenyl]sulfonyl]amino]phenyl]-acetamid.

In einer bevorzugten Ausführungsform betrifft die Erfindung Sulfonamide der obigen allgemeinen Formel (I), worin
- R¹ und R²: gleich oder verschieden sind und für Wasserstoff, Phenyl oder geradkettiges oder verzweigtes Alkyl oder Acyl mit jeweils bis zu 6 Kohlenstoffatomen stehen,
- A, D, E und G: gleich oder verschieden sind und für Wasserstoff, Halogen, Nitro, Cyano, Hydroxy, Carboxyl, Trifluormethyl, Trifluormethoxy oder für geradkettiges oder verzweigtes Alkyl, Alkoxy oder Alkoxycarbonyl mit jeweils bis zu 5 Kohlenstoffatomen stehen,
- R³: für geradkettiges oder verzweigtes Alkenyl mit bis zu 6 Kohlenstoffatomen steht, oder
für geradkettiges oder verzweigtes Alkyl mit bis zu 8 Kohlenstoffatomen steht, das gegebenenfalls eine Aminogruppe trägt, die durch Alkyl mit bis zu 4 Kohlenstoffatomen oder durch eine Aminoschutzgruppe substituiert sein kann, oder das Alkyl gegebenenfalls ein- bis 3-fach gleich oder verschieden durch Hydroxy, Cyano, Halogen, Azido, Nitro, Trifluormethyl, Carboxyl oder Phenyl substituiert ist, das seinerseits bis zu 2-fach, gleich oder verschieden durch Nitro, Halogen, Hydroxy oder durch geradkettiges oder verzweigtes Alkyl oder Alkoxy mit bis zu 4 Kohlenstoffatomen substituiert sein kann, oder
- R³: für Reste der Formeln 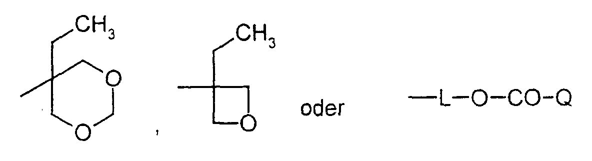 steht,
worin
L für eine geradkettige oder verzweigte Alkandiylgruppe mit bis zu 6 Kohlenstoffatomen steht,
Q für Alkyl mit bis zu 6 Kohlenstoffatomen steht, das gegebenenfalls durch Carboxyl substituiert ist, oder für Reste der Formeln 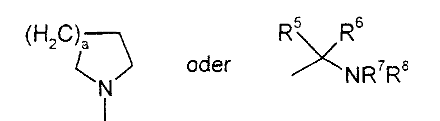 steht,
worin
a die Zahl 1 oder 2 bedeutet,
R⁵ Wasserstoff bedeutet,
R⁶ Cycloalkyl mit 3 bis 8 Kohlenstoffatomen oder Aryl mit 6 bis 10 Kohlenstoffatomen oder Wasserstoff bedeutet, oder geradkettiges oder verzweigtes Alkyl mit bis zu 8 Kohlenstoffatomen bedeutet,
wobei das Alkyl gegebenenfalls durch Cyano, Methylthio, Hydroxy, Mercapto, Guanidyl oder durch eine Gruppe der Formel -NR⁹R¹⁰ oder R¹¹-OC- substituiert ist,
worin
R⁹ und R¹⁰ unabhängig voneinander Wasserstoff, geradkettiges oder verzweigtes Alkyl mit bis zu 8 Kohlenstoffatomen oder Phenyl bedeuten,
und
R¹¹ Hydroxy, Benzyloxy, Alkoxy mit bis zu 6 Kohlenstoffatomen oder die oben aufgeführte Gruppe -NR⁹R¹⁰ bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe - NR⁹R¹⁰ substituiert ist,
worin
R⁹ und R¹⁰ die oben angegebene Bedeutung haben,
oder das Alkyl gegebenenfalls durch einen 5- bis 6-gliedrigen stickstoffhaltigen Heterocyclus oder durch Indolyl substituiert ist, worin die entsprechenden -NH-Funktionen gegebenenfalls durch Alkyl mit bis zu 6 Kohlenstoffatomen substituiert oder durch eine Aminoschutzgruppe geschützt sind,
R⁷ und R⁸ gleich oder verschieden sind und Wasserstoff oder eine Aminoschutzgruppe bedeuten,
- R⁴: für Wasserstoff oder für einen Rest der Formel 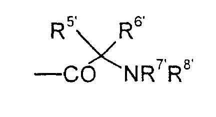 steht,
worin
R^{5'}, R^{6'}, R^{7'} und R^{8'} die oben angegebene Bedeutung von R⁵, R⁶, R⁷ und R⁸ haben und mit dieser gleich oder verschieden sind, und
- X: für Wasserstoff steht,
und deren Stereoisomere, stereoisomeren Gemische und Salze,
mit Ausnahme von N-[4-[[[5-(Dimethylamino)-1-naphthalenyl]sulfonyl]amino]phenyl]-acetamid.

Weitere bevorzugte Verbindungen sind Sulfonamide der obigen allgemeinen Formel (I), worin
- R³: für geradkettiges oder verzweigtes Alkenyl mit bis zu 6 Kohlenstoffatomen steht, oder
für geradkettiges oder verzweigtes Alkyl mit bis zu 8 Kohlenstoffatomen steht, worin das Alkyl eine Aminogruppe trägt, die durch Alkyl mit bis zu 4 Kohlenstoffatomen oder durch eine Aminoschutzgruppe substituiert sein kann, oder das Alkyl ein- bis 3-fach gleich oder verschieden durch Hydroxy, Cyano, Halogen, Azido, Nitro, Trifluormethyl, Carboxyl oder Phenyl substituiert ist, das seinerseits bis zu 2-fach, gleich oder verschieden durch Nitro, Halogen, Hydroxy oder durch geradkettiges oder verzweigtes Alkyl oder Alkoxy mit bis zu 4 Kohlenstoffatomen substituiert sein kann, oder
- R³: für Reste der Formeln 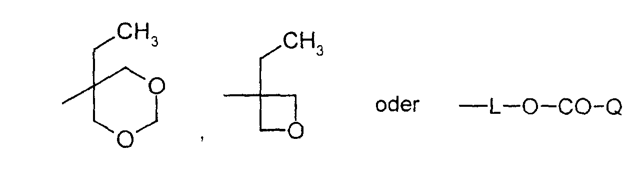 steht,
worin L und Q wie oben definiert sind, und X bevorzugt für Wasserstoff steht.

Weitere bevorzugte Verbindungen der Erfindung sind Sulfonamide der obigen allgemeinen Formel (I), worin
- R¹ und R²: gleich oder verschieden sind und für Wasserstoff, Phenyl oder geradkettiges oder verzweigtes Alkyl oder Acyl mit jeweils bis zu 5 Kohlenstoffatomen stehen,
- A, D, E und G: gleich oder verschieden sind und für Wasserstoff, Fluor, Chlor, Brom, Nitro, Cyano, Hydroxy oder für geradkettiges oder verzweigtes Alkyl, Alkoxy oder Alkoxycarbonyl mit jeweils bis zu 3 Kohlenstoffatomen stehen,
- R³: für geradkettiges oder verzweigtes Alkenyl mit bis zu 5 Kohlenstoffatomen steht, oder
für geradkettiges oder verzweigtes Alkyl mit bis zu 7 Kohlenstoffatomen steht, das gegebenenfalls eine Aminogruppe trägt, die durch Alkyl mit bis zu 3 Kohlenstoffatomen, tert.-Butyloxycarbonyl oder Benzyloxycarbonyl substituiert sein kann, oder das Alkyl gegebenenfalls ein- bis 3-fach, gleich oder verschieden durch Hydroxy, Cyano, Fluor, Chlor, Azido, Nitro, Trifluormethyl oder Phenyl substituiert ist, das seinerseits bis zu 2-fach gleich oder verschieden durch Nitro, Fluor, Chlor, Hydroxy oder durch geradkettiges oder verzweigtes Alkyl oder Alkoxy mit bis zu 3 Kohlenstoffatomen substituiert sein kann, oder
- R³: für Reste der Formeln 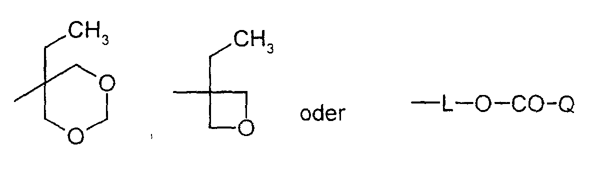 steht,
worin
L für eine geradkettige oder verzweigte Alkandiylgruppe mit bis zu 4 Kohlenstoffatomen steht,
Q für Alkyl mit bis zu 4 Kohlenstoffatomen steht, das gegebenenfalls durch Carboxyl substituiert ist, oder für Reste der Formeln 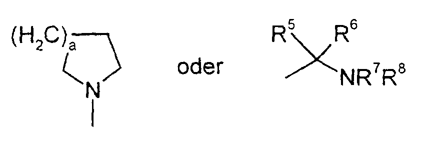 steht,
worin
a die Zahl 1 oder 2 bedeutet,
R⁵ Wasserstoff bedeutet,
R⁶ Cyclopentyl, Cyclohexyl, Phenyl oder Wasserstoff bedeutet, oder geradkettiges oder verzweigtes Alkyl mit bis zu 6 Kohlenstoffatomen bedeutet,
wobei das Alkyl gegebenenfalls durch Cyano, Methylthio, Hydroxy, Mercapto, Guanidyl, Amino, Carboxy oder H₂N-CO- substituiert sein kann,
oder das Alkyl durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann,
oder das Alkyl durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen substituiert sind oder durch tert.-Butyloxycarbonyl oder Benzyloxycarbonyl, geschützt sind,
R⁷ und R⁸ gleich oder verschieden sind und Wasserstoff, tert.-Butyloxycarbonyl oder Benzyloxycarbonyl bedeuten,
- R⁴: für Wasserstoff oder für einen Rest der Formel 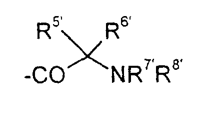 steht,
worin
R^{5'}, R^{6'}, R^{7'} und R^{8'} die oben angegebene Bedeutung von R⁵, R⁶, R⁷ und R⁸ haben und mit dieser gleich oder verschieden sind, und
X bevorzugt für Wasserstoff steht, und deren Stereoisomeren, stereoisomeren Gemische und Salze,
mit Ausnahme von N-[4-[[[5-(Dimethylamino)-1-naphthalenyl]sulfonyl]amino]phenyl]-acetamid.

Weitere bevorzugte Verbindungen der Erfindung sind Sulfonamide der obigen allgemeinen Formel (I). worin
- R³: für geradkettiges oder verzweigtes Alkenyl mit bis zu 5 Kohlenstoffatomen steht, oder
für geradkettiges oder verzweigtes Alkyl mit bis zu 7 Kohlenstoffatomen steht, worin das Alkyl eine Aminogruppe trägt, die durch Alkyl mit bis zu 3 Kohlenstoffatomen, tert.-Butyloxycarbonyl oder Benzyloxycarbonyl substituiert sein kann, oder das Alkyl ein- bis 3-fach, gleich oder verschieden durch Hydroxy, Cyano, Fluor, Chlor, Azido, Nitro, Trifluormethyl oder Phenyl substituiert ist, das seinerseits bis zu 2-fach gleich oder verschieden durch Nitro, Fluor, Chlor, Hydroxy oder durch geradkettiges oder verzweigtes Alkyl oder Alkoxy mit bis zu 3 Kohlenstoffatomen substituiert sein kann, oder
- R³: für Reste der Formeln 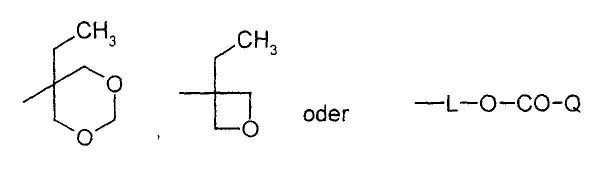 steht,
worin L und Q wie oben definiert sind, und
X bevorzugt für Wasserstoff steht.

Weitere bevorzugte Verbindungen der Erfindung sind Sulfonamide der obigen allgemeinen Formel (I), worin
- R¹ und R²: gleich oder verschieden sind und für Wasserstoffs Phenyl oder geradkettiges oder verzweigtes Alkyl oder Acyl mit jeweils bis zu 4 Kohlenstoffatomen stehen,
- A, D, E und G: gleich oder verschieden sind und für Wasserstoff, Fluor, Chlor, Brom, Hydroxy, Methyl oder Methoxy stehen,
- R³: für geradkettiges oder verzweigtes Alkenyl mit bis zu 4 Kohlenstoffatomen steht, oder
für geradkettiges oder verzweigtes Alkyl mit bis zu 5 Kohlenstoffatomen steht, das gegebenenfalls eine Aminogruppe trägt, die durch tert.- Butyloxycarbonyl oder Benzyloxycarbonyl substituiert sein kann, oder das gegebenenfalls ein- bis 3-fach gleich oder verschieden durch Hydroxy, Cyano, Fluor, Chlor, Nitro, Azido, Trifluormethyl oder Phenyl substituiert ist, das seinerseits bis zu 2-fach gleich oder verschieden, durch Nitro, Fluor, Chlor, Hydroxy, Methyl, Ethyl, Methoxy oder Ethoxy substituiert sein kann, oder
- R³: für Reste der Formeln 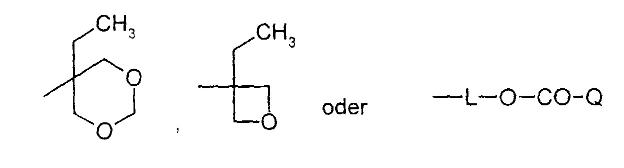 steht,
worin
L für eine geradkettige oder verzweigte Alkandiylgruppe mit bis zu 4 Kohlenstoffatomen steht,
Q für Alkyl mit bis zu 3 Kohlenstoffatomen steht, das gegebenenfalls durch Carboxyl substituiert ist, oder für einen Rest der Formel 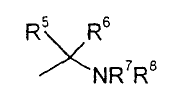 steht,
worin
R⁵ Wasserstoff bedeutet,
R⁶ Cyclopentyl, Cyclohexyl oder Wasserstoff bedeutet, oder geradkettiges oder verzweigtes Alkyl mit bis zu 4 Kohlenstoffatomen bedeutet,
wobei das Alkyl gegebenenfalls durch Cyano, Methylthio, Hydroxy, Mercapto, Guanidyl, Amino, Carboxy oder H₂N-CO- substituiert sein kann,
oder das Alkyl durch Cyclohexyl, Naphthyl oder Phenyl substituiert ist, das seinerseits durch Fluor, Chlor oder Alkoxy mit bis zu 4 Kohlenstoffatomen substituiert sein kann,
oder das Alkyl durch Indolyl, Imidazolyl, Triazolyl, Pyridyl oder Pyrazolyl substituiert ist, wobei die entsprechenden -NH-Funktionen gegebenenfalls durch Methyl substituiert oder durch Benzyloxymethylen oder tert.-Butyloxycarbonyl (BOC) geschützt sind,
R⁷ und R⁸ gleich oder verschieden sind und Wasserstoff oder tert.- Butyloxycarbonyl bedeuten,
- R⁴: für Wasserstoff oder für einen Rest der Formel 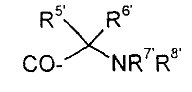 steht,
worin
R^{5'}, R^{6'}, R^{7'} und R^{8'} die oben angegebene Bedeutung von R⁵, R⁶_{,} R⁷ und R⁸ haben und mit dieser gleich oder verschieden sind, und
- X: bevorzugt für Wasserstoff steht,
und deren Stereoisomere, stereoisomeren Gemische und Salze,
mit Ausnahme von N-[4-[[[5-(Dimethylamino)-1-naphthalenyl]sulfonyl]amino]phenyl]-acetamid.

Weitere bevorzugte Verbindungen der Erfindung sind Sulfonamide der obigen allgemeinen Formel (I), worin
- R³: für geradkettiges oder verzweigtes Alkenyl mit bis zu 4 Kohlenstoffatomen steht, oder
für geradkettiges oder verzweigtes Alkyl mit bis zu 5 Kohlenstoffatomen steht, worin das Alkyl eine Aminogruppe trägt, die durch tert.- Butyloxycarbonyl oder Benzyloxycarbonyl substituiert sein kann, oder das Alkyl ein- bis 3-fach gleich oder verschieden durch Hydroxy, Cyano, Fluor, Chlor, Nitro, Azido, Trifluormethyl oder Phenyl substituiert ist, das seinerseits bis zu 2-fach gleich oder verschieden, durch Nitro, Fluor, Chlor, Hydroxy, Methyl, Ethyl, Methoxy oder Ethoxy substituiert sein kann, oder
- R³: für Reste der Formeln 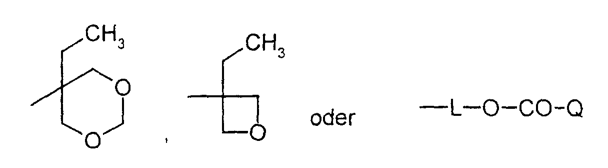 steht,
worin L oder Q wie oben definiert sind, und
X bevorzugt für Wasserstoff steht, und deren Stereoisomere, stereoisomeren Gemische und Salze.

Weitere besonders bevorzugte Verbindungen der Erfindung sind Sulfonamide der obigen allgemeinen Formel (I), worin
- R¹ und R²: für geradkettiges oder verzweigtes Alkyl mit bis zu 4 Kohlenstoffatomen stehen,
- A, D, E und G: für Wasserstoff, stehen,
- R³: für geradkettiges oder verzweigtes Alkyl mit bis zu 5 Kohlenstoffatomen steht, das durch Hydroxy substituiert ist, oder
- R³: für einen Rest der Formel

- L-O-CO-Q

steht,
worin
L für eine geradkettige oder verzweigte Alkandiylgruppe mit bis zu 4 Kohlenstoffatomen steht,
Q für einen Rest der Formel 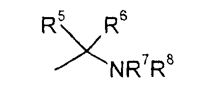 steht,
worin
R⁵ und R⁶ Wasserstoff bedeuten, und
R⁷ und R⁸ Wasserstoff bedeuten, und
- R⁴: für Wasserstoff steht, und
- X: bevorzugt für Wasserstoff steht,
und deren Stereoisomere, stereoisomeren Gemische und Salze.

Ganz besonders bevorzugte Verbindungen der Erfindung sind Sulfonamide, die ausgewählt sind aus der Gruppe der folgenden Verbindungen : 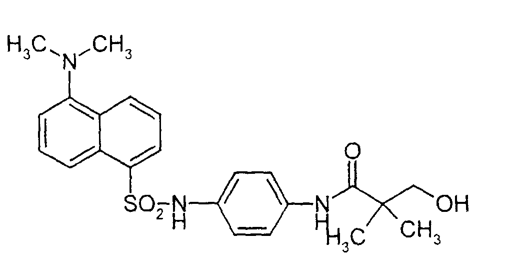 und

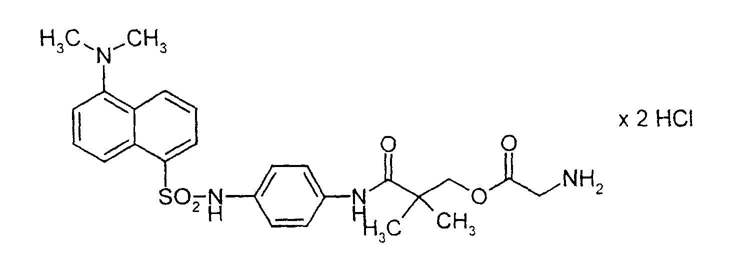

Die Erfindung betrifft weiterhin ein Verfahren zur Herstellung von Verbindungen der obigen allgemeinen Formel (I), das dadurch gekennzeichnet ist, daß man
[A] Verbindungen der allgemeinen Formel (II) 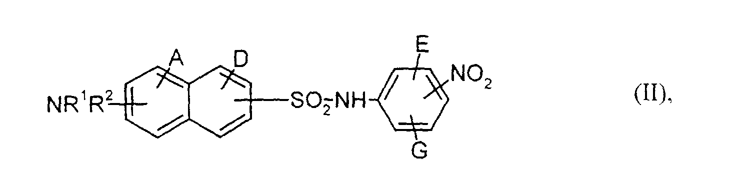 in welcher
   - A, D, E, G, R¹ und R²: die oben angegebene Bedeutung haben,
   zunächst durch katalytische Hydrierung an Palladium/C oder durch Reduktion mit SnCl₂ in inerten Lösemitteln in die Verbindungen der allgemeinen Formel (III) 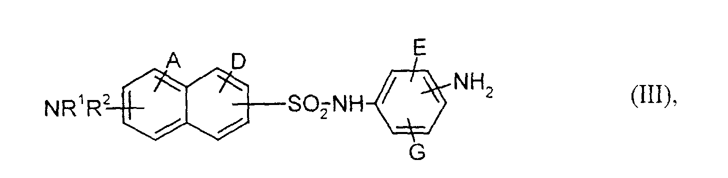 in welcher
   - A, D, E, G, R¹ und R²: die oben angegebene Bedeutung haben,
   überführt,
   und abschließend mit Verbindungen der allgemeinen Formel (IV)

   T-CO-R³ (IV)

   in welcher
   - R³: die oben angegebene Bedeutung hat
   und
   - T: für Hydroxy, Halogen, vorzugsweise für Chlor steht,
   in inerten Lösemitteln, gegebenenfalls in Anwesenheit einer Base und/oder eines Hilfsmittels umsetzt,
   oder
[B] Verbindungen der allgemeinen Formel (V) 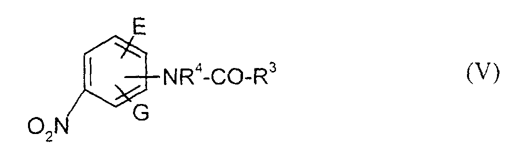 in welcher
   - E, G, R³ und R⁴: die oben angegebene Bedeutung haben,
   zunächst wie unter [A] beschrieben durch Hydrierung an Pd/C oder durch Reduktion mit SnCl₂ in inerten Lösemitteln in die Verbindungen der allgemeinen Formel (VI) 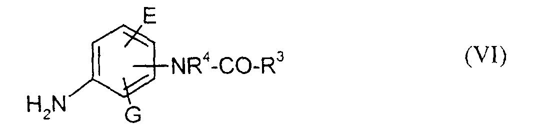 in welcher
   - E, G, R³ und R⁴: die oben angegebene Bedeutung haben, überführt
   und abschließend mit Verbindungen der allgemeinen Formel (VII) 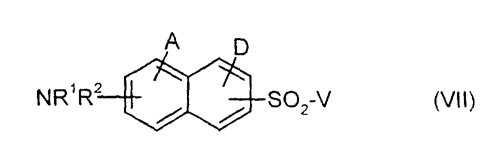 in welcher
   - A, D, R¹ und R² die: oben angegebene Bedeutung haben,
   und
   - V: für Halogen, vorzugsweise für Chlor steht,
   in inerten Lösemitteln, gegebenenfalls in Anwesenheit einer Base und/oder eines Hilfsmittels umsetzt,
   oder
[C] im Fall, daß R³ und/oder R³' für einen Rest der Formel -L-O-CO-Q stehen, Verbindungen der allgemeinen Formel (Ia) 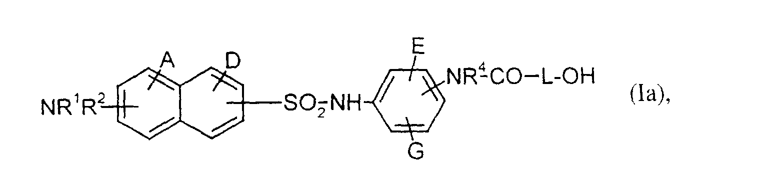 in welcher
   - R¹, R², R⁴, A, D, E, G und L: die oben angegebene Bedeutung haben,
   mit Aminosäureresten der allgemeinen Formel (VIII)

   HO-CO-Q' (VIII)

   in welcher
   - Q': die oben angegebene Bedeutung von Q hat, wobei einer der dort aufgeführten endständigen Reste am Stickstoff für eine der oben aufgeführten Schutzgruppen, vorzugsweise für tert.-Butyloxycarbonyl steht,
gegebenenfalls unter Aktivierung der Carbonsäure nach üblichen Methoden, in inerten Lösemitteln und in Anwesenheit einer Base und eines Hilfsstoffes umsetzt,
und abschließend die Schutzgruppe nach denen in der Peptid-Chemie üblichen Methoden abspaltet,
und im Fall X, R⁴ ≠ H mit 2 oder mehr Equivalenten der Verbindungen der allgemeinen Formel (VIII) umsetzt,
gegebenenfalls die Stereoisomere nach an sich bekannten Methoden trennt und gegebenenfalls die freien Basen in die Salze oder die Salze in die freien Basen überführt.

Die Erfindung betrifft weiterhin Sulfonamide der obigen allgemeinen Formel (I) zur Prophylaxe oder Behandlung von Krankheiten.

Weiterhin betrifft die Erfindung die Verwendung von Sulfonamiden der obigen allgemeinen Formel (I) zur Herstellung von Arzneimitteln, insbesondere Arzneimittel zur Bekämpfung viraler Erkrankungen und bevorzugt Arzneimittel zur Bekämpfung des Cytomegalievirus.

Die Erfindung betrifft schließlich Arzneimittel enthaltend Sulfonamide der obigen allgemeinen Formel (I).

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Mit dem Begriff "Alkandiylgruppe" werden hier Kohlenwasserstoff-Gruppen bezeichnet, die an zwei Positionen mit weiteren Resten verknüpft sind. Bevorzugt befinden sich diese Verknüpfungsstellen an verschiedenen Kohlenstoffatomen. Beispiele für Alkandiylgruppen sind: -CH₂-CH₂-, -CH₂-CH₂-CH₂-, -C(CH₃)₂-CH₂-, - CH(CH₃)-CH₂-, -C(CH₃)₂-CH₂-CH₂-, -CH(CH₃)-CH₂-CH₂- etc. Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1 -Dimethylethoxycarbonyl, Adamantylcarbonyl, Phthaloyl, 2,2,2-Trichlorethoxycarbonyl, 2,2,2-Trichlor-tertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen. 4-Nitrobenzyl, 2,4-Dinitrobenzyl oder 4-Nitrophenyl.

Die erfindungsgemäßen Verbindungen der allgemeinen Formel (I) können in verschiedenen stereochemischen Formen auftreten, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Die erfindungsgemäßen Verbindungen der allgemeinen Formel (I) können im Hinblick auf den Aminosäurerest sowohl in der D- oder L-Form, und R- oder S-Konfiguration vorliegen. Die Erfindung umfaßt die optischen Antipoden ebenso wie die Isomerengemische oder Racemate.

Im Rahmen der Erfindung können die erfindungsgemäßen Verbindungen der allgemeinen Formel (I) in Bezug auf die -SO₂-NX-Gruppe in der α- oder β-Position, an das Naphthylgerüst gebunden sein und die NH-CO-R³-Gruppe in o-, m- oder p-Position mit dem Phenylring verknüpft sein.

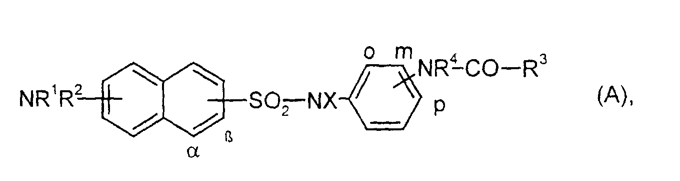

Bevorzugt wird die -SO₂-NX-Gruppe in α- oder β-Position an das Naphthylgerüst und die -NR⁴-CO-R³-Gruppe in m- oder p-Position an den Phenylrest gebunden.

Besonders bevorzugt wird die -SO₂-NX-Gruppe in α-Position an das Naphthylgerüst und die -NR⁴-CO-R³-Gruppe in p-Position an den Phenylrest geknüpft.

Besonders bevorzugte Verbindungen sind die in der Tabelle A aufgeführten:

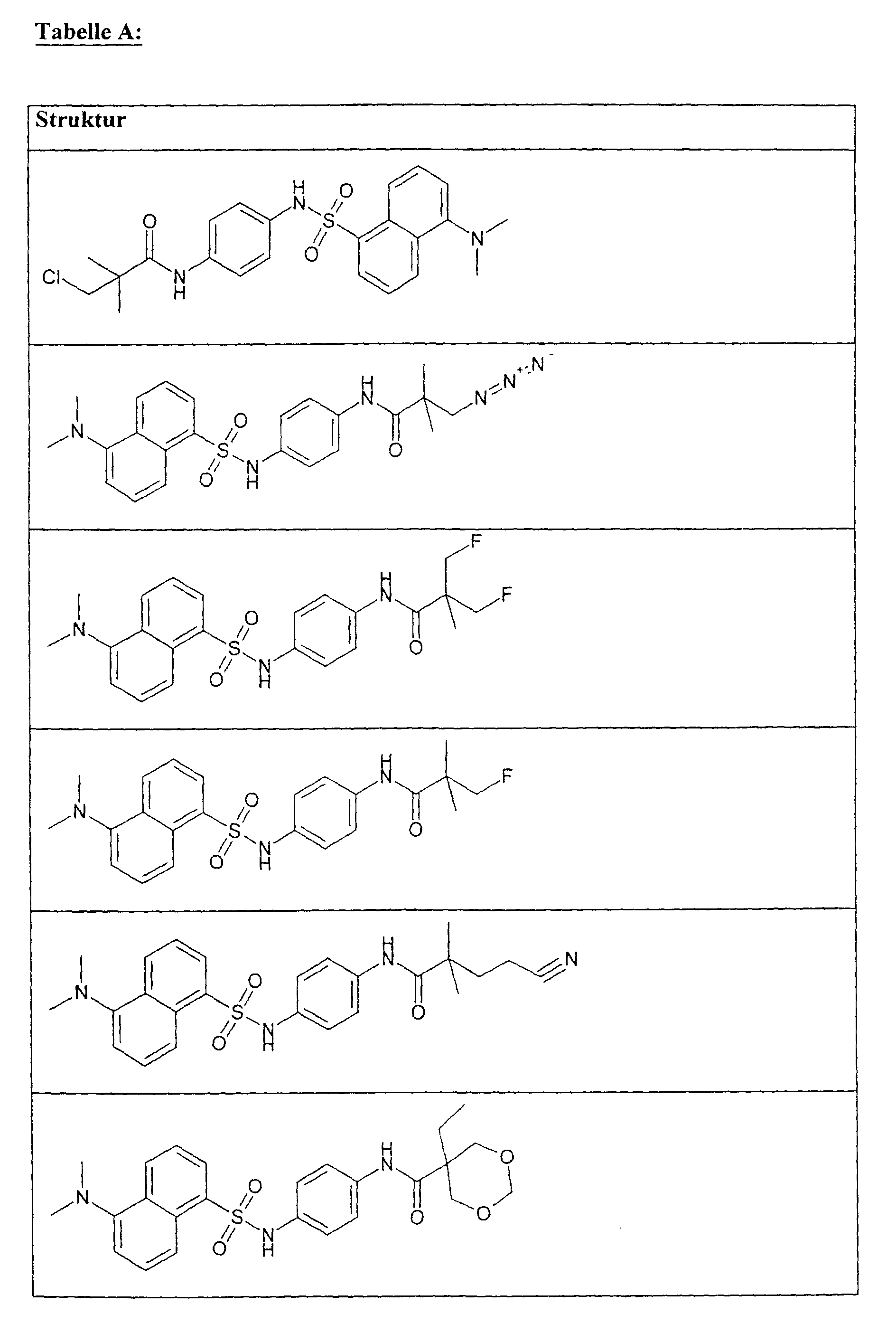

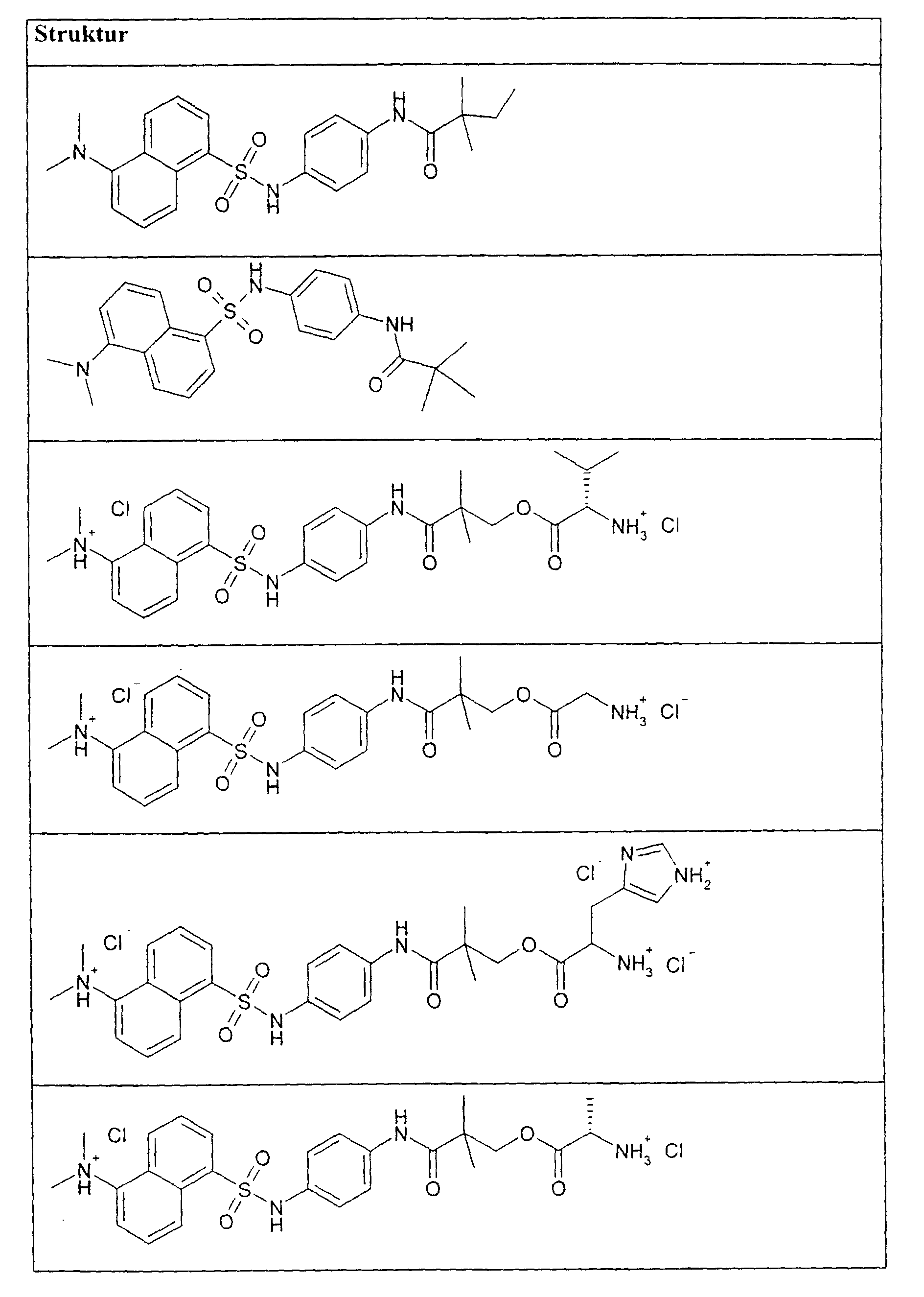

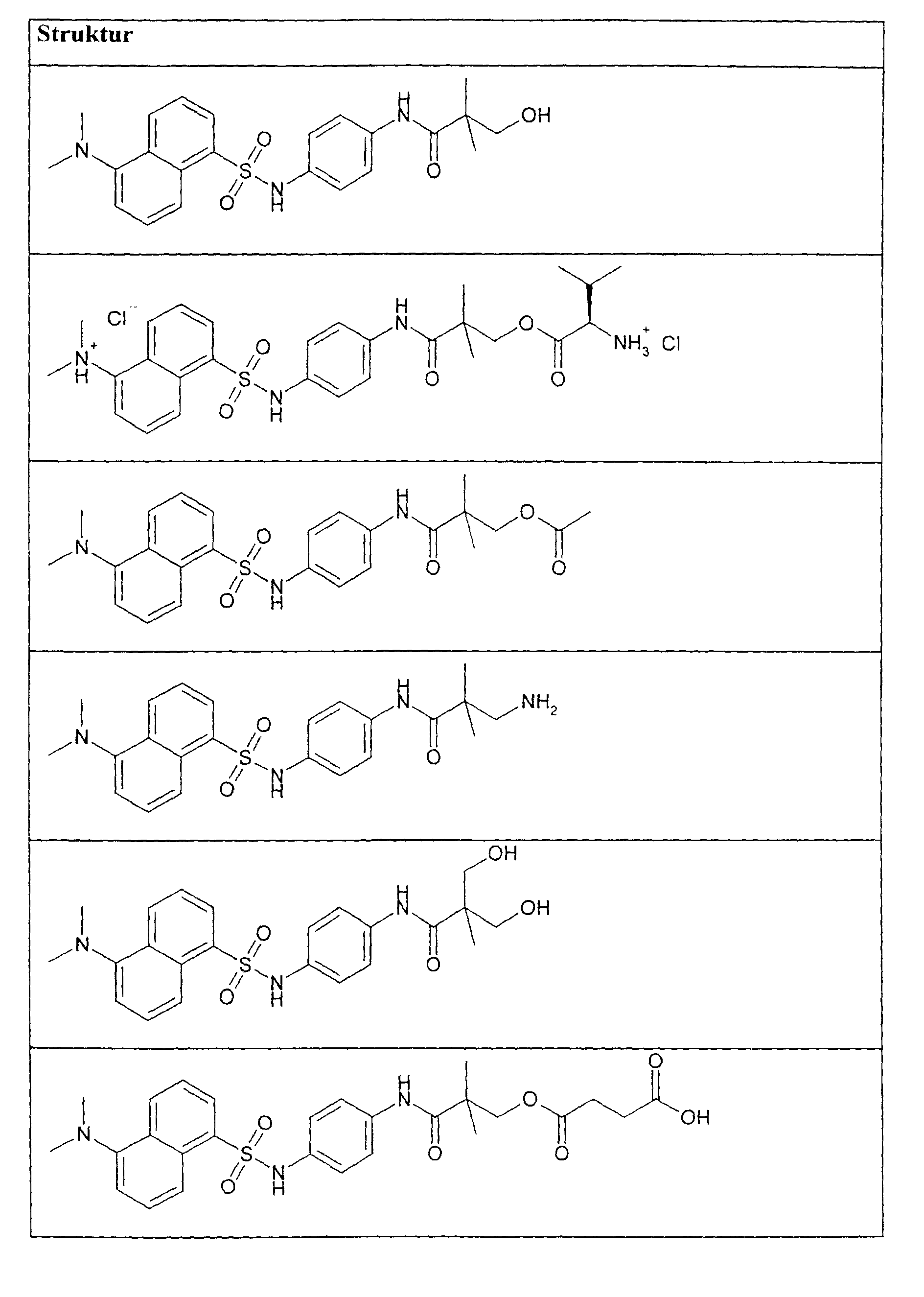

Die in Tabelle A aufgeführten, besonders bevorzugten Verbindungen können in Form der freien Base, als pharmazeutisch annehmbare Salze, insbesondere Hydrochloride, oder gegebenenfalls als Zwitterionen vorliegen.

Die erfindungsgemäßen Verbindungen der allgemeinen Formel (I) können hergestellt werden, indem man
[A] Verbindungen der allgemeinen Formel (II) 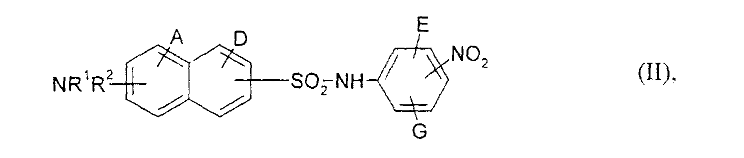 in welcher
   - A, D, E, G, R¹ und R²: die oben angegebene Bedeutung haben,
   zunächst durch katalytische Hydrierung an Palladium/C oder durch Reduktion mit SnCl₂ in inerten Lösemitteln in die Verbindungen der allgemeinen Formel (III) 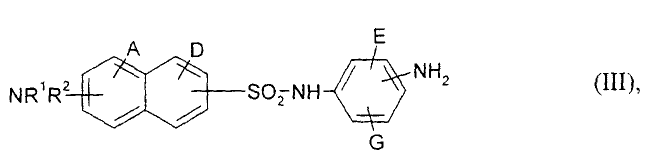 in welcher
   - A. D, E, G, R¹ und R²: die oben angegebene Bedeutung haben,
   überführt,
   und abschließend mit Verbindungen der allgemeinen Formel (IV)

   T-CO-R³ (IV)

   in welcher
   - R³: die oben angegebene Bedeutung hat
   und
   - T: für Hydroxy, Halogen, vorzugsweise für Chlor steht,
   in inerten Lösemitteln, gegebenenfalls in Anwesenheit einer Base und/oder eines Hilfsmittels umsetzt,
   oder
[B] Verbindungen der allgemeinen Formel (V) 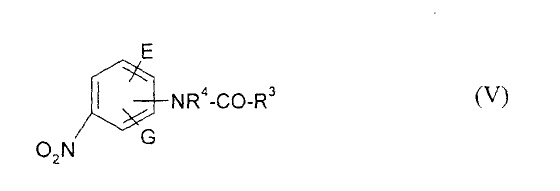 in welcher
   - E, G, R³ und R⁴: die oben angegebene Bedeutung haben,
   zunächst wie unter [A] beschrieben durch Hydrierung an Pd/C oder durch Reduktion mit SnCI₂ in inerten Lösemitteln in die Verbindungen der allgemeinen Formel (VI) 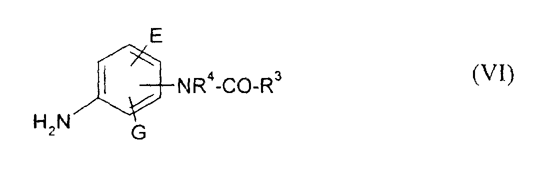 in welcher
   - E, G, R³ und R⁴: die oben angegebene Bedeutung haben,
   überführt
   und abschließend mit Verbindungen der allgemeinen Formel (VII) 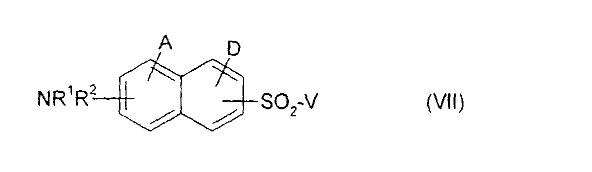 in welcher
   - A, D, R¹ und R²: die oben angegebene Bedeutung haben,
   und
   - V: für Halogen, vorzugsweise für Chlor steht,
   in inerten Lösemitteln, gegebenenfalls in Anwesenheit einer Base und/oder eines Hilfsmittels umsetzt,
   oder
[C] im Fall, daß R³ und/oder R^{3'} für einen Rest der Formel -L-O-CO-Q stehen, Verbindungen der allgemeinen Formel (Ia) 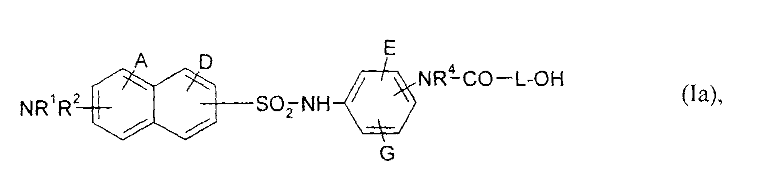 in welcher
   - R¹, R²_{,} R⁴, A, D, E, G und L: die oben angegebene Bedeutung haben,
   mit Aminosäureresten der allgemeinen Formel (VIII)

   HO-CO-Q' (VIII)

   in welcher
   - Q': die oben angegebene Bedeutung von Q hat, wobei einer der dort aufgeführten endständigen Reste am Stickstoff für eine der oben aufgeführten Schutzgruppen, vorzugsweise für tert.-Butyloxycarbonyl steht,
   gegebenenfalls unter Aktivierung der Carbonsäure nach üblichen Methoden, in inerten Lösemitteln und in Anwesenheit einer Base und eines Hilfsstoffes umsetzt,
   und abschließend die Schutzgruppe nach denen in der Peptid-Chemie üblichen Methoden abspaltet,
   und im Fall X, R⁴ ≠ H mit 2 oder mehr Equivalenten der Verbindungen der allgemeinen Formel (VIII) umsetzt.

Das erfindungsgemäße Verfahren kann durch folgende Formelschemata beispielhaft erläutert werden:

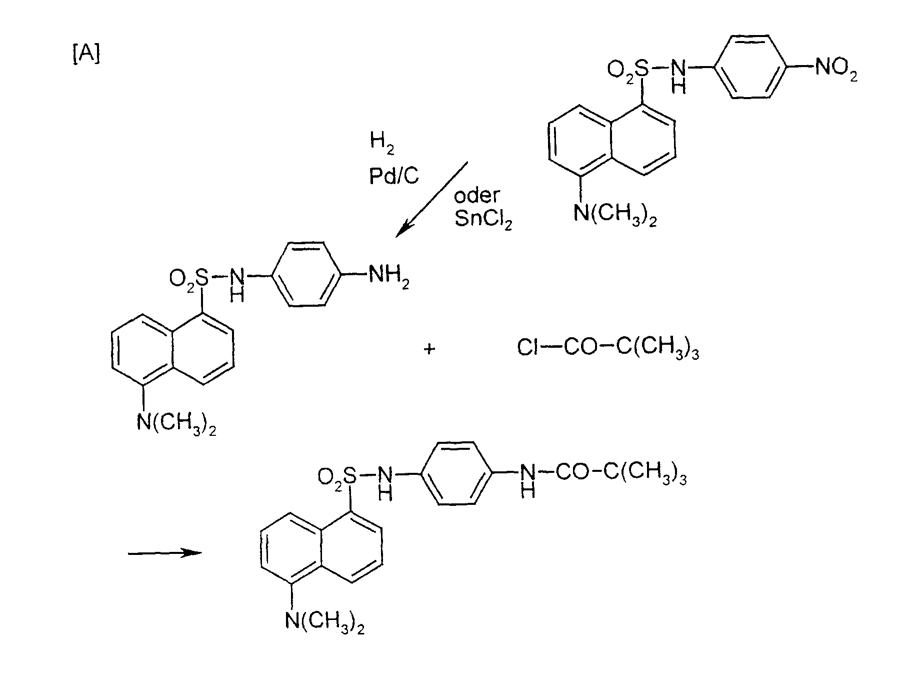

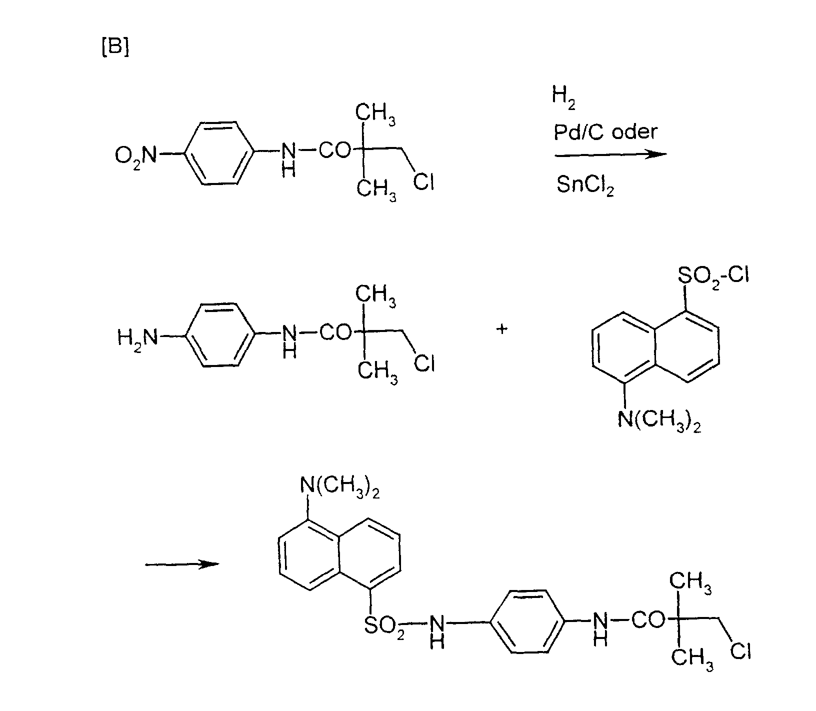

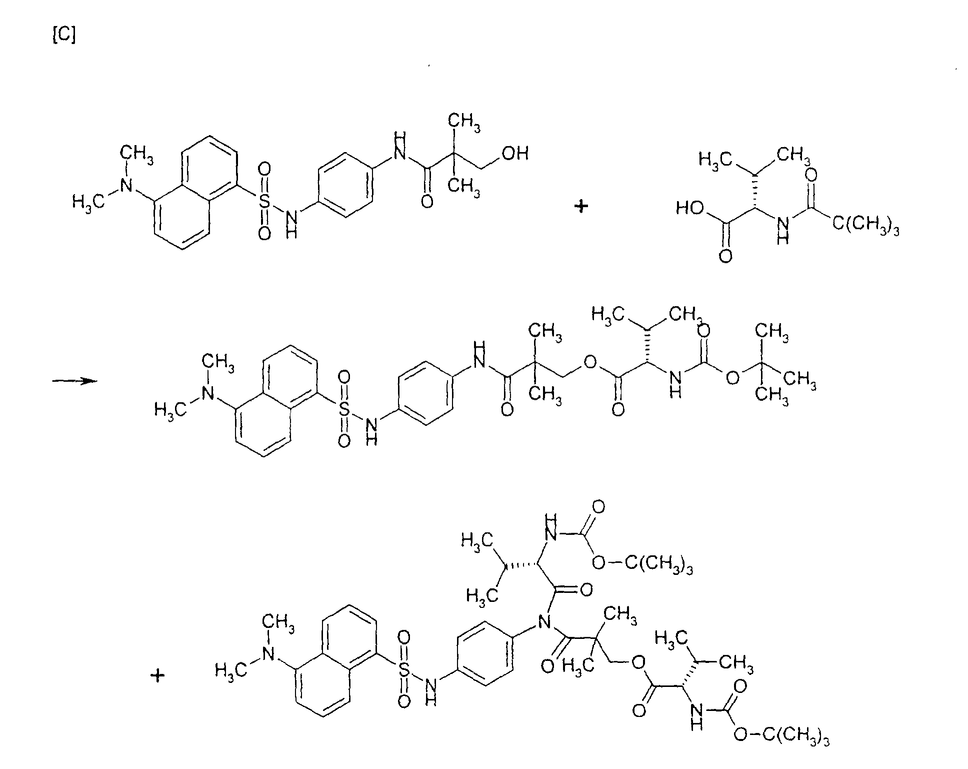

Als Lösemittel eignen sich für alle Verfahrensschritte die üblichen inerten Lösemittel, die sich unter den Reaktionsbedingungen nicht verändern. Hierzu gehören bevorzugt organische Lösemittel wie Ether z.B. Diethylether, Glykolmonooder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester. Pyridin, Triethylamin oder Picolin. Ebenso ist es möglich, Gemische der genannten Lösemittel, gegebenenfalls auch mit Wasser zu verwenden. Besonders bevorzugt sind Methylenchlorid, Tetrahydrofuran, Pyridin und Dioxan.

Als Basen eignen sich organische Amine, insbesondere Trialkyl(C₁-C₆)amine wie beispielsweise Triethylamin oder Heterocyclen wie Pyridin, Methylpiperidin, Piperidin oder N-Methylmorpholin. Bevorzugt sind Pyridin, Triethylamin und N-Methyimorpholin.

Die Basen werden im allgemeinen in einer Menge von 0,1 mol bis 5 mol, bevorzugt von 1 mol bis 3 mol jeweils bezogen auf 1 mol der Verbindungen der allgemeinen Formeln (III) und (IV) eingesetzt.

Als Hilfsmittel eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazolidumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionen werden in einem Temperaturbereich von 0°C bis 100°C, vorzugsweise bei 0°C bis 30°C und bei Normaldruck durchgeführt.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden wie SnCl₂, oder Boranen in inerten Lösemitteln, gegebenenfalls in Anwesenheit eines Katalysators durchgeführt werden. Bevorzugt ist Palladium auf Tierkohle oder SnCl₂.

Die Umsetzung kann bei normalem, erhöhtem oder bei emiedrigtem Druck durchgeführt werden (z.B. 0,5 bis 5 bar). Im allgemeinen arbeitet man bei Normaldruck.

Die Reduktionen werden im allgemeinen in einem Temperaturbereich von 0°C bis +60°C, vorzugsweise bei +10°C bis +40°C durchgeführt.

Als Lösemittel für die Acylierung eignen sich übliche organische Lösemittel, die sich unter den Reaktionsbedingungen nicht verändern. Hierzu gehören bevorzugt Ether wie Diethylether, Dioxan,Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dichlormethan und Pyridin.

Die Acylierung wird in den oben aufgeführten Lösemitteln bei Temperaturen von 0°C bis +150°C, vorzugsweise bei Raumtemperatur bis +100°C und bei Normaldruck durchgeführt.

Als Lösemittel für das Verfahren [C] eignen sich übliche organische Lösemittel, die sich unter den Reaktionsbedingungen nicht verändern. Hierzu gehören bevorzugt organische Lösemittel wie Alkohole, z.B. Methanol, Ethanol oder n-Propanol, Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Dichlorethan (DCE), Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden.

Besonders bevorzugt sind Dichlormethan, Dichlorethan, Dimethylformamid oder n-Propanol.

Als Hilfsstoffe für die jeweiligen Peptidkupplungen werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,'-Dipropyl-, n;n'-Diisopropyl-, N,N'-Dicyclohexylcarbodiimid, N-(3-Dimethylaminoisopropyl)-N'ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl- 1,2-dihydrochinolin, oder Propanphosphonsäureanhydrid, oder Isobutylchloroformat, oder Benzotriazolyloxy-tri-(dimethylamino)phosphoniumhexafluorophosphat, oder 1-Hydroxybenzotriazol und als Basen Alkalicarbonate z.B. Natrium- oder Kaliumcarbonat, oder -hydrogencarbonat, oder organische Basen wie Trialkylamine z.B. Triethylamin, N-Ethylmorpholin, N-Methylpiperidin oder Diisopropylethylamin eingesetzt. Besonders bevorzugt sind Dicyclohexylcarbodiimid, N-Methylmorpholin und 1-Hydroxybenztriazol.

Die Abspaltung der Aminoschutzgruppe erfolgt in an sich bekannter Weise unter sauren oder basischen Bedingungen, oder reduktiv durch katalytische Hydrierung beispielsweise mit Pd/C in organischen Lösemitteln wie Ethern z.B. Tetrahydrofuran oder Dioxan, oder Alkoholen z.B. Methanol, Ethanol oder Isopropanol.

Die Abspaltung der Aminoschutzgruppen kann ebenfalls nach üblichen Methoden mit Säuren, wie beispielsweise Chlorwasserstoffsäure oder Trifluoressigsäure erfolgen.

Die Umsetzungen werden im allgemeinen in einem Temperaturbereich von -20°C bis +80°C, bevorzugt von 0°C bis +60°C durchgeführt.

Im allgemeinen wird die Umsetzung bei Normaldruck durchgerührt. Es ist aber auch möglich, bei Unterdruck oder bei Überdruck zu arbeiten (z.B. von 0,5 bis 5 bar).

Die Verbindungen der allgemeinen Formel (VIII) sind bekannt.

Die Verbindungen der allgemeinen Formel (Ia) sind neu und können wie oben unter den Verfahren [A] und [B] aufgeführt, hergestellt werden.

Die Verbindungen der allgemeinen Formeln (II), (III), (IV), (V), (VI) und (VII) sind an sich bekannt oder nach literaturbekannten Methoden herstellbar.

Die vorliegende Erfindung umfaßt außerdem die Verwendung von N-[4-[[[5-(Dimethylamino)-1-naphthalenyl]sulfonyl]amino]phenyl]-acetamid als antivirales Mittel, insbesondere gegen Cytomegalieviren.

Die erfindungsgemäßen Verbindungen der allgemeinen Formeln (I) und (Ia) zeigen ein nicht vorhersehbares überraschendes Wirkspektrum. Sie zeigen eine antivirale Wirkung gegenüber Vertretern der Gruppe der Herpes viridae, besonders gegenüber dem humanen Cytomegalievirus (HCMV). Sie eignen sich somit zur Behandlung und Prophylaxe von Erkrankungen, die durch Herpes-Viren, insbesondere Erkrankungen, die durch humanes Cytomegalievirus (HCMV) hervorgerufen werden.

Die Anti-HCMV-Wirkung wurde in einem Screening-Testsystem in 96-Well-Mikrotiterplatten unter Zuhilfenahme von humanen embryonalen Lungenfibroblasten (HELF)-Zellkulturen bestimmt. Der Einfluß der Substanzen auf die Ausbreitung des cytopathogenen Effektes wurde im Vergleich zu der Referenzsubstanz Ganciclovir (Cymevene^{R}-Natrium), einem klinisch zugelassenen anti-HCMV-Chemotherapeutikum, bestimmt.

Die in DMSO (Dimethylsulfoxid) gelösten Substanzen (50 mM) werden auf Mikrotiterplatten (96-Well) in Doppelbestimmungen (4 Substanzen/Platte) untersucht. Toxische und cytostatische Substanzwirkungen werden dabei miterfaßt. Nach den entsprechenden Substanzverdünnungen (1:2) auf der Mikrotiterplatte wird eine Suspension von 50 - 100 HCMV-infizierten HELF-Zellen und 30 x 10⁵ nichtinfizierten HELF-Zellen in Eagle's MEM mit 10% fötalem Kälberserum in jedes Näpfchen gegeben, und die Platten bei 37°C in einem CO₂-Brutschrank über mehrere Tage inkubiert. Nach dieser Zeit ist der Zellrasen in den substanzfreien Viruskontrollen, ausgehend von 50 - 100 infektiösen Zentren, durch den cytopathogenen Effekt des HCMV völlig zerstört (100% CPE). Nach einer Anfärbung mit Neutralrot und Fixierung mit Formalin / Methanol werden die Platten mit Hilfe eines Projektions-Mikroskopes (Plaque-Viewer) ausgewertet. Die Ergebnisse sind für einige Verbindungen in der folgenden Tabelle zusammengefaßt:

**Tabelle:**

| Bsp.-Nr. | HCMV EC₅₀ |
|---|---|
| 1 | 0,16 |
| 2 | 0,27 |
| 13 | 0,064 |
| 16 | 0,028 |
| 24 | 0,88 |
| 25 | 0,64 |

Es wurde nun gefunden, daß die erfindungsgemäßen Verbindungen, die Vermehrung des HCMV in HELF-Zellen in z.T. 10-50fach niedrigeren Konzentrationen als Cymeven^{R}-Natrium hemmen und einen mehrfach höheren Selektivitätsindex aufweisen.

Die erfindungsgemäßen Verbindungen stellen somit wertvolle Wirkstoffe zur Behandlung und Prophylaxe von Erkrankungen dar, die durch humanes Cytomegalievirus ausgelöst werden. Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.

### In vivo-Wirkung

### Tiere

5 Wochen alte männliche Mäuse, Stamm NOD/LtSz-Prkdc(scid)/J, wurden von einem kommerziellen Züchter (The Jackson Lab., Bar Harbor) bezogen. Die Tiere wurden unter sterilen Bedingungen (einschließlich Einstreu und Futter) in Isolatoren gehalten.

### Virus/Infektion

Murines Cytomegalievirus (MCMV), Stamm Smith, wurde in vivo (BALB/c) passagiert und über eine fraktionierte Zentrifugation aufgereinigt. Der Titer wurde mit Hilfe eines Plaqueassays auf primären embryonalen Mäusefibroblasten untersucht. Die Infektion der Mäuse erfolgte mit einer Dosis von 5x10⁵ pfu in einem Gesamtvolumen von 0,2 ml intraperitoneal. Diese Dosis führt bei 100% der infizierten Tiere nach ca. 11 Tagen zum Tode.

### Behandlung/Auswertung

24 Stunden nach der Infektion wurden die Mäuse über einen Zeitraum von 8 Tagen zweimal täglich (morgens und abends) per os mit Substanz behandelt. Die Dosis betrug 25 mg/kg Körpermasse, das Applikationsvolumen 10 ml/kg Körpermasse. Die Formulierung der Substanzen erfolgte in Form einer 0,5%igen Tylosesuspension. 16 Stunden nach der letzten Substanzapplikation wurden die Tiere schmerzlos getötet und Speicheldrüse, Leber und Niere entnommen.
Aus 25 mg der Gewebe wurde über Phenol/Chloroform-Extraktion genomische DNA aufgereinigt. Die Quantifizierung der DNA erfolgte photometrisch und mit Hilfe der Formel OD₂₆₀x50=mg/ml.
Die Reinheit der DNA wurde über den Quotienten OD₂₆₀/OD₂₈₀ kontrolliert und die DNA anschließend mit Tris-EDTA pH = 8,0 eingestellt.
Die Quantifizierung der MCMV-DNA erfolgte mittels DNA-Dot-Blot-Hybridisierung. Als Sonde wurde ein Digoxygenin-gelabeltes (Boehringer-Mannheim, ebenfalls aufgeführte Puffer, wenn nicht anders beschrieben) 1,2 kb Fragment aus dem Bereich MCMV, Smith, HindIII J, verwendet. Die Detektion der Signale erfolgte mittels Chemolumineszenz. Dafür wurde die Membran für 3 Minuten in 1 x Digoxygenin-Waschpuffer 1 gewaschen. Im Anschluß wurden die Filter für 30 Minuten bei Raumtemperatur unter Schütteln in 1 x Digoxygenin Blockierungslösung inkubiert. Die Filter wurden danach für 30 Minuten in 20 ml/100 cm² Membran mit der Anti-DIG-Alkalische-Phosphatase-Konjugatlösung (1:20000 in 1 x Digoxygenin Blockierungslösung) inkubiert. 2 je 15 Minuten dauernde Waschschritte mit 1 x Digoxygenin-Waschpuffer schlossen sich an. Es folgten 5 Minuten Äquilibrierung der Filter in 1 x Digoxygenin-Detektionspuffer und die Detektion mittels 1 ml / 100 cm² Membranfläche 1:100 verdünnte CDP-Star-Lösung. Nach Ausstreichen der CDP-Star-Lösung und 5 minütiger Inkubation in einer dunklen Box erfolgte der Nachweis der Chemolumineszenz bzw. die Auswertung mittels Röntgenfilm (Kodak) oder Lumilmager (Boehringer Mannheim).

Alle Ergebnisse wurden statistisch gesichert (Varianzanalyse mittels Statistika; StatSoft Inc.).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilftlösemittel verwendet werden können.

Die Applikation erfolgt in üblicher Weise, vorzugsweise oral, parenteral oder topisch, insbesondere perlingual, intravenös oder intravital gegebenenfalls als Depot in einem Implantat.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

Gegebenenfalls kann es sinnvoll sein, die erfindungsgemäßen Verbindungen mit anderen Wirkstoffen zu kombinieren.

### Laufmittelgemische:

- A: Methylenchlorid : Methanol 100:0
- B: Methylenchlorid : Methanol 100:1
- C: Methylenchlorid : Methanol 100:2
- D: Methylenchlorid : Methanol 100:3
- E: Methylenchlorid : Methanol 100:5
- F: Methylenchlorid : Methanol 10:1
- G: Methylenchlorid : Methanol : Ammoniak 10:1:0,1
- H: Methylenchlorid : Cyclohexan 1:1
- I: Cyclohexan : Essigester 95:5
- K: Cyclohexan : Essigester 90:10
- L: Cyclohexan : Essigester 85:15
- M: Cyclohexan : Essigester 80:20
- N: Cyclohexan : Essigester 75:25
- O: Cyclohexan : Essigester 70:30
- P: Cyclohexan : Essigester 60:40
- Q: Cyclohexan : Essigester 50:50
- R: Cyclohexan : Essigester 40:60
- S: Cyclohexan : Essigester 30:70
- T: Butanol : Eisessig : Wasser 4:1:1
- U: Methylenchlorid : Methanol 9:1
- V: Acetonitril : Wasser 9:1
- W: Cyclohexan : Essigester 1:10
- X: Acetonitril : Wasser 95:5
- Y: Methylenchlorid : Methanol 95:5
- Z: Petrolether : Essigester 1:1
- ZA: Petrolether : Essigester 1:2
- ZB: Petrolether : Essigester 1:3

### Ausgangsverbindungen

### Beispiel I

### 4-[5-N,N-Dimethylamino-naphthyl-1 -sulfonamino]nitrobenzol

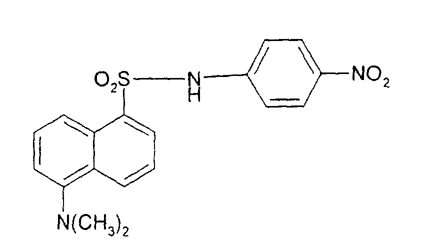

51,2 g (0,37 mol) p-Nitroanilin werden unter Argon und Eisbadkühlung in 700 ml Pyridin vorgelegt und portionsweise mit 100 g (0,37 mol) Dansylchlorid versetzt. Anschließend wird über Nacht bei Raumtemperatur nachgerührt. Die DC-Kontrolle in PE:EE 7:3 zeigt eine einheitliche Umsetzung an. Zur Aufarbeitung wird die Reaktionsmischung am Vakuum vom Pyridin befreit und der Rückstand in 1 N Natronlauge aufgenommen. Die wäßrige Phase wird von restlichem Pyridin erneut am Vakuum befreit und dann mit Salzsäure auf pH 4 gestellt. Das ausgefallene Produkt wird abgesaugt. Nach Trocknung im Umluftschrank bei 60°C werden 82 g (60%) des Sulfonamids als zitronengelber Feststoff erhalten.
MS (CI, NH₃, m/z): 372 ([M+H]⁺, 100 %).
¹H-NMR (200 MHz, DMSO-d6, δ/ppm): 8.46 (d, J = 9.0 Hz; 1H). 8.40 - 8.31 (m; 2H), 8.05 (d, J = 10 Hz; 2H), 7.65 (dt, J₁ = 7.5 Hz, J₂ = 5 Hz; 2H), 7.28 - 7.18 (m; 3H), 2.80 (s; 6H).

### Beispiel II

### 4-[5-N,N-Dimethylamino-naphthyl-1-sulfonylamino]anilin

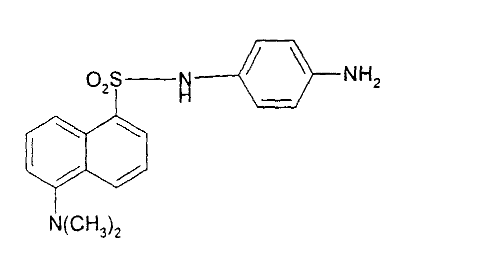

63 g (0,17 mol) der Verbindung aus Beispiel I werden in 400 ml Ethanol gelöst, mit 1,5 g 10% Pd auf Aktivkohle versetzt und bei 3 bar Wasserstoff 25 h lang hydriert. DC-Kontrolle PE:EE 1:1 (R_{f}= 0,28) zeigt vollständige Umsetzung an. Zur Aufarbeitung wird vom Katalysator abfiltriert und am Vakuum vom Solvenz befreit. Der so erhaltene Hartschaum wird in 1 N Schwefelsäure gelöst und anschließend ausgeethert. Mit 2,5 N Natronlauge wird neutral gestellt und der Niederschlag abgesaugt. Das Produkt wird bei 60°C im Umlufttrockenschrank getrocknet. Man erhält 50 g (86%) des Amins als weißen Feststoff.
R_{f} = 0,29 (E)
MS (CI, NH₃, m/z): 342 ([M+H]⁺, 100 %).
¹H-NMR (200 MHz, DMSO-d6, δ/ppm): 9.80 (s; 1H), 8.42 (d, J = 8.0 Hz; 1H), 8.38 (d, J = 8.0 Hz; 1H), 8.00 (d, J = 7.5 Hz; 1H), 7.60 (t, J = 7.5 Hz; 1H), 7.54 (t, J = 7.5 Hz; 1H), 7.25 (d, J = 7.5 Hz; 1H), 6.60 (d, J = 9.0 Hz; 2H), 6.40 (d, J = 9.0 Hz; 2H), 4.90 (s; 2H), 2.80 (s; 6H).

### Beispiel III

### N-(4-Nitrophenyl)-pivaloylamid

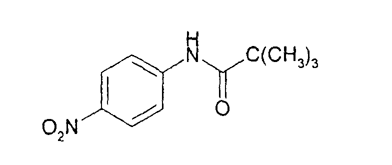

20 g (0,14 mol) 4-Nitroanilin werden im 1 l Dreihalskolben in 400 ml Methylenchlorid und 100 ml Dioxan gelöst. Anschließend wird mit 23,4 ml Pyridin versetzt. Unter Eiskühlung werden 19,2 g (0,16 mol) Pivalinsäurechlorid zugetropft. Es wird 24 h bei RT gerührt. Danach wird 3 x mit 200 ml Wasser ausgeschüttelt und die organische Phase mit Natriumsulfat getrocknet. Die organische Phase wird einrotiert und der Rückstand in 100 ml Diisopropylether 2 h gut gerührt, abgesaugt, mit Diisopropylether und Pentan gewaschen und der Rückstand getrocknet.

Ausbeute: 30 g (93% d.Th.)

### Beispiel IV

### N-(4-Aminophenyl)-pivaloylamid

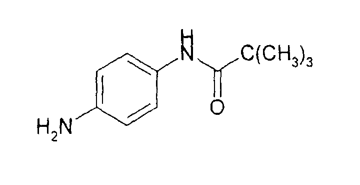

25 g (0,11 mol) der Verbindung aus Beispiel III werden im 1 l Rundkolben in 500 ml Dioxan unter Rühren gelöst. Anschließend wird mit Argon überschichtet und 5 g 10% Pd auf Aktivkohle zugegeben. Es wird bei Raumtemperatur und Normaldruck 20 h hydriert. Der Katalysator wird über Kieselgur abgesaugt und die Mutterlauge einrotiert. Der Rückstand wird in 200 ml Heptan 2 h gut verrührt, abgesaugt und mit Heptan gewaschen, die Kristalle werden getrocknet.
Ausbeute: 19,6 g (90,6%)

### Herstellungsbeispiele

### Beispiel 1

### N-[4-(5-N,N-Dimethylamino-naphthyl-1-sulfonylamino)phenyl]-2,2-dimethylpropionamid

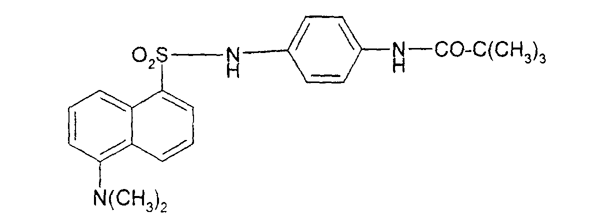

17,8 g (0,09 mmol) der Verbindung aus Beispiel IV werden unter Argon in Pyridin vorgelegt und 25 g (0,09 mol) Dansylchlorid portionsweise zugegeben. Anschlißend wird 18 h bei Raumtemperatur nachgerührt (DC-Kontrolle PE:EE 7:3). Zur Aufarbeitung wird das Pyridin im Vakuum entfernt, der Rückstand in Dioxan aufgenommen und mit Wasser wieder ausgefällt. Da das Produkt zunächst als Öl anfällt, wird vom wässrigen Solvens getrennt, das Öl mit Ether verrührt und der kristalline Niederschlag abgesaugt. Es wird ein leicht gelblicher Feststoff erhalten.
Fp.: 174°C

### Beispiel 2

### N-[4-(5-(N,N-Dimethylamino)naphthyl-1-sulfonylamino)phenyl]-3-hydroxy-2,2-dimethylpropionamid

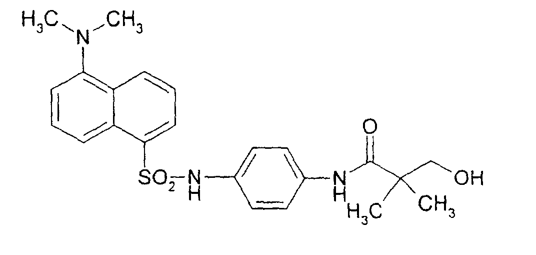

50 g (0,15 mol) der Verbindung aus Beispiel II werden in 800 ml Methylenchlorid mit 203 ml (1,46 mol) Triethylamin und 51,9 g (0,44 mol) 3-Hydroxy-2,2-dimethylpropionsäure versetzt. Unter Kühlung werden 200 ml einer 1,7 M Lösung von n-Propanphosphonsäureanhydrid in Ethylacetat innerhalb 30 min so zugetropft, daß die Innentemperatur 15°C nicht überschreitet. Über Nacht wird bei Raumtemperatur nachgerührt. DC-Kontrolle in Methylenchlorid / Methanol 100:5 (R_{f} = 0,29) zeigt vollständige Umsetzung an. Zur Aufarbeitung wird mit 800 ml Methylenchlorid verdünnt, 4 mal mit Wasser und 1 mal mit verdünnter Essigsäure gewaschen. Nach Trocknung über Natriumsulfat wird am Vakuum vom Solvenz befreit. Der zurückbleibende Hartschaum wird über eine Kieselgelsäule gereinigt (Laufmittel: D, E, F, G). Der so erhaltene Hartschaum wird aus Toluol umkristallisiert. So werden 33 g (51 %) des Amids als weißer Feststofferhalten.
MS (CI, NH₃, m/z): 442 ([M+H]⁺, 100 %).
¹H-NMR (200 MHz, DMSO-d6, δ/ppm): 10.46 (s; 1H), 9.10 (s; 1H), 8.40 (t breit, J = 7.5 Hz; 2H), 8.14 (d, J = 7.5 Hz; 1H), 7.62 (t, J = 7.5 Hz; 1H), 7.55 (t, J = 7.5 Hz; 1H), 7.36 (d, J = 10.0 Hz; 2H), 7.24 (d, J = 7.5 Hz; 1H), 6.92 (d, J = 10.0 Hz; 2H), 5.04 (t, J = 5.0 Hz; 1H), 3.42 (d, J = 5.0 Hz; 2H), 2.80 (s; 6H), 1.07 (s; 6H).

### Beispiel 3 und Beispiel 4

### N-[4-(5-N,N-Dimethylaminonaphthyl-1-sulfonylamino)phenyl]-3-((2S)-2-(N-tert.butyloxycarbonylamino)-2-isopropylacetoxy)-2,2-dimethylpropionamid (Beispiel 3) und N- [4-(5-N,N-Dimethylaminonaphthyl-1-sulfonylamino)phenyl]-N-[(2S)-2-(Ntert.-butyloxycarbonylamino)-2-isopropylacetyl]-3-(2S)-2-(N-tertbutyloxycarbonylamino)-2-isopropylacetoxy)-2,2-dimethylpropionamid (Beispiel 4)

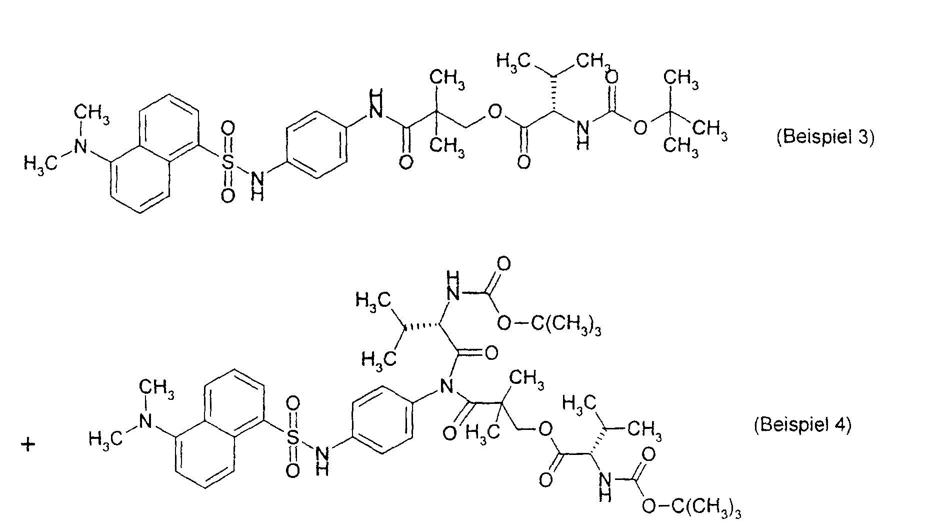

10,0 g (0,02 mol) der Verbindung aus Beispiel 2, 6,4 g (0,03 mol) N-Boc-L-Valin und 1,1 g (0,01 mol) Dimethylaminopyridin werden in 170 ml Methylenchlorid vorgelegt und 6,1 g (0,03 mol) EDC zugegeben. Danach wird über Nacht bei Raumtemperatur gerührt. Anschließend wird mit Essigester verdünnt, 2 mal mit gesättigter NaHCO₃-Lösung extrahiert und getrocknet. Die Reinigung erfolgt über eine Kieselgelsäule mit Petrolether / Essigester 2:1.
Beispiel 3: Ausbeute: 10,8 g (74,4 %) R_{f}= 0,55 (Z)
Beispiel 4: Ausbeute: 1,7 g (8,9 %) R_{f}= 0,65 (Z)

### Beispiel 5

### N-[4-(5-N,N-Dimethylaminonaphthyl- 1-sulfonylamino)phenyl]-3-((2S)-2-amino-2-isopropylacetoxy)-2,2-dimethylpropionamid dihydrochlorid

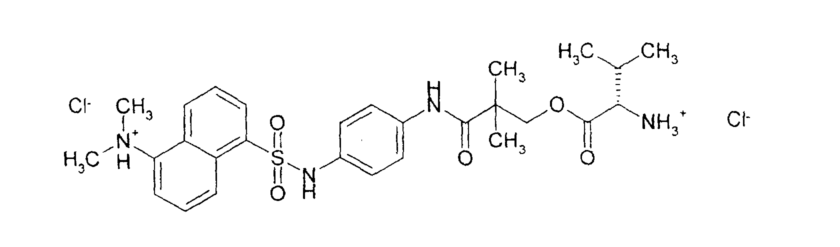

10,5 g (0,02 mol) der Verbindung aus Beispiel 3 werden mit 200 ml 4 N HCl in Dioxan versetzt und 4 h gerührt. Anschließend wird einrotiert, der Rückstand 2 mal in 100 ml Wasser gelöst und erneut einrotiert. Anschließend wird 2 mal mit 100 ml Acetonitril versetzt, einrotiert und 2 mal mit Ether versetzt und erneut einrotiert. Über Nacht wird an der Ölpumpe getrocknet.

Man erhält 9,7 g (96 %) eines hellgelben Pulvers (Smp. 130°C Zers.).

### Beispiel 6

### N-[4-(5-N,N-Dimethylaminonaphthyl-1-sulfonylamino)phenyl]-3-(2-(N-tert.butyloxycarbonylamino)-2-acetoxy)-2,2-dimethylpropionamid

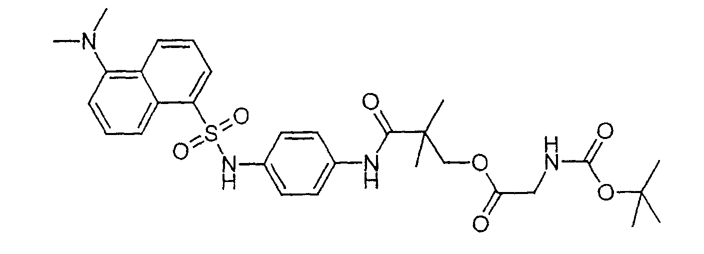

14,3 g [0.08 mol] BOC-Glycin werden in 300 ml Methylenchlorid bei Raumtemperatur vorgelegt, mit 13,2 g [0.08 mol] Carbonyldiimidazol versetzt und 2 h bei dieser Temperatur verrührt. Nach DC Kontrolle werden 30,0 g [0.07 mol] Beispiel 2 zugegeben und über Nacht bei Raumtemperatur nachgerührt.

Zur Aufarbeitung wird mit 500 ml Methylenchlorid verdünnt, 2x mit 300ml 0.01 N Schwefelsäure und 1x mit Wasser gewaschen. Nach Trocknung über Natriumsulfat wird einrotiert und ein grünlicher Hartschaum erhalten, der aus Toluol umkristallisiert wird.

Man erhält 43,0 g [88% d. Th.] Beispiel 43 als grünliche Kristalle.
MS (CI, NH₃, m/z): 616 ([M+H]⁺, 100 %).
¹H-NMR (200 MHz, DMSO-d6, ?/ppm): 10.50 (s; 1H), 9.12 (s; 1H), 8.40 (t, J = 8.0 Hz; 2H), 8.14 (d, J = 8.0 Hz; 1H), 7.68 - 7.52 (m; 2H), 7.35 (d, J = 10.0 Hz; 2H), 7.26 (d, J = 8.0 Hz; 1H), 6.94 (d, J = 10.0 Hz; 2H), 4.12 (s; 2H), 3.62 (d, J = 6.0 Hz; 2H), 2.80 (s; 6H), 1.32 (s; 9H) 1.15 (s; 6H).

### Beispiel 7

### N-[4-(5-N,N-Dimethylaminonaphthyl-1-sulfonylamino)phenyl]-3-(2-aminoacetoxy)-2,2-dimethylpropionamid hydrochlorid

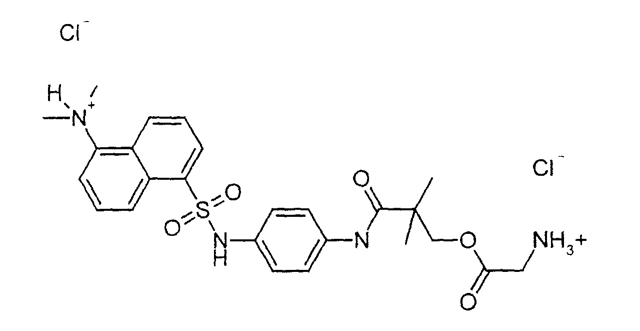

39,5 g [0.07 mol] der Verbindung aus Beispiel 6 werden in 200 ml Dichlormethan vorgelegt und langsam mit 80 ml einer 4 N Dioxan/HCl Lösung unter Rühren versetzt. Bis zur Beendigung der Gasentwicklung wird nachgerührt (ca. 3 h). Das als Suspension vorliegende Produkt wird fein zermahlen, abgesaugt, mit 100 ml Dichlormethan nachgewaschen und über Nacht im Hochvakuum getrocknet.

Man erhält 34,5 g [98%] Beispiel 7 als weißen Feststoff.
MS (CI, NH₃, m/z): 442 ([M-Glycin+H]⁺, 100 %), 499 ([M+H]⁺, 20 %).
¹H-NMR (400 MHz, D₂O-d2, ?/ppm): 8.80 (d, J = 8.0 Hz; 1H), 8.42 (d, J = 8.0 Hz; 1H), 8.35 (d, J = 8.0 Hz; 1H), 8.08 (d, J = 8.0 Hz; 1H), 7.92 (t, J = 8.0 Hz; 1H), 7.82 (dd, J₁ = 7.0 Hz, J₂ = 8.0 Hz; 1H), 7.15 (d, J = 10.0 Hz; 2H), 6.98 (d, J = 10.0 Hz; 2H), 4.34 (s; 2H), 3.94 (s; 2H), 3.50 (s; 6H), 1.32 (s; 6H).

### Beispiel 8

### 4-Acetamidonaphthalin-1-sulfonsäure-N-[4-(N-2,2-dimethylpropanoyl)aminophenyl]amid

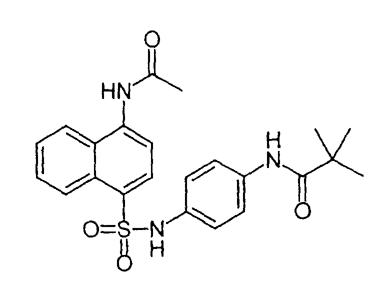

Eine Lösung von 3.46 g (18.00 mmol) 4-(N-2,2-Dimethylpropanoyl)aminoanilin (Beispiel IV) in 72 ml wasserfreiem Pyridin wird mit einer Lösung von 5.87 g (20.70 mmol) 4-Acetamidonaphthalin-1-sulfonylchlorid in 72 ml wasserfreiem THF versetzt und über Nacht bei 40°C gerührt. Anschließend wird das Lösemittelgemisch am Rotationsverdampfer entfernt und der Rückstand mit einem Gemisch von 20 ml Ethylacetat und 150 ml 2-molarer Salzsäure verrührt. Der dabei anfallende Feststoff wird abgesaugt und mit Wasser, wenig Ethylacetat und Ether gewaschen.

Nach Trocknen im Hochvakuum werden 6.0 g [76 %] Beispiel 8 in Form einer blaß rosafarbenen Verbindung erhalten, die sich aus Ethanol umkristallisieren läßt.
m.p.: >240 °C.
R_{f}: 0.13 (CH₂Cl₂/MeOH, 100:5).
MS (CI, NH₃, m/z): 457 (M+NH₄⁺), 440 (M+H⁺).
¹H-NMR (300 MHz, DMSO-d₆, δ/ppm): 10.39 (1H, s), 10.11 (1H, s), 8.98 (1H, s), 8.74 (1H, dd), 8.28 (1H, dd), 8.12 (1H, d), 7.91 (1H, d), 7.73 (1H, dt), 7.68 (1H, dt), 7.33 (2H, d), 6.90 (2H, d), 2.21 (3H, s), 1.13 (9H, s).

### Beispiel 9

### 4-Aminonaphthalin-1-sulfonsäure-N-[4-(N-2,2-dimethylpropanoyl)aminophenyl]amid

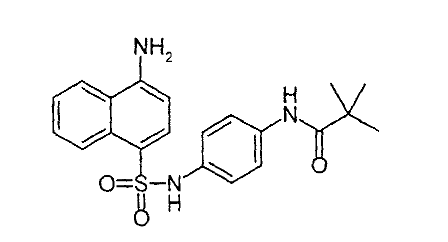

5.83 g (13.26 mmol) 4-Acetamidonaphthalin-1-sulfonsäure-*N*-[4-(*N*-2,2-dimethylpropanoyl)aminophenyl]amid (Bsp. 8) werden in 165 ml 5%iger wässriger Lithiumhydroxid-Lösung gelöst und 14 Stunden auf 60°C erwärmt. Nach dem Abkühlen wird das Reaktionsgemisch mit 2-molarer Salzsäure auf pH 4 gebracht. Dabei fällt ein blaß rosafarbener Niederschlag aus, der abgesaugt und mit Wasser gewaschen wird.

Nach Trocknen im Hochvakuum werden 5.0 g (12.58 mmol, 91% Ausbeute) erhalten.
m.p.: 216 °C.
R_{f}: 0.28 (CH₂Cl₂/MeOH, 100:5).
MS (CI, NH₃, m/z): 415.1 (M+NH₄⁺), 398 (M+H⁺).
¹H-NMR (300 MHz, DMSO-d₆, δ/ppm): 9.98 (1H, s), 8.93 (1H, s), 8.57 (1H, d), 8.17 (1H, d), 7.87 (1H, d), 7.60 (1H, t), 7.45 (1H, t), 7.31 (2H, d), 6.87 (2H, d), 6.69 (2H, s), 6.57 (1H, d), 1.13 (9H, s).

### Beispiel 10

### 4-Methylaminonaphthalin-1-sulfonsäure-N-[4-(N-2,2-dimethylpropanoyl)aminophenyl]amid

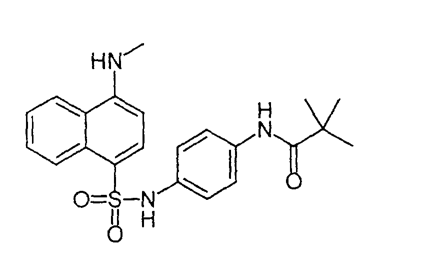

Bei 0°C wird eine Lösung von 1.24 ml (3.72 mmol) 3-molarer Schwefelsäure in 20 ml THF nacheinander mit 1.67 ml (20.6 mmol) einer 37%igen Formaldehyd-Lösung, einer Lösung von 1.50 g (3.77 mmol) 4-Aminonaphthalin-1-sulfonsäure-N-[4-(N-2,2-dimethylpropanoyl)aminophenyl]amid (Bsp. 9) in 20 ml THF und 1.66 g (26.42 mmol) festem Natriumcyanoborhydrid versetzt. Nach zwei Stunden bei 0°C wird das Reaktionsgemisch mit 30 ml 2-molarer Natronlauge versetzt und zur Trockene eingedampft. Der Rückstand wird mit einem Gemisch aus 2-molarer Salzsäure und wenig Ethylacetat aufgenommen. Der unlösliche Rückstand wird abgesaugt und chromatographisch gereinigt (Flash-Chromatographie, Kieselgel, Cyclohexan/Essigester, 3:2). Es werden zwei Fraktionen erhalten. Die Dimethylaminoverbindung wird als Nebenkomponente erhalten. Hauptprodukt ist die Monomethylaminoverbindung, die in Form eines weissen Feststoffs anfällt.

Man erhält 1,0 g [64%] Beispiel 10
m.p.: 248 °C (Zers.).
R_{f}: 0.30 (Cyclohexan/Ethylacetat, 3:2).
MS (ESI, m/z): 434 (M+Na⁺), 412 (M+H⁺).
¹H-NMR (300 MHz, DMSO-d₆, δ/ppm): 10.04 (1H, s), 8.94 (1H, s), 8.60 (1H, d), 8.19 (1H, d), 7.97 (1H, d),7.62 (1H, t), 7.49 (1H, t), 7.32 (2H, d), 7.23 (1H, quart), 6.87 (2H, d), 6.38 (1H, d), 2.88 (3H, d), 1.13 (9H, s).

Analog der oben aufgeführten Vorschriften werden die in der folgenden Tabelle 1 aufgeführten Verbindungen hergestellt:

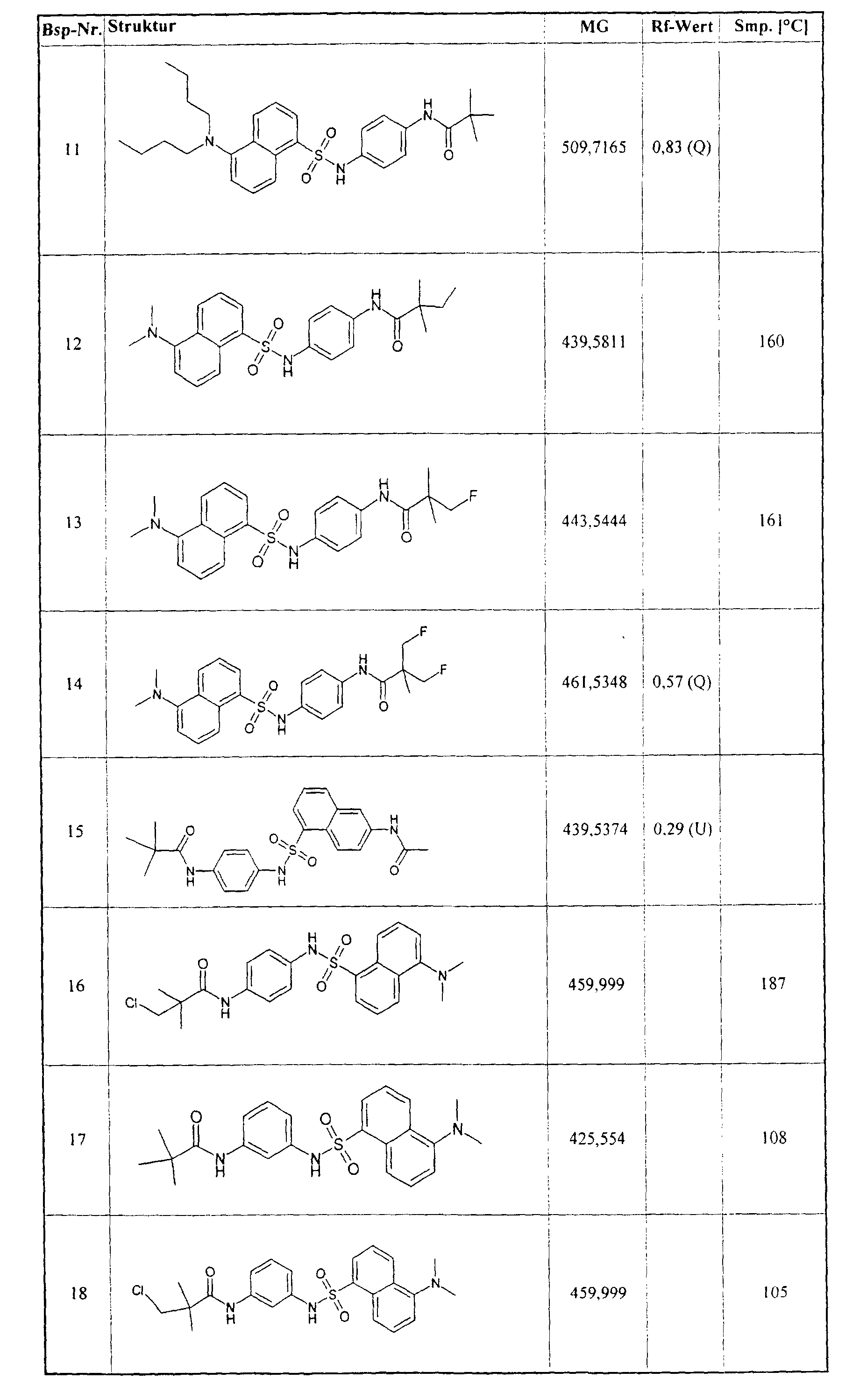

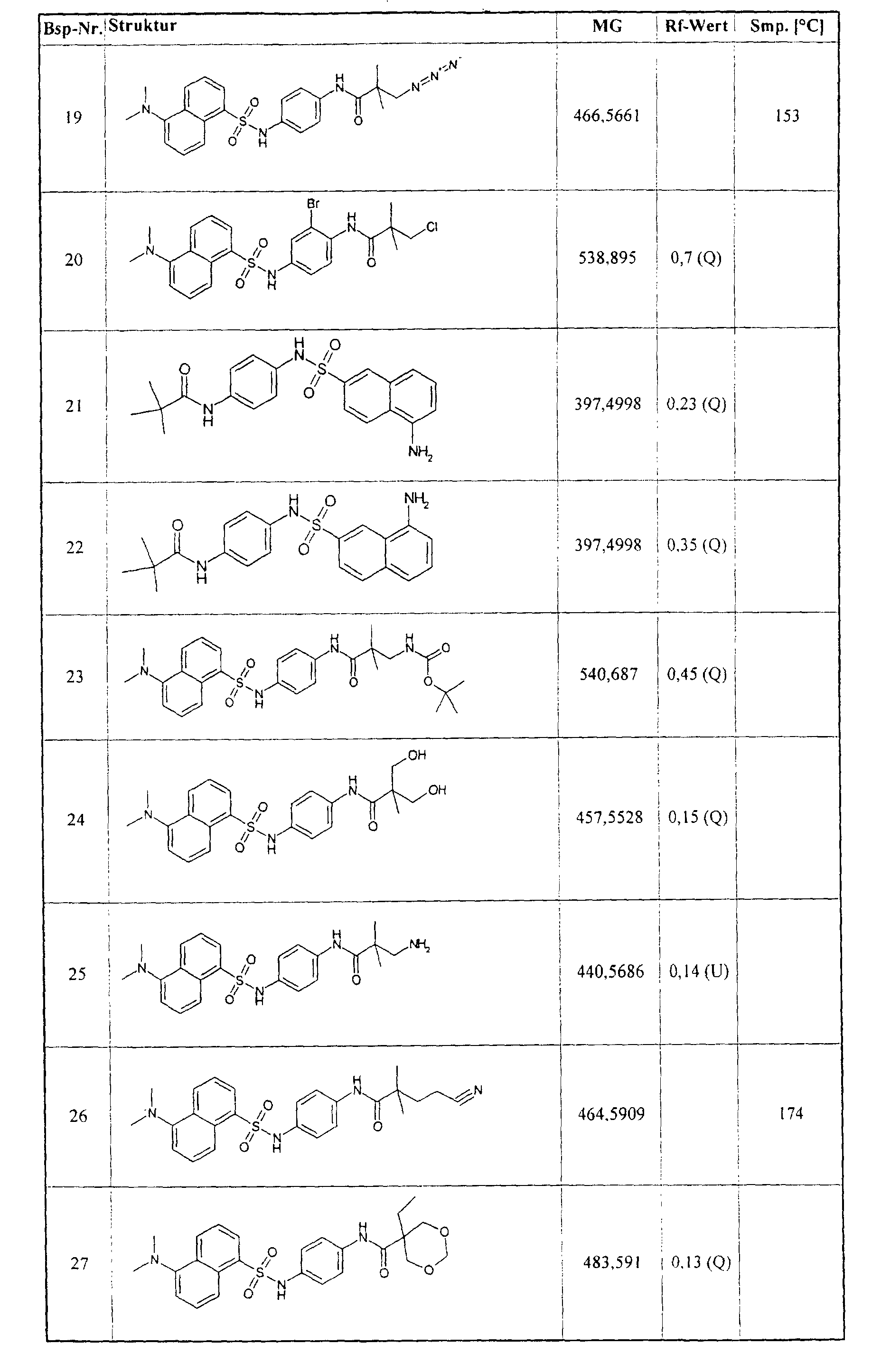

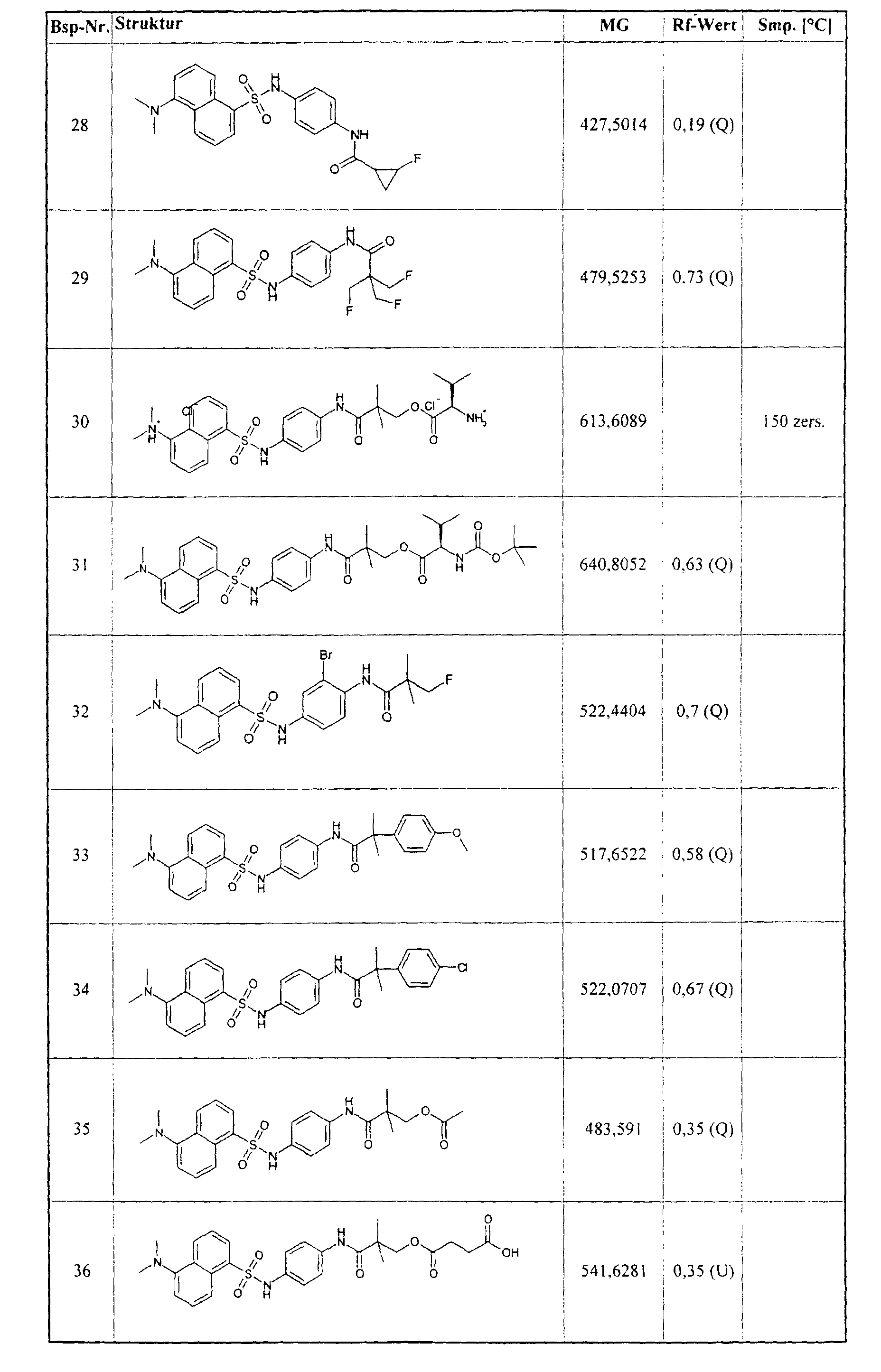

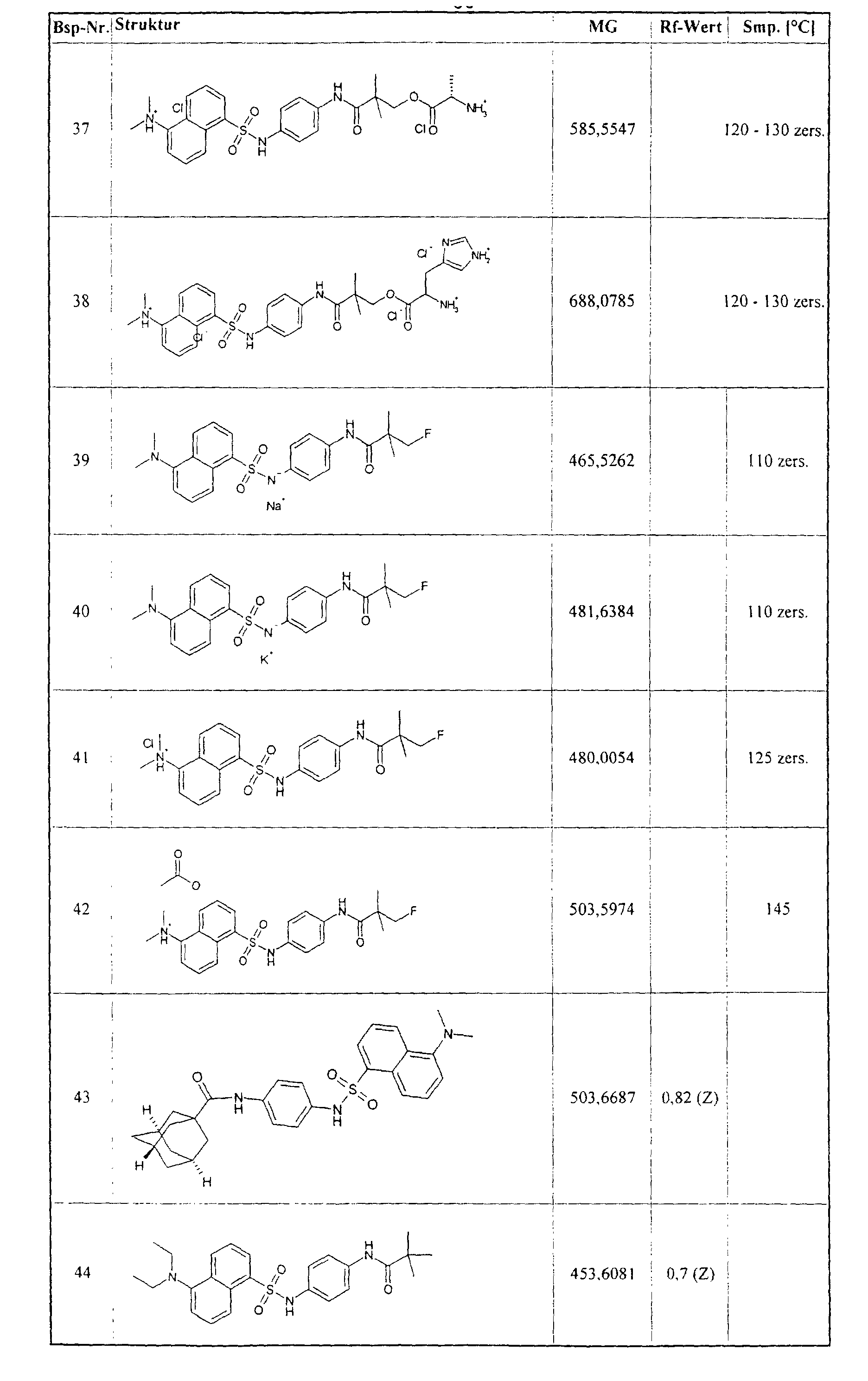

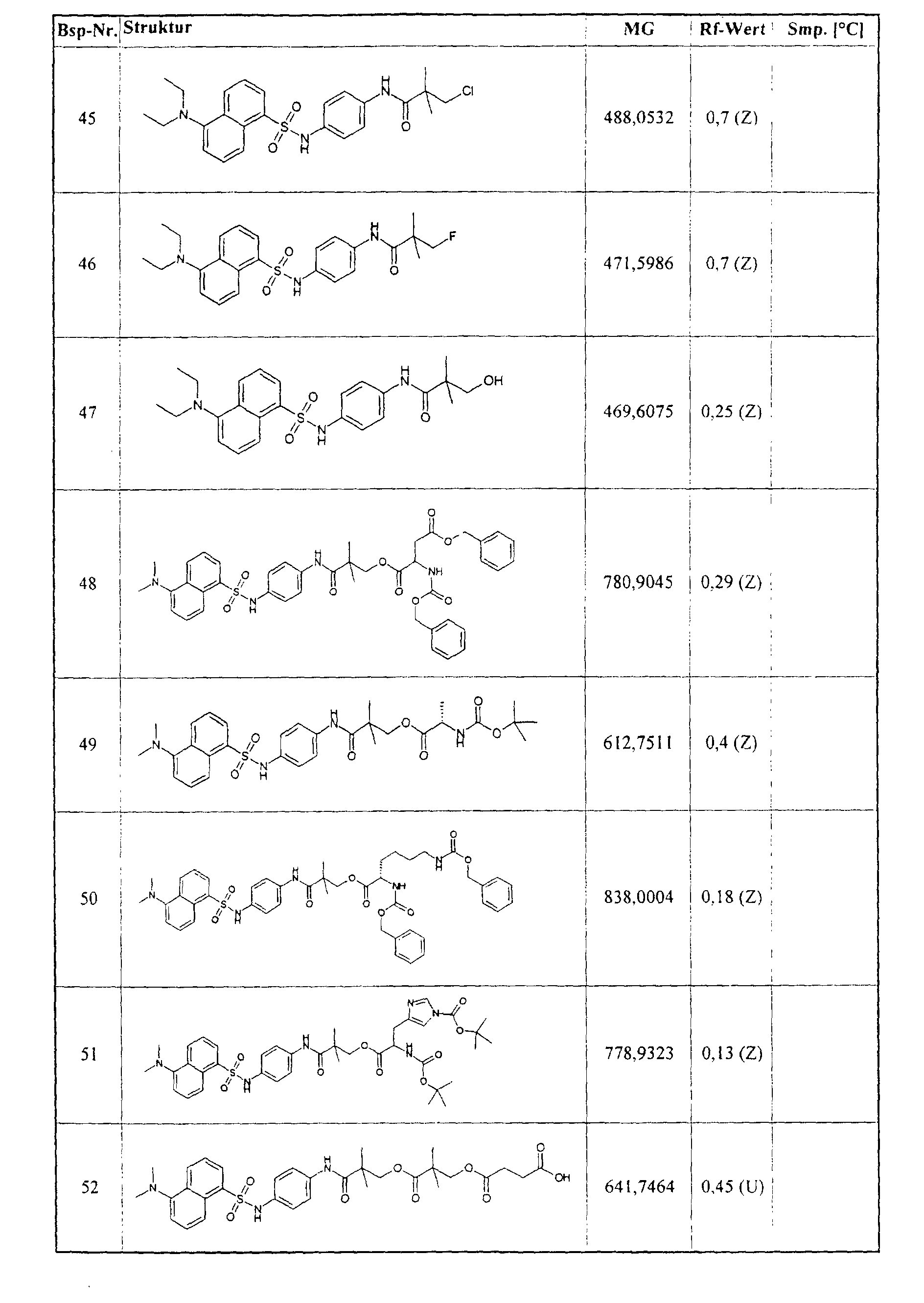

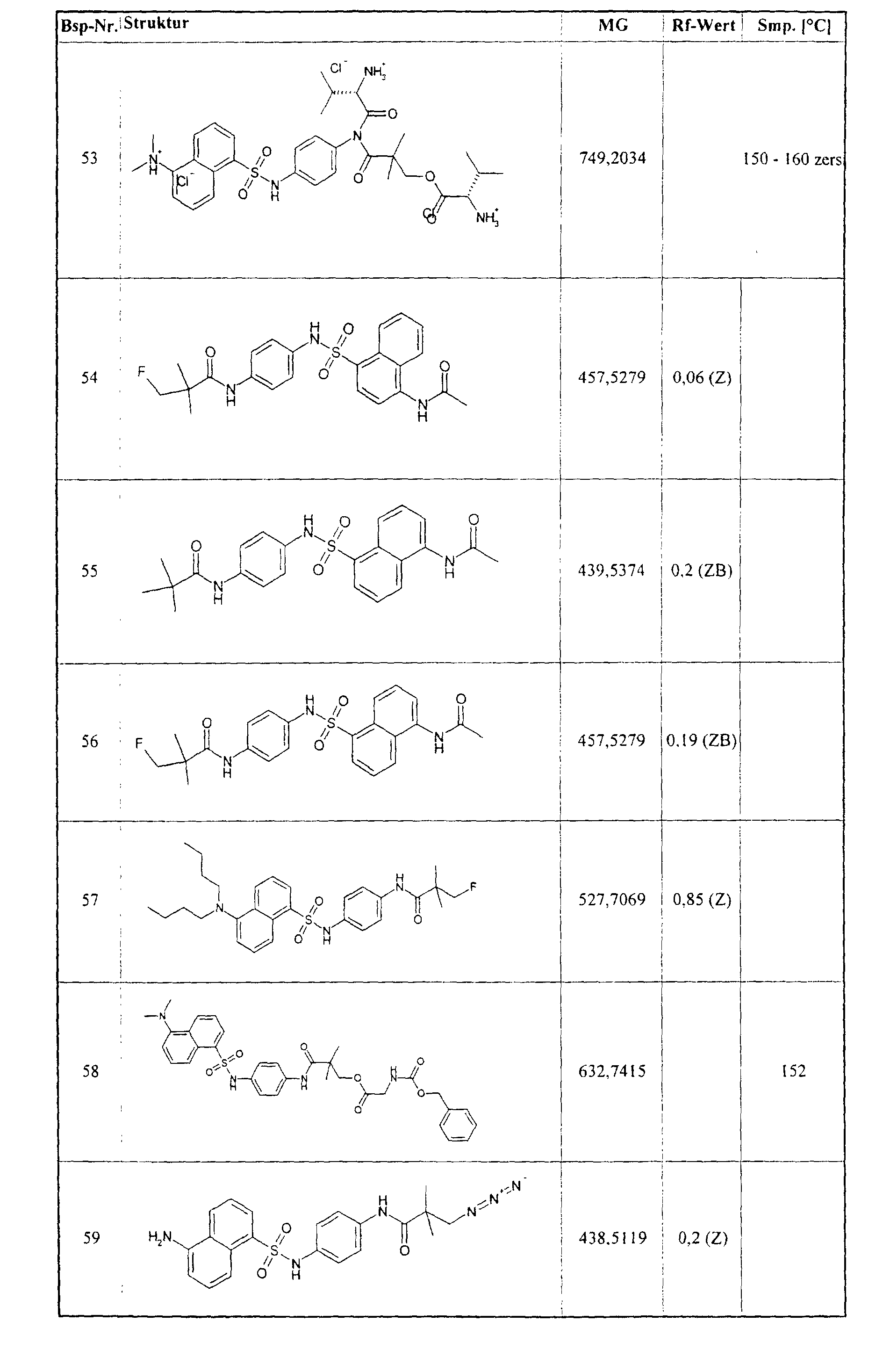

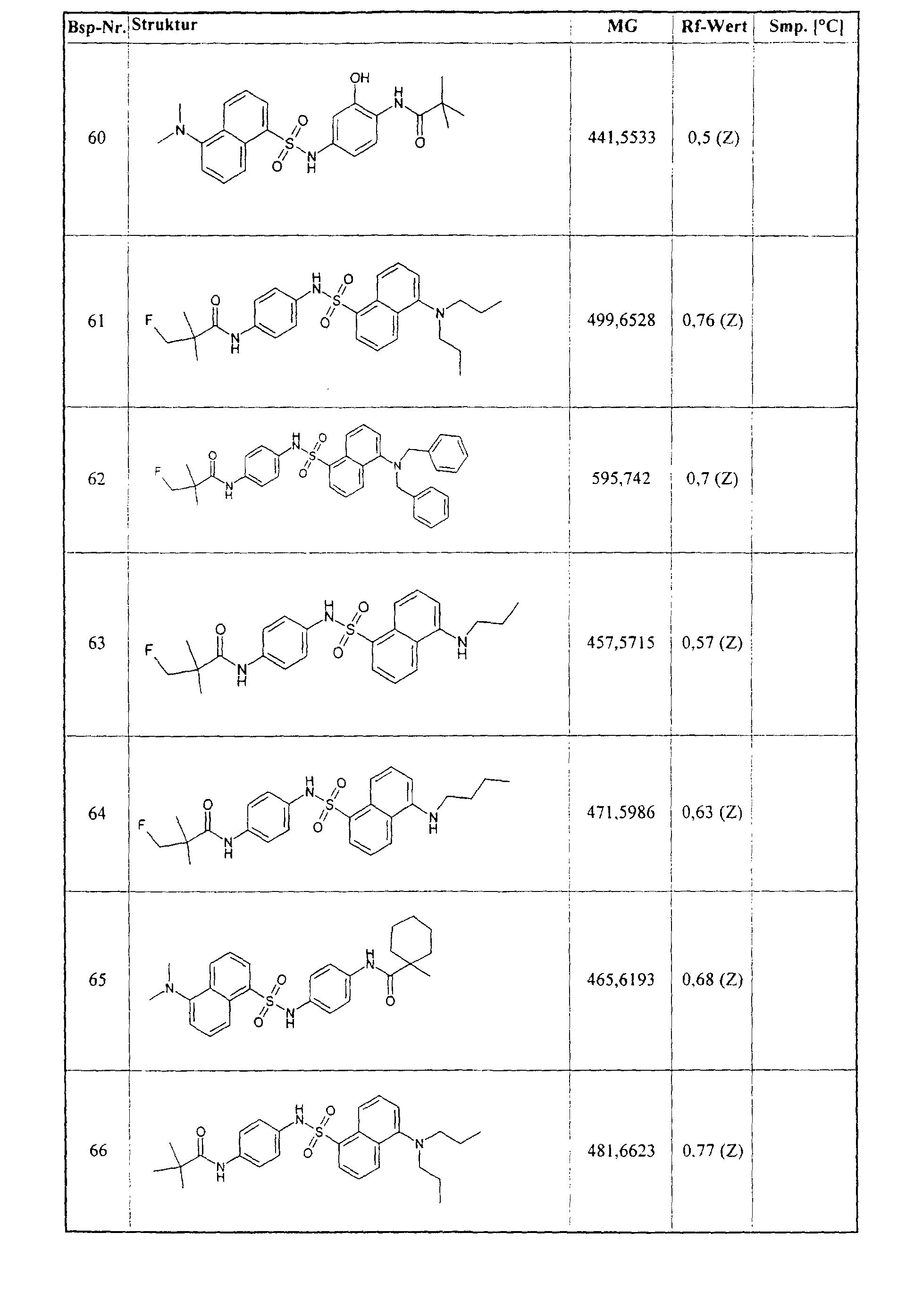

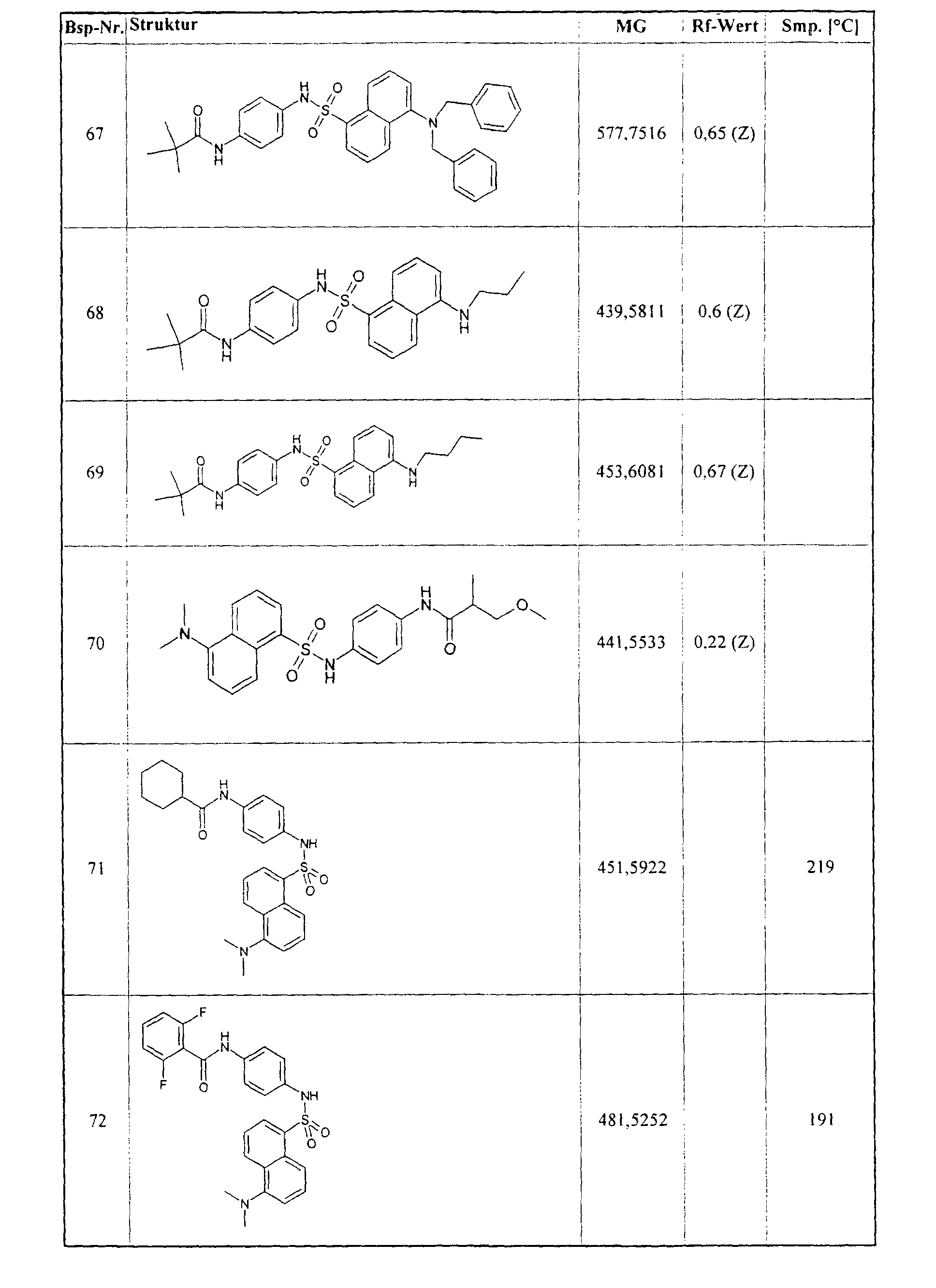

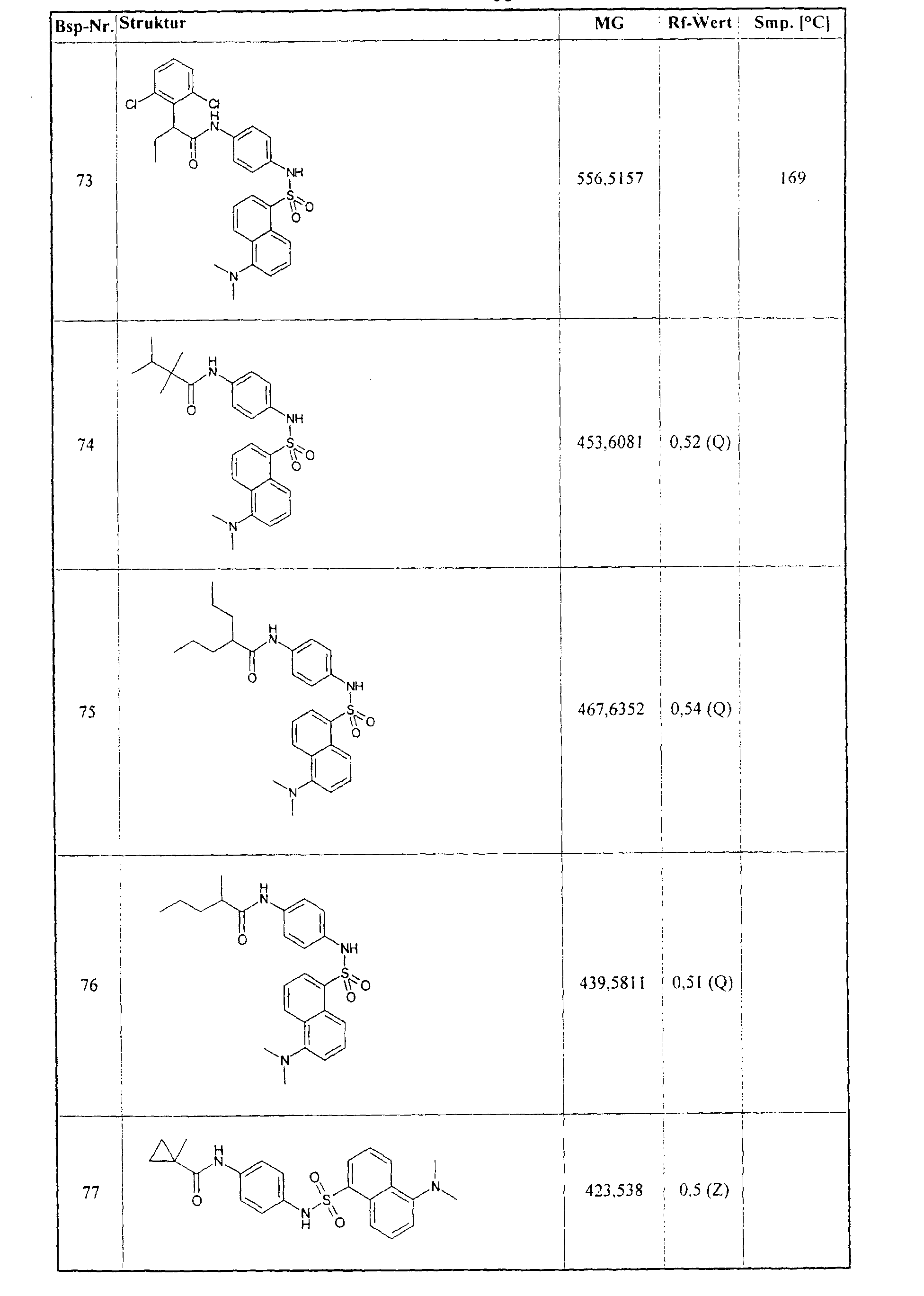

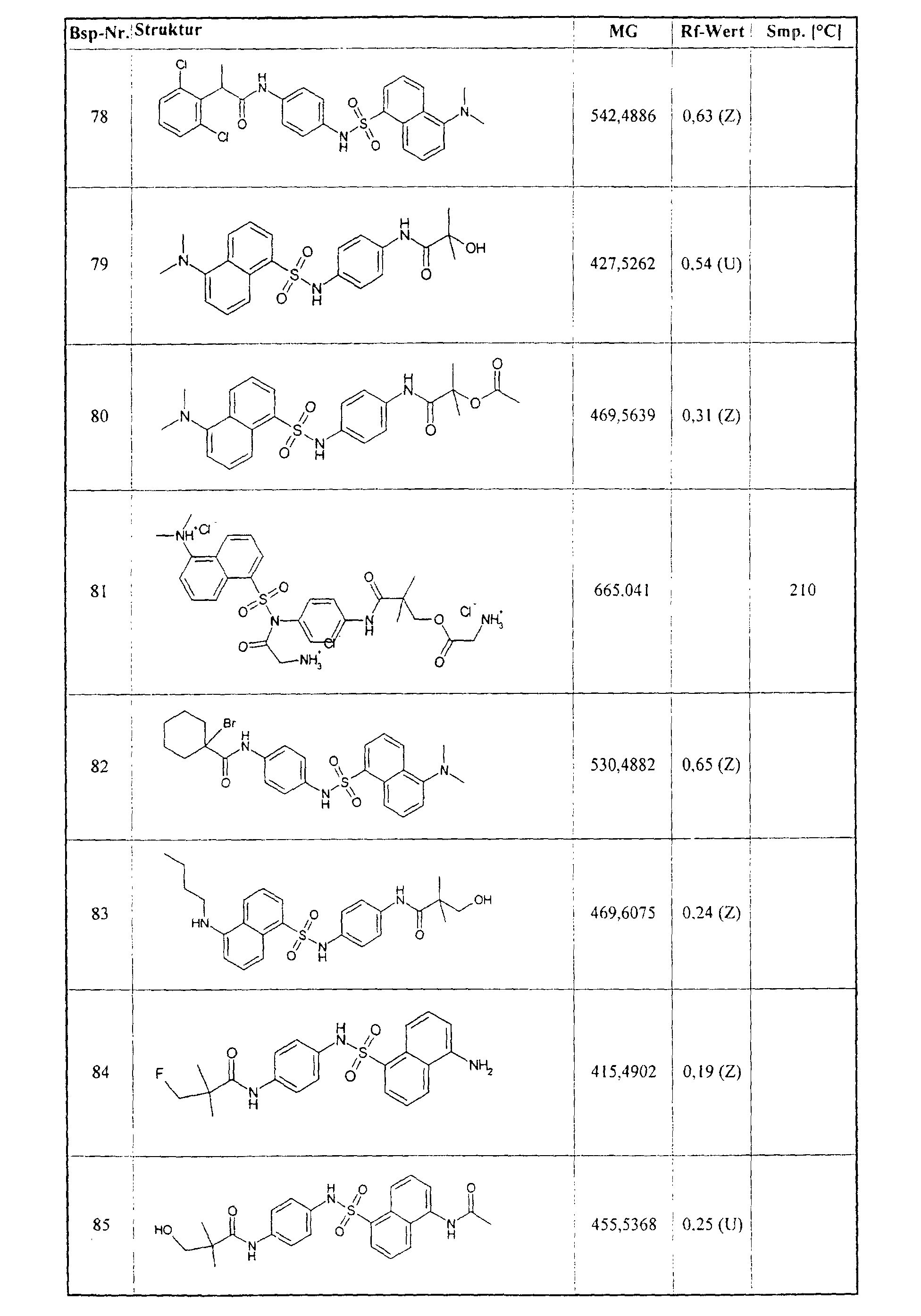

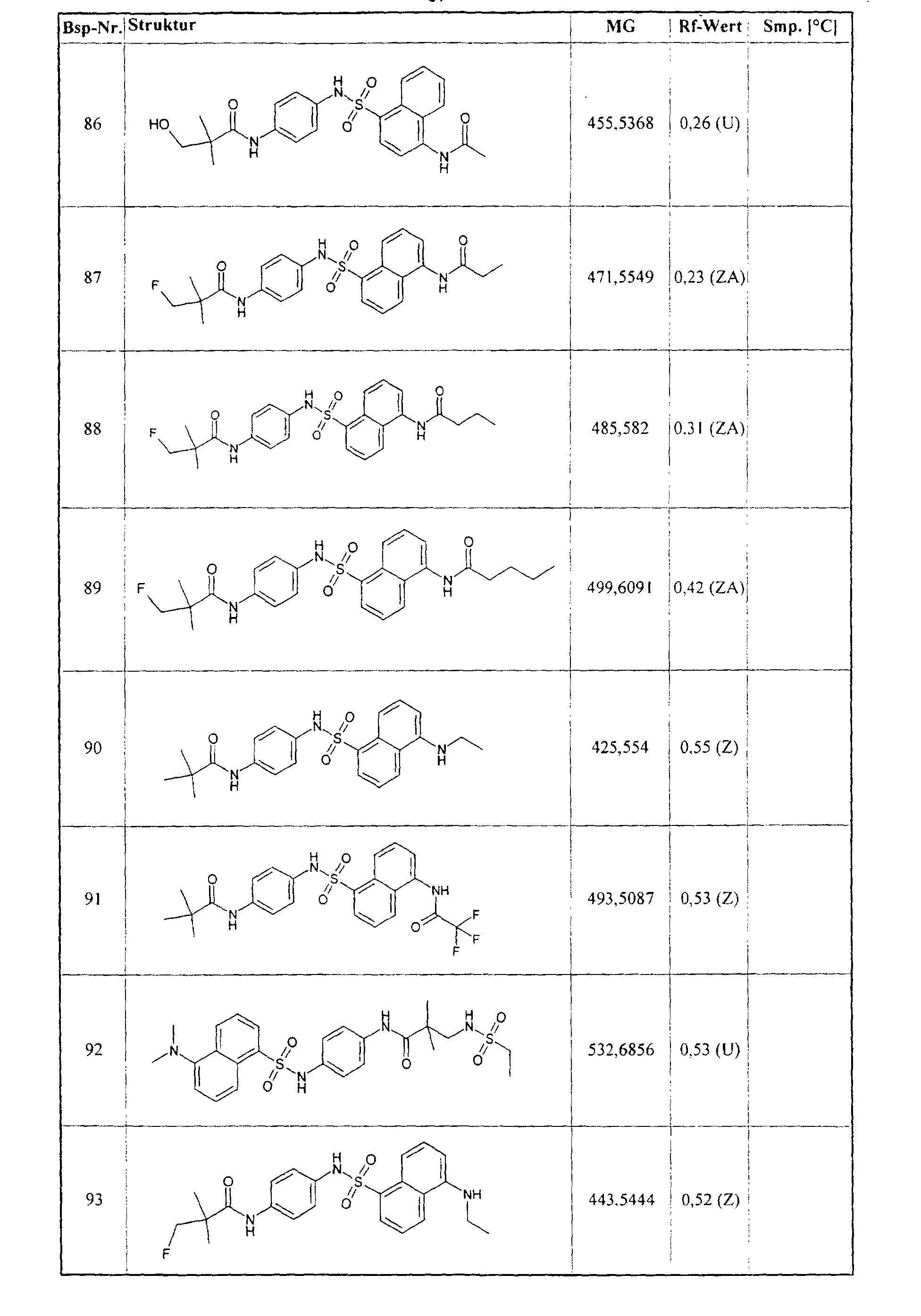

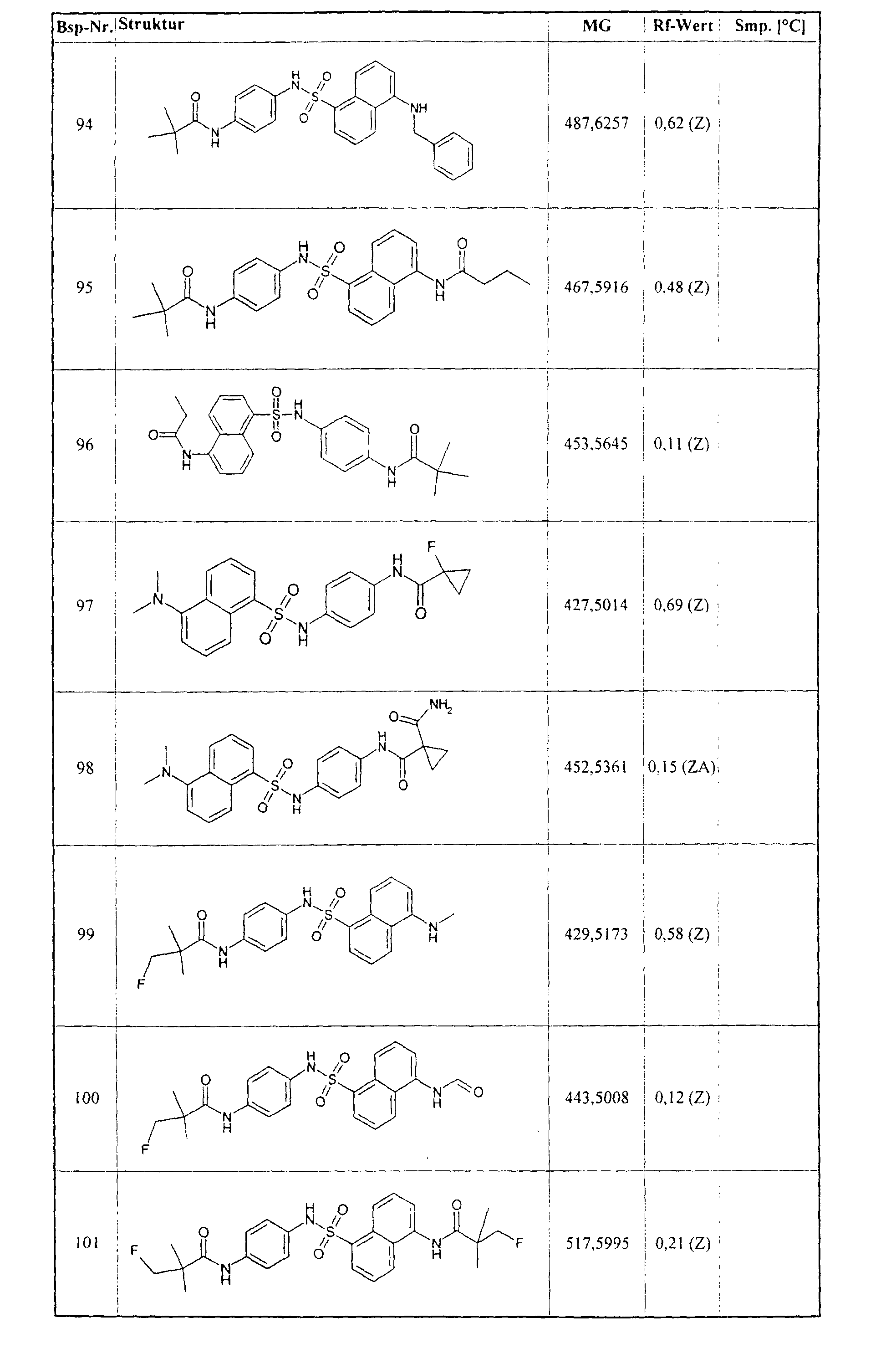

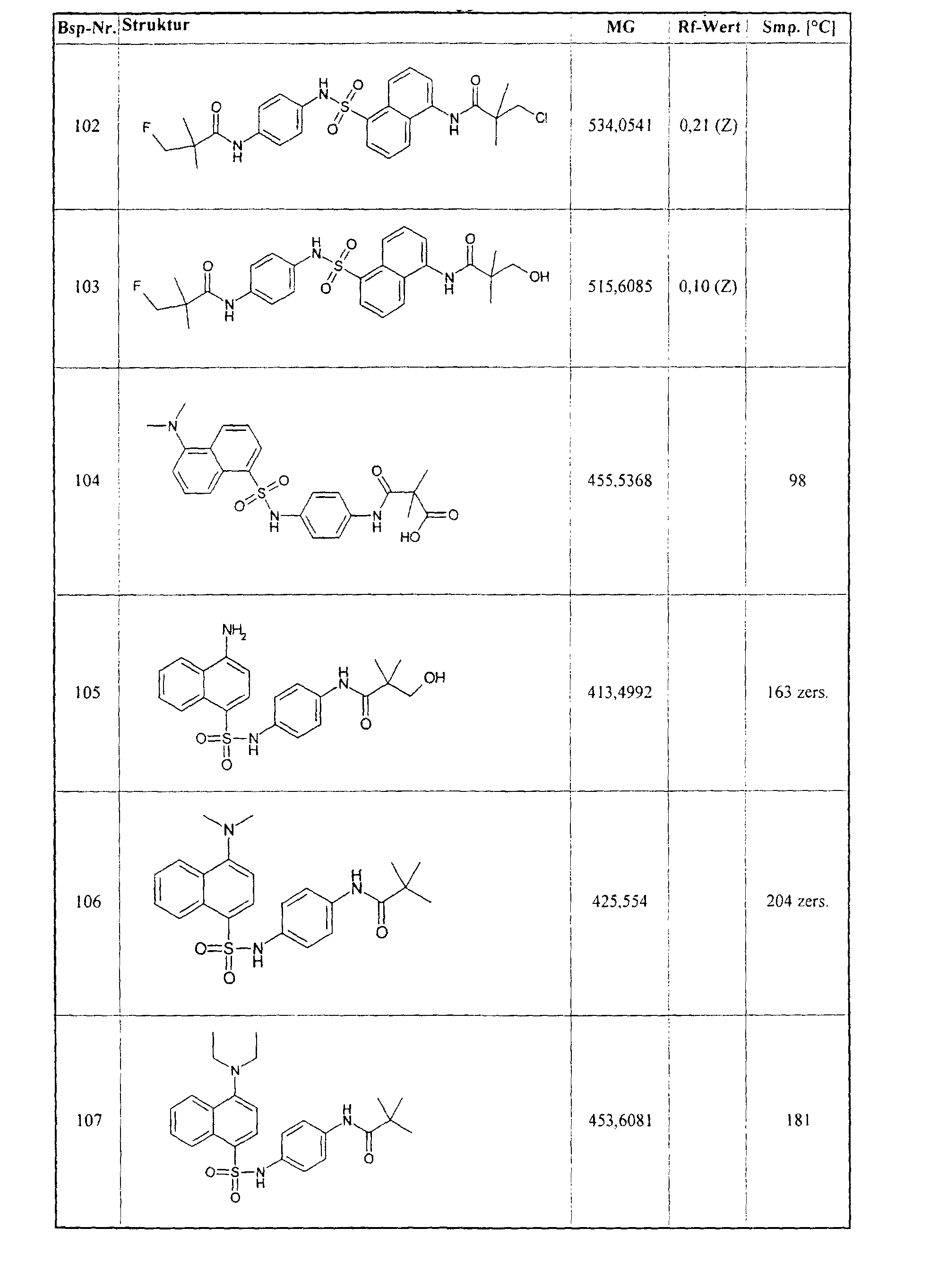

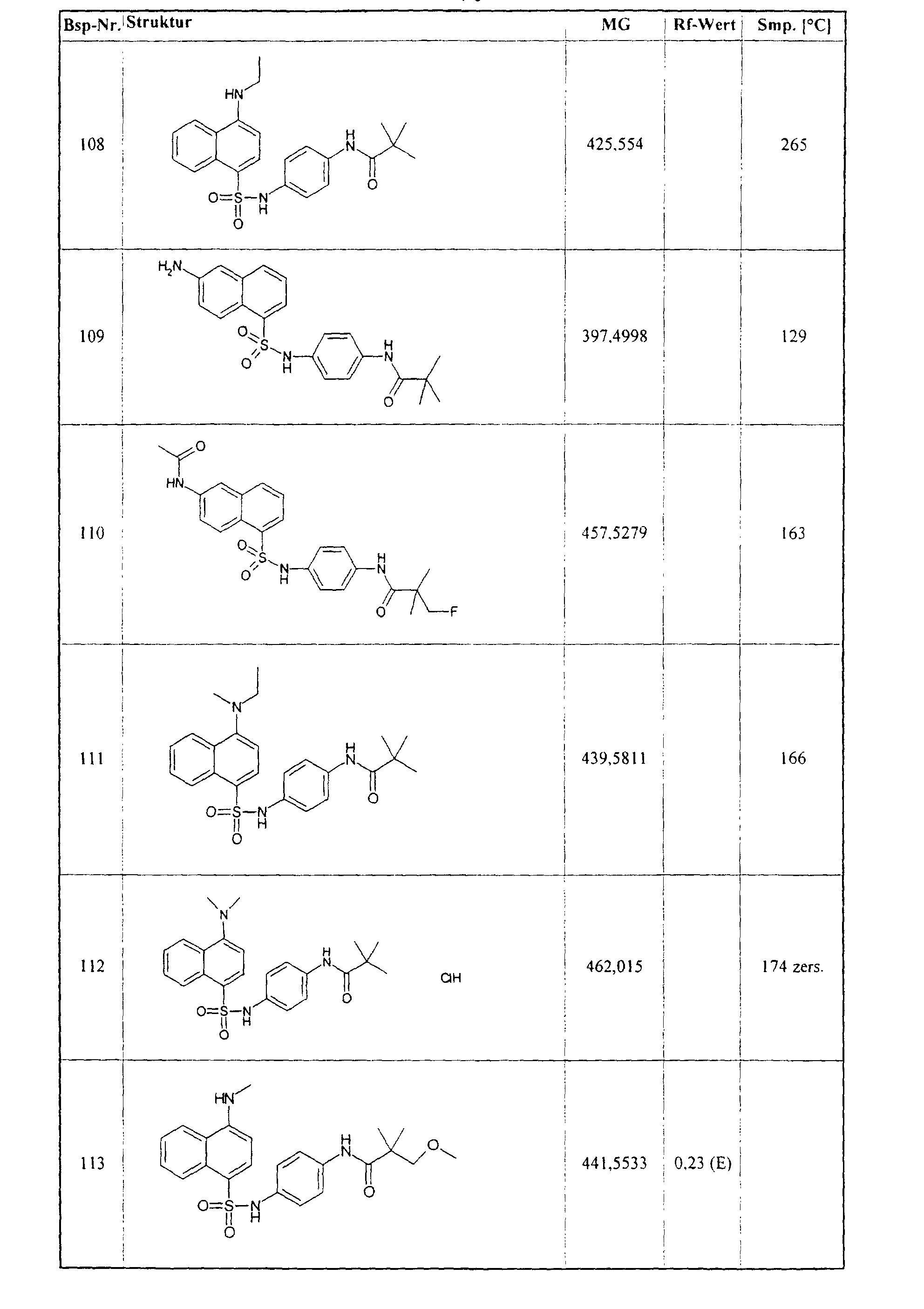

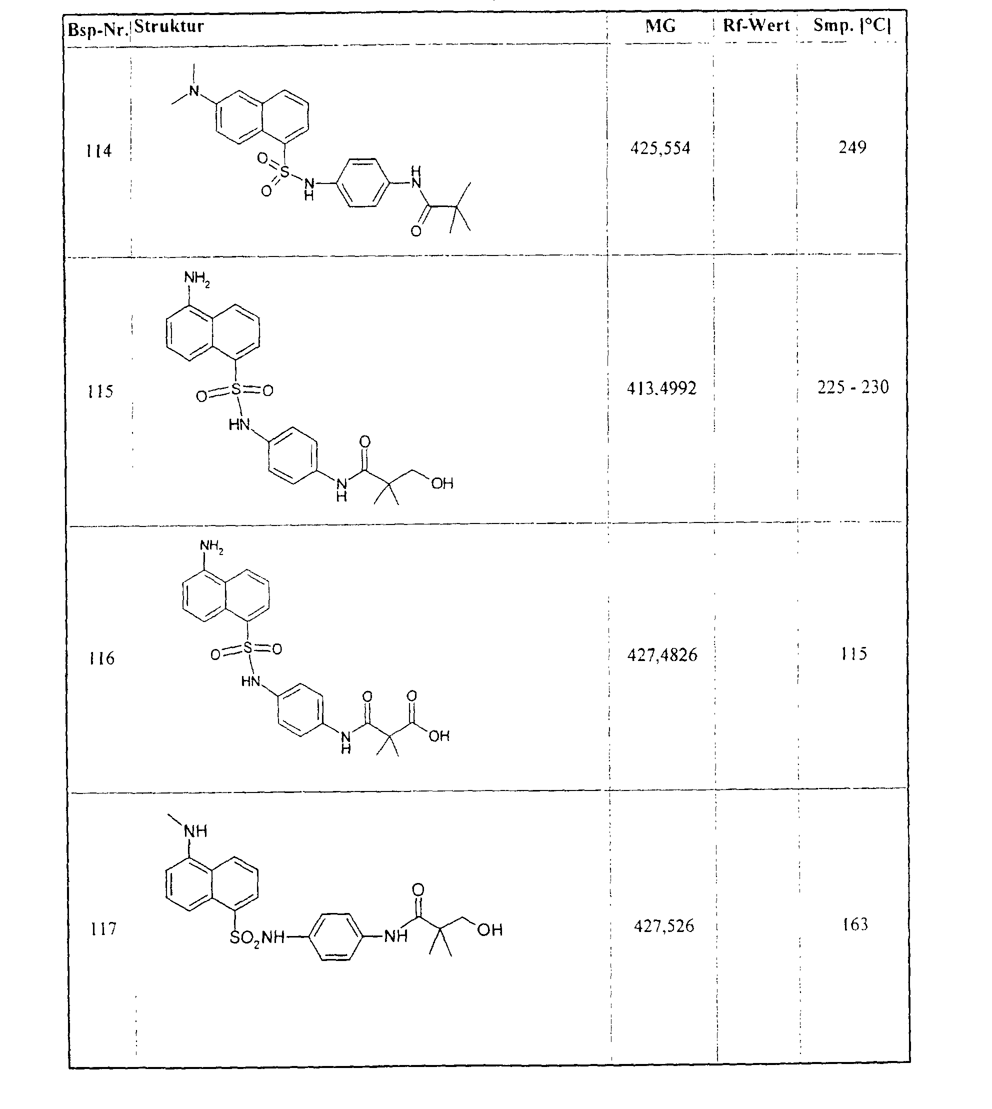

## Patentansprüche

1. Sulfonamide der allgemeinen Formel (I) 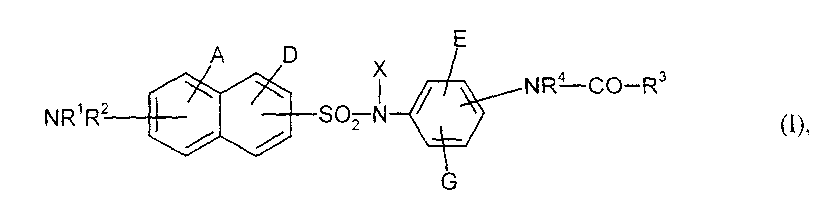 in welcher
R¹ und R² gleich oder verschieden sind und für Wasserstoff, Formyl, gegebenenfalls durch ein bis drei Halogenatome substituiertes Phenyl oder Benzyl, oder geradkettiges oder verzweigtes Alkyl oder Acyl mit jeweils bis zu 6 Kohlenstoffatomen stehen, wobei Alkyl oder Acyl gegebenenfalls durch ein bis drei Substituenten ausgewählt aus Halogen und Hydroxy substituiert sein können,
A, D, E und G gleich oder verschieden sind und für Wasserstoff, Halogen, Nitro, Cyano, Hydroxy, Carboxyl, Trifluormethyl, Trifluormethoxy oder für geradkettiges oder verzweigtes Alkyl, Alkoxy oder Alkoxycarbonyl mit jeweils bis zu 5 Kohlenstoffatomen stehen,
R³ für geradkettiges oder verzweigtes Alkenyl mit bis zu 6 Kohlenstoffatomen steht, oder
für geradkettiges oder verzweigtes Alkyl mit bis zu 8 Kohlenstoffatomen steht, das gegebenenfalls eine Aminogruppe trägt, die durch Alkyl mit bis zu 4 Kohlenstoffatomen oder durch eine Aminoschutzgruppe substituiert sein kann, oder das Alkyl gegebenenfalls ein- bis 3-fach gleich oder verschieden durch Hydroxy, Cyano, Halogen, Azido, Nitro, Trifluormethyl, Carboxyl oder Phenyl substituiert ist, das seinerseits bis zu 2-fach, gleich oder verschieden durch Nitro, Halogen, Hydroxy oder durch geradkettiges oder verzweigtes Alkyl oder Alkoxy mit bis zu 4 Kohlenstoffatomen substituiert sein kann, oder
R³ für Reste der Formeln 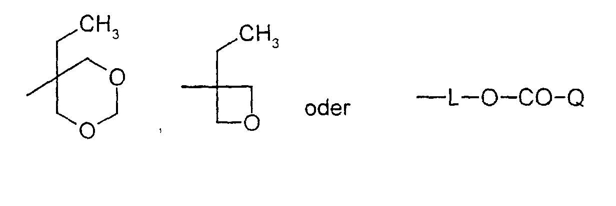 steht,
worin
L für eine geradkettige oder verzweigte Alkandiylgruppe mit bis zu 6 Kohlenstoffatomen steht,
Q für Alkyl mit bis zu 6 Kohlenstoffatomen steht, das gegebenenfalls durch Carboxyl substituiert ist, oder
für Reste der Formeln 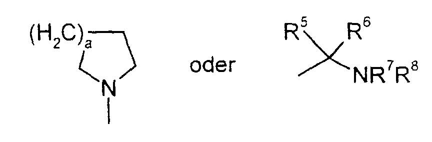 steht,
worin
a die Zahl 1 oder 2 bedeutet,
R⁵ Wasserstoff bedeutet,
R⁶ Cycloalkyl mit 3 bis 8 Kohlenstoffatomen oder Aryl mit 6 bis 10 Kohlenstoffatomen oder Wasserstoff bedeutet, oder
geradkettiges oder verzweigtes Alkyl mit bis zu 8 Kohlenstoffatomen bedeutet,
wobei das Alkyl gegebenenfalls durch Cyano, Methylthio, Hydroxy, Mercapto, Guanidyl oder durch eine Gruppe der Formel NR⁹R¹⁰ oder -R¹¹-OC- substituiert ist,
worin
R⁹ und R¹⁰ unabhängig voneinander Wasserstoff, geradkettiges oder verzweigtes Alkyl mit bis zu 8 Kohlenstoffatomen oder Phenyl bedeuten,
und
R¹¹ Hydroxy, Benzyloxy, Alkoxy mit bis zu 6 Kohlenstoffatomen oder die oben aufgeführte Gruppe -NR⁹R¹⁰ bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe -NR⁹R¹⁰ substituiert ist,
worin
R⁹ und R¹⁰ die oben angegebene Bedeutung haben,
oder das Alkyl gegebenenfalls durch einen 5- bis 6-gliedrigen stickstoffhaltigen Heterocyclus oder durch Indolyl substituiert ist, worin die entsprechenden -NH-Funktionen gegebenenfalls durch Alkyl mit bis zu 6 Kohlenstoffatomen substituiert oder durch eine Aminoschutzgruppe geschützt sind,
R⁷ und R⁸ gleich oder verschieden sind und Wasserstoff oder eine Aminoschutzgruppe bedeuten,
R⁴ für Wasserstoff oder für einen Rest der Formel 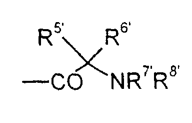 steht,
worin
R⁵', R⁶', R^{7'} und R⁸' die oben angegebene Bedeutung von R⁵, R⁶, R⁷ und R⁸ haben und mit dieser gleich oder verschieden sind,
X die oben angegebene Bedeutung von R⁴ besitzt und von dieser Bedeutung gleich oder verschieden sein kann,
und deren Stereoisomere, stereoisomeren Gemische und Salze,
mit Ausnahme von N-[4-[[[5-(Dimethylamino)-1-naphthalenyl]sulfonyl]amino]phenyl]-acetamid.

2. Sulfonamide der allgemeinen Formel (I) nach Anspruch 1, worin
R¹ und R² gleich oder verschieden sind und für Wasserstoff, Phenyl oder geradkettiges oder verzweigtes Alkyl oder Acyl mit jeweils bis zu 6 Kohlenstoffatomen stehen,
A, D, E und G gleich oder verschieden sind und für Wasserstoff, Halogen, Nitro, Cyano, Hydroxy, Carboxyl, Trifluormethyl, Trifluormethoxy oder für geradkettiges oder verzweigtes Alkyl, Alkoxy oder Alkoxycarbonyl mit jeweils bis zu 5 Kohlenstoffatomen stehen,
R³ für geradkettiges oder verzweigtes Alkenyl mit bis zu 6 Kohlenstoffatomen steht, oder
für geradkettiges oder verzweigtes Alkyl mit bis zu 8 Kohlenstoffatomen steht, das gegebenenfalls eine Aminogruppe trägt, die durch Alkyl mit bis zu 4 Kohlenstoffatomen oder durch eine Aminoschutzgruppe substituiert sein kann, oder das Alkyl gegebenenfalls ein- bis 3-fach gleich oder verschieden durch Hydroxy, Cyano, Halogen, Azido, Nitro, Trifluormethyl, Carboxyl oder Phenyl substituiert ist, das seinerseits bis zu 2-fach, gleich oder verschieden durch Nitro, Halogen, Hydroxy oder durch geradkettiges oder verzweigtes Alkyl oder Alkoxy mit bis zu 4 Kohlenstoffatomen substituiert sein kann, oder
R³ für Reste der Formeln 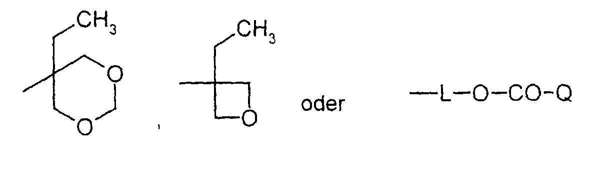 steht,
worin
L für eine geradkettige oder verzweigte Alkandiylgruppe mit bis zu 6 Kohlenstoffatomen steht,
Q für Alkyl mit bis zu 6 Kohlenstoffatomen steht, das gegebenenfalls durch Carboxyl substituiert ist, oder
für Reste der Formeln 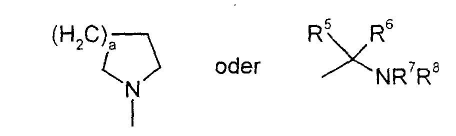 steht,
worin
a die Zahl 1 oder 2 bedeutet,
R⁵ Wasserstoff bedeutet,
R⁶ Cycloalkyl mit 3 bis 8 Kohlenstoffatomen oder Aryl mit 6 bis 10 Kohlenstoffatomen oder Wasserstoff bedeutet, oder
geradkettiges oder verzweigtes Alkyl mit bis zu 8 Kohlenstoffatomen bedeutet,
wobei das Alkyl gegebenenfalls durch Cyano, Methylthio, Hydroxy, Mercapto, Guanidyl oder durch eine Gruppe der Formel -NR⁹R¹⁰ oder R¹¹-OC- substituiert ist,
worin
R⁹ und R¹⁰ unabhängig voneinander Wasserstoff, geradkettiges oder verzweigtes Alkyl mit bis zu 8 Kohlenstoffatomen oder Phenyl bedeuten,
und
R¹¹ Hydroxy, Benzyloxy, Alkoxy mit bis zu 6 Kohlenstoffatomen oder die oben aufgeführte Gruppe -NR⁹R¹⁰ bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe -NR⁹R¹⁰ substituiert ist,
worin
R⁹ und R¹⁰ die oben angegebene Bedeutung haben,
oder das Alkyl gegebenenfalls durch einen 5- bis 6-gliedrigen stickstoffhaltigen Heterocyclus oder durch Indolyl substituiert ist, worin die entsprechenden -NH-Funktionen gegebenenfalls durch Alkyl mit bis zu 6 Kohlenstoffatomen substituiert oder durch eine Aminoschutzgruppe geschützt sind,
R⁷ und R⁸ gleich oder verschieden sind und Wasserstoff oder eine Aminoschutzgruppe bedeuten,
R⁴ für Wasserstoff oder für einen Rest der Formel 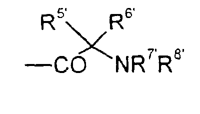 steht,
worin
R^{5'}, R^{6'}, R^{7'} und R^{8'} die oben angegebene Bedeutung von R⁵, R⁶, R⁷ und R⁸ haben und mit dieser gleich oder verschieden sind, und
X für Wasserstoff steht,
und deren Stereoisomere, stereoisomeren Gemische und Salze,
mit Ausnahme von N-[4-[[[5-(Dimethylamino)-1-naphthalenyl]sulfonyl]amino]phenyl]-acetamid.

3. Sulfonamide der allgemeinen Formel (I) nach Anspruch 1 oder 2, worin
R³ für geradkettiges oder verzweigtes Alkenyl mit bis zu 6 Kohlenstoffatomen steht, oder
für geradkettiges oder verzweigtes Alkyl mit bis zu 8 Kohlenstoffatomen steht, worin das Alkyl eine Aminogruppe trägt, die durch Alkyl mit bis zu 4 Kohlenstoffatomen oder durch eine Aminoschutzgruppe substituiert sein kann, oder das Alkyl ein- bis 3-fach gleich oder verschieden durch Hydroxy, Cyano, Halogen, Azido, Nitro, Trifluormethyl, Carboxyl oder Phenyl substituiert ist, das seinerseits bis zu 2-fach, gleich oder verschieden durch Nitro, Halogen, Hydroxy oder durch geradkettiges oder verzweigtes Alkyl oder Alkoxy mit bis zu 4 Kohlenstoffatomen substituiert sein kann, oder
R³ für Reste der Formeln 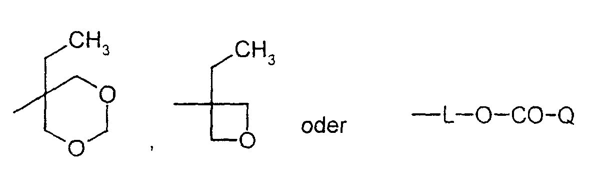 steht,
worin L und Q wie oben definiert sind.

4. Sulfonamide der allgemeinen Formel (I) gemäß Anspruch 1 oder 2, worin
R¹ und R² gleich oder verschieden sind und für Wasserstoff. Phenyl oder geradkettiges oder verzweigtes Alkyl oder Acyl mit jeweils bis zu 5 Kohlenstoffatomen stehen,
A, D, E und G gleich oder verschieden sind und für Wasserstoff, Fluor, Chlor, Brom, Nitro, Cyano, Hydroxy oder für geradkettiges oder verzweigtes Alkyl, Alkoxy oder Alkoxycarbonyl mit jeweils bis zu 3 Kohlenstoffatomen stehen,
R³ für geradkettiges oder verzweigtes Alkenyl mit bis zu 5 Kohlenstoffatomen steht, oder
für geradkettiges oder verzweigtes Alkyl mit bis zu 7 Kohlenstoffatomen steht, das gegebenenfalls eine Aminogruppe trägt, die durch Alkyl mit bis zu 3 Kohlenstoffatomen, tert.-Butyloxycarbonyl oder Benzyloxycarbonyl substituiert sein kann, oder das Alkyl gegebenenfalls ein- bis 3-fach, gleich oder verschieden durch Hydroxy, Cyano, Fluor, Chlor, Azido, Nitro, Trifluormethyl oder Phenyl substituiert ist, das seinerseits bis zu 2-fach gleich oder verschieden durch Nitro. Fluor, Chlor, Hydroxy oder durch geradkettiges oder verzweigtes Alkyl oder Alkoxy mit bis zu 3 Kohlenstoffatomen substituiert sein kann,oder
R³ für Reste der Formeln 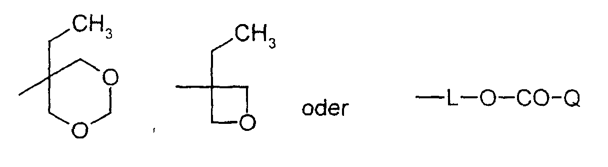 steht,
worin
L für eine geradkettige oder verzweigte Alkandiylgruppe mit bis zu 4 Kohlenstoffatomen steht,
Q für Alkyl mit bis zu 4 Kohlenstoffatomen steht, das gegebenenfalls durch Carboxyl substituiert ist, oder für Reste der Formeln 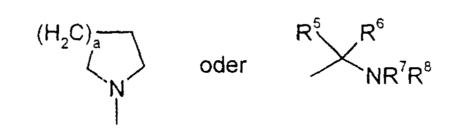
steht,
worin
a die Zahl 1 oder 2 bedeutet,
R⁵ Wasserstoff bedeutet,
R⁶ Cyclopentyl, Cyclohexyl, Phenyl oder Wasserstoff bedeutet, oder
geradkettiges oder verzweigtes Alkyl mit bis zu 6 Kohlenstoffatomen bedeutet,
wobei das Alkyl gegebenenfalls durch Cyano, Methylthio, Hydroxy, Mercapto, Guanidyl, Amino, Carboxy oder H₂N-CO- substituiert sein kann,
oder das Alkyl durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann,
oder das Alkyl durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen substituiert sind oder durch tert.-Butyloxycarbonyl oder Benzyloxycarbonyl, geschützt sind,
R⁷ und R⁸ gleich oder verschieden sind und Wasserstoff, tert- Butyloxycarbonyl oder Benzyloxycarbonyl bedeuten,
R⁴ für Wasserstoff oder für einen Rest der Formel 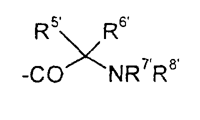 steht,
worin
R^{5'}, R^{6'}, R^{7'} und R^{8'} die oben angegebene Bedeutung von R⁵, R⁶, R⁷ und R⁸ haben und mit dieser gleich oder verschieden sind,
und deren Stereoisomeren, stereoisomeren Gemische und Salze,
mit Ausnahme von N-[4-[[[5-(Dimethylamino)-1-naphthalenyl]sulfonyl]amino]phenyl]-acetamid.

5. Sulfonamide der allgemeinen Formel (I) gemäß Anspruch 4, worin
R³ für geradkettiges oder verzweigtes Alkenyl mit bis zu 5 Kohlenstoffatomen steht, oder
für geradkettiges oder verzweigtes Alkyl mit bis zu 7 Kohlenstoffatomen steht, worin das Alkyl eine Aminogruppe trägt, die durch Alkyl mit bis zu 3 Kohlenstoffatomen, tert.-Butyloxycarbonyl oder Benzyloxycarbonyl substituiert sein kann, oder das Alkyl ein- bis 3-fach, gleich oder verschieden durch Hydroxy, Cyano, Fluor, Chlor, Azido, Nitro, Trifluormethyl oder Phenyl substituiert ist, das seinerseits bis zu 2-fach gleich oder verschieden durch Nitro, Fluor, Chlor, Hydroxy oder durch geradkettiges oder verzweigtes Alkyl oder Alkoxy mit bis zu 3 Kohlenstoffatomen substituiert sein kann, oder
R³ für Reste der Formeln 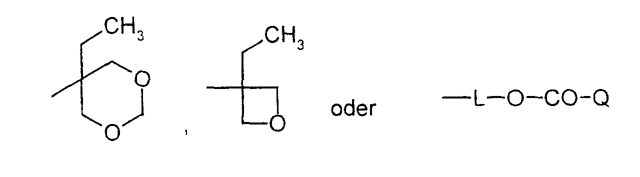 steht,
worin L und Q wie oben definiert sind.

6. Sulfonamide der allgemeinen Formel (I) gemäß Anspruch 1, 2, oder 4, worin
R¹ und R² gleich oder verschieden sind und für Wasserstoff, Phenyl oder geradkettiges oder verzweigtes Alkyl oder Acyl mit jeweils bis zu 4 Kohlenstoffatomen stehen,
A, D, E und G gleich oder verschieden sind und für Wasserstoff, Fluor, Chlor, Brom, Hydroxy, Methyl oder Methoxy stehen,
R³ für geradkettiges oder verzweigtes Alkenyl mit bis zu 4 Kohlenstoffatomen steht, oder
für geradkettiges oder verzweigtes Alkyl mit bis zu 5 Kohlenstoffatomen steht, das gegebenenfalls eine Aminogruppe trägt, die durch tert.-Butyloxycarbonyl oder Benzyloxycarbonyl substituiert sein kann, oder das gegebenenfalls ein- bis 3-fach gleich oder verschieden durch Hydroxy, Cyano, Fluor, Chlor, Nitro, Azido, Trifluormethyl oder Phenyl substituiert ist, das seinerseits bis zu 2-fach gleich oder verschieden, durch Nitro, Fluor, Chlor, Hydroxy, Methyl, Ethyl. Methoxy oder Ethoxy substituiert sein kann, oder
R³ für Reste der Formeln 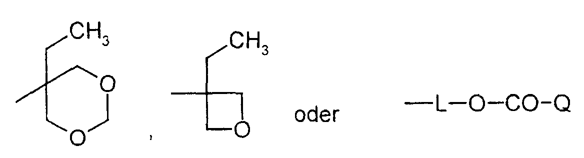 steht,
worin
L für eine geradkettige oder verzweigte Alkandiylgruppe mit bis zu 4 Kohlenstoffatomen steht,
Q für Alkyl mit bis zu 3 Kohlenstoffatomen steht, das gegebenenfalls durch Carboxyl substituiert ist, oder für einen Rest der Formel 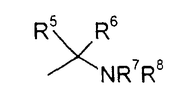 steht,
worin
R⁵ Wasserstoff bedeutet,
R⁶ Cyclopentyl, Cyclohexyl oder Wasserstoff bedeutet, oder
geradkettiges oder verzweigtes Alkyl mit bis zu 4 Kohienstoffatomen bedeutet,
wobei das Alkyl gegebenenfalls durch Cyano, Methylthio, Hydroxy, Mercapto, Guanidyl, Amino, Carboxy oder H₂N-CO- substituiert sein kann,
oder das Alkyl durch Cyclohexyl, Naphthyl oder Phenyl substituiert ist, das seinerseits durch Fluor, Chlor oder Alkoxy mit bis zu 4 Kohlenstoffatomen substituiert sein kann,
oder das Alkyl durch Indolyl, Imidazolyl, Triazolyl, Pyridyl oder Pyrazolyl substituiert ist, wobei die entsprechenden -NH-Funktionen gegebenenfalls durch Methyl substituiert oder durch Benzyloxymethylen oder tert.-Butyloxycarbonyl (BOC) geschützt sind,
R⁷ und R⁸ gleich oder verschieden sind und Wasserstoff oder tert.-Butyloxycarbonyl bedeuten,
R⁴ für Wasserstoff oder für einen Rest der Formel 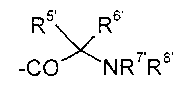 steht,
worin
R^{5'}, R⁶', R^{7'} und R^{8'} die oben angegebene Bedeutung von R⁵, R⁶, R⁷ und R⁸ haben und mit dieser gleich oder verschieden sind,
und deren Stereoisomere, stereoisomeren Gemische und Salze,
mit Ausnahme von N-[4-[[[5-(Dimethylamino)-1-naphmalenyl]sulfonyl]amino]phenyl]-acetamid.

7. Sulfonamide der allgemeinen Formel (I) gemäß irgendeinem der Ansprüche 1 bis 6, worin
R³ für geradkettiges oder verzweigtes Alkenyl mit bis zu 4 Kohlenstoffatomen steht, oder
für geradkettiges oder verzweigtes Alkyl mit bis zu 5 Kohlenstoffatomen steht, worin das Alkyl eine Aminogruppe trägt, die durch tert.- Butyloxycarbonyl oder Benzyloxycarbonyl substituiert sein kann, oder das Alkyl ein- bis 3-fach gleich oder verschieden durch Hydroxy, Cyano, Fluor, Chlor, Nitro, Azido, Trifluormethyl oder Phenyl substituiert ist, das seinerseits bis zu 2-fach gleich oder verschieden, durch Nitro, Fluor, Chlor, Hydroxy, Methyl, Ethyl, Methoxy oder Ethoxy substituiert sein kann, oder
R³ für Reste der Formeln 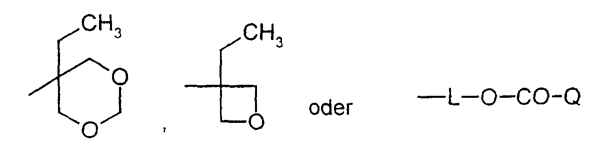 steht,
worin L oder Q wie oben definiert sind,
und deren Stereoisomere, stereoisomeren Gemische und Salze.

8. Sulfonamide der allgemeinen Formel (I) gemäß irgendeinem der Ansprüche 1 bis 7, worin
R¹ und R² für geradkettiges oder verzweigtes Alkyl mit bis zu 4 Kohlenstoffatomen stehen,
A, D, E und G für Wasserstoff, stehen,
R³ für geradkettiges oder verzweigtes Alkyl mit bis zu 5 Kohlenstoffatomen steht, das durch Hydroxy substituiert ist, oder
R³ für einen Rest der Formel
-L-O-CO-Q
steht, worin
L für eine geradkettige oder verzweigte Alkandiylgruppe mit bis zu 4 Kohlenstoffatomen steht,
Q für einen Rest der Formel 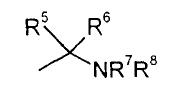 steht,
worin
R⁵ und R⁶ Wasserstoff bedeuten, und
R⁷ und R⁸ Wasserstoff bedeuten, und
R⁴ für Wasserstoff steht,
und deren Stereoisomere, stereoisomeren Gemische und Salze.

9. Sulfonamide der allgemeinen Formel (I) gemäß irgendeinem der Ansprüche 1 bis 8, die ausgewählt sind aus der Gruppe der folgenden Verbindungen : 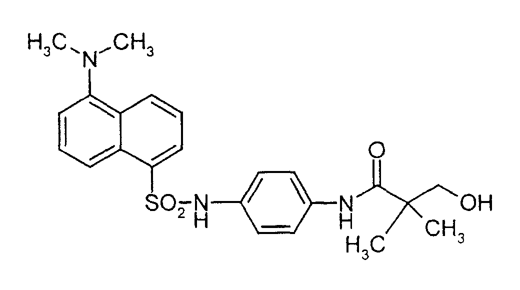 und

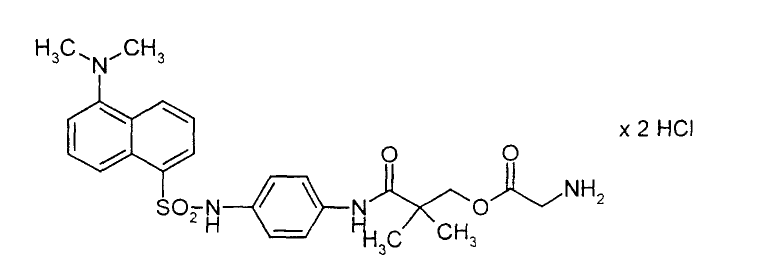

10. Verfahren zur Herstellung von Verbindungen der allgemeinen Formel (1), **dadurch gekennzeichnet, daß** man
[A] Verbindungen der allgemeinen Formel (II) 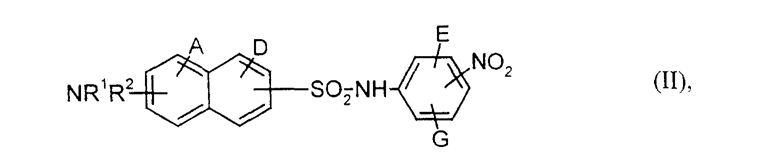 in welcher
A, D,E, G, R¹ und R² die oben angegebene Bedeutung haben,
zunächst durch katalytische Hydrierung an Palladium/C oder durch Reduktion mit SnCl₂ in inerten Lösemitteln in die Verbindungen der allgemeinen Formel (III) 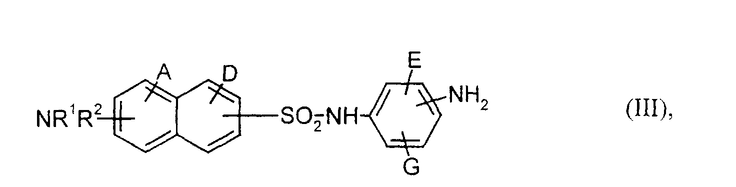 in welcher
A, D, E, G, R¹ und R² die oben angegebene Bedeutung haben, überführt,
und abschließend mit Verbindungen der allgemeinen Formel (IV)
T-CO-R³ (IV)
in welcher
R³ die oben angegebene Bedeutung hat
und
T für Hydroxy, Halogen, vorzugsweise für Chlor steht,
in inerten Lösemitteln, gegebenenfalls in Anwesenheit einer Base und/oder eines Hilfsmittels umsetzt,
oder
[B] Verbindungen der allgemeinen Formel (V) 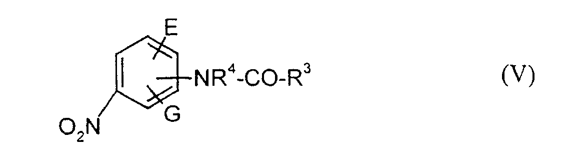 in welcher
E, G, R³ und R⁴ die oben angegebene Bedeutung haben,
zunächst wie unter [A] beschrieben durch Hydrierung an Pd/C oder durch Reduktion mit SnCl₂ in inerten Lösemitteln in die Verbindungen der allgemeinen Formel (VI) 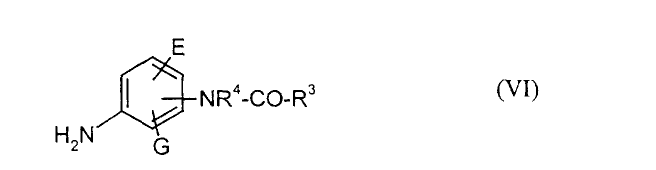 in welcher
E, G, R³ und R⁴ die oben angegebene Bedeutung haben,
überführt
und abschließend mit Verbindungen der allgemeinen Formel (VII) 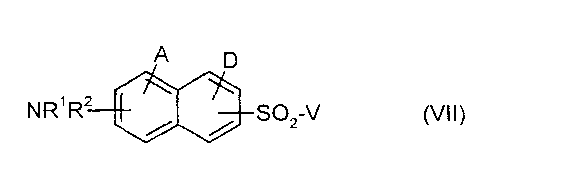 in welcher
A, D, R¹ und R² die oben angegebene Bedeutung haben,
und
V für Halogen, vorzugsweise für Chlor steht,
in inerten Lösemitteln, gegebenenfalls in Anwesenheit einer Base und/oder eines Hilfsmittels umsetzt,
oder
[C] im Fall, daß R³ und/oder R³' für einen Rest der Formel -L-O-CO-Q stehen, Verbindungen der allgemeinen Formel (Ia) 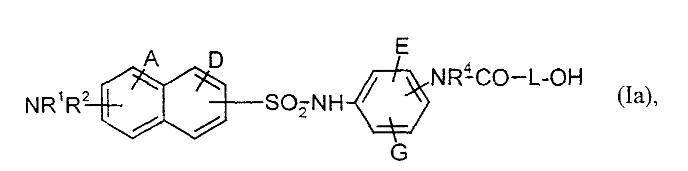
in welcher
R¹, R², R⁴, A, D, E, G und L die oben angegebene Bedeutung haben,
mit Aminosäureresten der allgemeinen Formel (VIII)
HO-CO-Q' (VIII)
in welcher
Q' die oben angegebene Bedeutung von Q hat, wobei einer der dort aufgerührten endständigen Reste am Stickstoff für eine der oben aufgeführten Schutzgruppen, vorzugsweise für tert.-Butyloxycarbonyl steht,
gegebenenfalls unter Aktivierung der Carbonsäure nach üblichen Methoden, in inerten Lösemitteln und in Anwesenheit einer Base und eines Hilfsstoffes umsetzt,
und abschließend die Schutzgruppe nach denen in der Peptid-Chemie üblichen Methoden abspaltet,
und im Fall X, R⁴ ≠ H mit 2 oder mehr Equivalenten der Verbindungen der allgemeinen Formel (VIII) umsetzt,
gegebenenfalls die Stereoisomere nach an sich bekannten Methoden trennt und gegebenenfalls die freien Basen in die Salze oder die Salze in die freien Basen überführt.

11. Sulfonamide der allgemeinen Formel (I) gemäß irgendeinem der Ansprüche 1 bis 9 zur Prophylaxe oder Behandlung von Krankheiten.

12. Verwendung von Sulfonamiden der allgemeinen Formel (I) gemäß irgendeinem der Ansprüche 1 bis 9 zur Herstellung von Arzneimitteln.

13. Verwendung nach Anspruch 12, worin die Arzneimittel zur Bekämpfung viraler Erkrankungen sind.

14. Verwendung nach Anspruch 12 oder 13, worin die Arzneimittel zur Bekämpfung des Cytomegalievirus sind.

15. Arzneimittel enthaltend Sulfonamide der allgemeinen Formel (I) gemäß irgendeinem der Ansprüche 1 bis 9.

## Claims

1. Sulphonamides of the general formula (I) 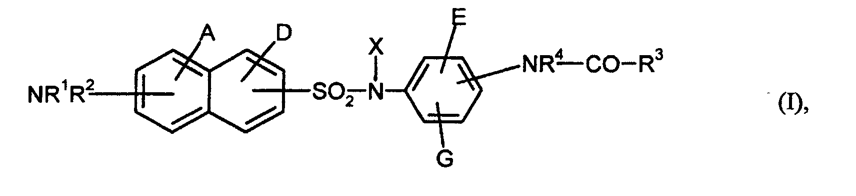 in which
R¹ and R² are identical or different and represent hydrogen, formyl, phenyl or benzyl optionally substituted by one to three halogen atoms, or straight-chain or branched alkyl or acyl each having up to 6 carbon atoms, where alkyl or acyl can optionally be substituted by one to three substituents selected from halogen and hydroxyl,
A, D, E and G are identical or different and represent hydrogen, halogen, nitro, cyano, hydroxyl, carboxyl, trifluoromethyl, trifluoromethoxy or straight-chain or branched alkyl, alkoxy or alkoxycarbonyl each having up to 5 carbon atoms,
R³ represents straight-chain or branched alkenyl having up to 6 carbon atoms, or
represents straight-chain or branched alkyl having up to 8 carbon atoms, which optionally carries an amino group which can optionally be substituted by alkyl having up to 4 carbon atoms or by an amino protective group, or the alkyl is optionally identically or differently substituted one to 3 times by hydroxyl, cyano, halogen, azido, nitro, trifluoromethyl, carboxyl or phenyl which, for its part, can be identically or differently substituted up to 2 times by nitro, halogen, hydroxyl or by straight-chain or branched alkyl or alkoxy having up to 4 carbon atoms, or R³ represents radicals of the formulae 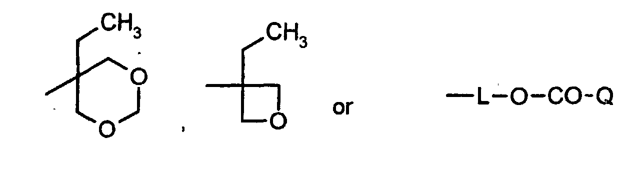 in which
L represents a straight-chain or branched alkanediyl group having up to 6 carbon atoms,
Q represents alkyl having up to 6 carbon atoms, which is optionally substituted by carboxyl, or
represents radicals of the formulae 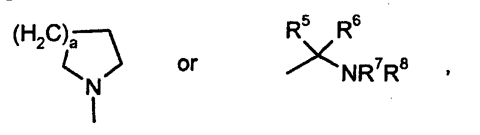 in which
a denotes the number 1 or 2,
R⁵ denotes hydrogen,
R⁶ denotes cycloalkyl having 3 to 8 carbon atoms or aryl having 6 to 10 carbon atoms or hydrogen, or denotes straight-chain or branched alkyl having up to 8 carbon atoms,
where the alkyl is optionally substituted by cyano, methylthio, hydroxyl, mercapto, guanidyl or by a group of the formula -NR⁹R¹⁰ or -R¹¹-OC-,
in which
R⁹ and R¹⁰ independently of one another denote hydrogen, straight-chain or branched alkyl having up to 8 carbon atoms or phenyl,
and
R¹¹ denotes hydroxyl, benzyloxy, alkoxy having up to 6 carbon atoms or the abovementioned group -NR⁹R¹⁰,
or the alkyl is optionally substituted by cycloalkyl having 3 to 8 carbon atoms or by aryl having 6 to 10 carbon atoms, which, for its part, is substituted by hydroxyl, halogen, nitro, alkoxy having up to 8 carbon atoms or by the group -NR⁹R¹⁰, in which
R⁹ and R¹⁰ have the meaning indicated above,
or the alkyl is optionally substituted by a 5- to 6-membered nitrogen-containing heterocycle or by indolyl, in which the corresponding -NH functions are optionally substituted by alkyl having up to 6 carbon atoms or protected by an amino protective group,
R⁷ and R⁸ are identical or different and denote hydrogen or an amino protective group,
R⁴ denotes hydrogen or a radical of the formula 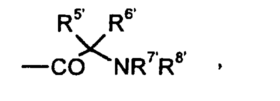 in which
R^{5'}, R^{6'}, R^{7'} and R^{8'} have the meaning of R⁵, R⁶, R⁷ and R⁸ indicated above and are identical to or different from this,
X has the meaning of R⁴ indicated above and can be identical to or different from this meaning,
and their stereoisomers, stereoisomeric mixtures and salts,
with the exception of N-[4-[[[5-(dimethylamino)-1-naphthalenyl]sulphonyl]-amino]phenyl]acetamide.

2. Sulphonamides of the general formula (I) according to Claim 1, in which
R¹ and R² are identical or different and represent hydrogen, phenyl or straight-chain or branched alkyl or acyl each having up to 6 carbon atoms ,
A, D, E and G are identical or different and represent hydrogen, halogen, nitro, cyano, hydroxyl, carboxyl, trifluoromethyl, trifluoromethoxy or straight-chain or branched alkyl, alkoxy or alkoxycarbonyl each having up to 5 carbon atoms,
R³ represents straight-chain or branched alkenyl having up to 6 carbon atoms, or
represents straight-chain or branched alkyl having up to 8 carbon atoms, which optionally carries an amino group which can be substituted by alkyl having up to 4 carbon atoms or by an amino protective group, or the alkyl is optionally identically or differently substituted one to 3 times by hydroxyl, cyano, halogen, azido, nitro, trifluoromethyl, carboxyl or phenyl which, for its part, can be identically or differently substituted up to 2 times by nitro, halogen, hydroxyl or by straight-chain or branched alkyl or alkoxy having up to 4 carbon atoms, or
R³ represents radicals of the formulae 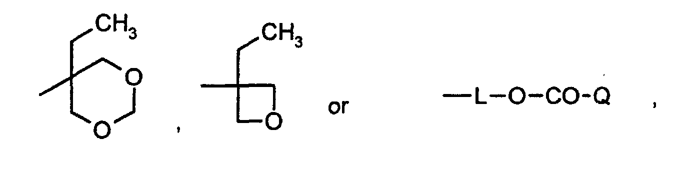 in which
L represents a straight-chain or branched alkanediyl group having up to 6 carbon atoms,
Q represents alkyl having up to 6 carbon atoms, which is optionally substituted by carboxyl, or represents radicals of the formulae 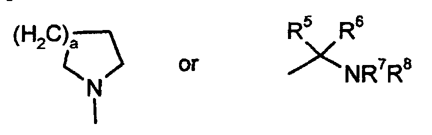 in which
a denotes the number 1 or 2,
R⁵ denotes hydrogen,
R⁶ denotes cycloalkyl having 3 to 8 carbon atoms or aryl having 6 to 10 carbon atoms or hydrogen, or
denotes straight-chain or branched alkyl having up to 8 carbon atoms,
where the alkyl is optionally substituted by cyano, methylthio, hydroxyl, mercapto, guanidyl or by a group of the formula -NR⁹R¹⁰ or -R¹¹-OC-,
in which
R⁹ and R¹⁰ independently of one another denote hydrogen, straight-chain or branched alkyl having up to 8 carbon atoms or phenyl,
and
R¹¹ denotes hydroxyl, benzyloxy, alkoxy having up to 6 carbon atoms or the abovementioned group -NR⁹R¹⁰,
or the alkyl is optionally substituted by cycloalkyl having 3 to 8 carbon atoms or by aryl having 6 to 10 carbon atoms which, for its part, is substituted by hydroxyl, halogen, nitro, alkoxy having up to 8 carbon atoms or by the group -NR⁹R¹⁰,
in which
R⁹ and R¹⁰ have the meaning indicated above,
or the alkyl is optionally substituted by a 5- to 6-membered nitrogen-containing heterocycle or by indolyl, in which the corresponding -NH functions are optionally substituted by alkyl having up to 6 carbon atoms or protected by an amino protective group,
R⁷ and R⁸ are identical or different and denote hydrogen or an amino protective group,
R⁴ represents hydrogen or a radical of the formula 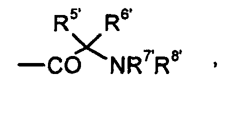 in which
R^{5'}, R^{6'}, R^{7'} and R^{8'} have the meaning of R⁵, R⁶, R⁷ and R⁸ indicated above and are identical to or different from these, and
X represents hydrogen,
and their stereoisomers, stereoisomer mixtures and salts ,
with the exception of N-[4-[[[5-(dimethylamino)-1-naphthalenyl]sulphonyl]-amino]phenyl]acetamide.

3. Sulphonamides of the general formula (I) according to Claim 1 or 2, in which
R³ represents straight-chain or branched alkenyl having up to 6 carbon atoms, or
represents straight-chain or branched alkyl having up to 8 carbon atoms, in which the alkyl carries an amino group which can be substituted by alkyl having up to 4 carbon atoms or by an amino protective group, or the alkyl is identically or differently substituted one to 3 times by hydroxyl, cyano, halogen, azido, nitro, trifluoromethyl, carboxyl or phenyl which, for its part, can be identically or differently substituted up to 2 times by nitro, halogen or hydroxyl or by straight-chain or branched alkyl or alkoxy having up to 4 carbon atoms, or
R³ represent radicals of the formulae 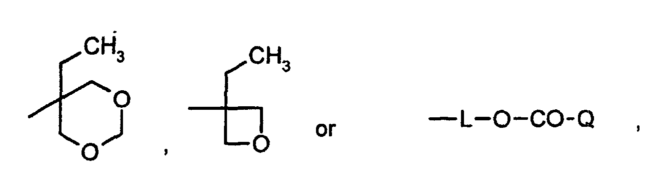 in which L and Q are as defined above.

4. Sulphonamides of the general formula (I) according to Claim 1 or 2, in which
R¹ and R² are identical or different and represent hydrogen, phenyl or straight-chain or branched alkyl or acyl each having up to 5 carbon atoms,
A, D, E and G are identical or different and represent hydrogen, fluorine, chlorine, bromine, nitro, cyano, hydroxyl or straight-chain or branched alkyl, alkoxy or alkoxycarbonyl each having up to 3 carbon atoms,
R³ represents straight-chain or branched alkenyl having up to 5 carbon atoms, or
represents straight-chain or branched alkyl having up to 7 carbon atoms which optionally carries an amino group which can be substituted by alkyl having up to 3 carbon atoms, tert- butyloxycarbonyl or benzyloxycarbonyl, or the alkyl is optionally identically or differently substituted one to 3 times by hydroxyl, cyano, fluorine, chlorine, azido, nitro, trifluoromethyl or phenyl which, for its part, can be identically or differently substituted up to 2 times by nitro, fluorine, chlorine or hydroxyl or by straight-chain or branched alkyl or alkoxy having up to 3 carbon atoms, or
R³ represent radicals of the formulae 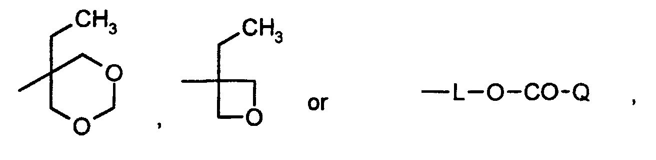 in which
L represents a straight-chain or branched alkanediyl group having up to 4 carbon atoms,
Q represents alkyl having up to 4 carbon atoms, which is optionally substituted by carboxyl, or
represents radicals of the formulae 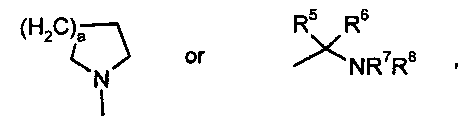 in which
a denotes the number 1 or 2,
R⁵ denotes hydrogen,
R⁶ denotes cyclopentyl, cyclohexyl, phenyl or hydrogen, or denotes straight-chain or branched alkyl having up to 6 carbon atoms,
where the alkyl can optionally be substituted by cyano, methylthio, hydroxyl, mercapto, guanidyl, amino, carboxyl or H₂N-CO-,
or the alkyl is substituted by cyclohexyl, naphthyl or phenyl which, for its part, can be substituted by fluorine, hydroxyl, nitro or alkoxy having up to 4 carbon atoms,
or the alkyl is substituted by indolyl, imidazolyl, pyridyl, triazolyl or pyrazolyl, where the corresponding -NH functions are optionally substituted by alkyl having up to 4 carbon atoms or protected by tert-butyloxycarbonyl or benzyloxycarbonyl,
R⁷ and R⁸ are identical or different and denote hydrogen, tert- butyloxycarbonyl or benzyloxycarbonyl,
R⁴ represents hydrogen or a radical of the formula 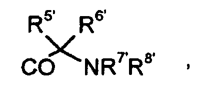 in which
R^{5'}, R^{6'}, R^{7'} and R^{8'} have the meaning of R⁵, R⁶, R⁷ and R⁸ indicated above and are identical to or different from these,
and their stereoisomers, stereoisomer mixtures and salts,
with the exception of N-[4-[[[5-(dimethylamino)-1-naphthalenyl]sulphonyl]-amino]phenyl]acetamide.

5. Sulphonamides of the general formula (I) according to Claim 4, in which
R³ represents straight-chain or branched alkenyl having up to 5 carbon atoms, or
represents straight-chain or branched alkyl having up to 7 carbon atoms, in which the alkyl carries an amino group which can be substituted by alkyl having up to 3 carbon atoms, tert-butyloxycarbonyl or benzyloxycarbonyl, or the alkyl is identically or differently substituted one to 3 times by hydroxyl, cyano, fluorine, chlorine, azido, nitro, trifluoromethyl or phenyl which, for its part, can be identically or differently substituted up to 2 times by nitro, fluorine, chlorine or hydroxyl or by straight-chain or branched alkyl or alkoxy having up to 3 carbon atoms, or
R³ represents radicals of the formulae 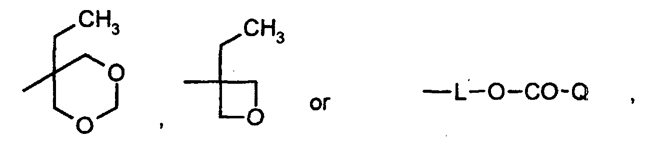 in which L and Q are as defined above.

6. Sulphonamides of the general formula (I) according to Claim 1, 2 or 4, in which
R¹ and R² are identical or different and represent hydrogen, phenyl or straight-chain or branched alkyl or acyl each having up to 4 carbon atoms,
A, D, E and G are identical or different and represent hydrogen, fluorine, chlorine, bromine, hydroxyl, methyl or methoxy,
R³ represents straight-chain or branched alkenyl having up to 4 carbon atoms, or
represents straight-chain or branched alkyl having up to 5 carbon atoms, which optionally carries an amino group which can be substituted by tert-butyloxycarbonyl or benzyloxycarbonyl, or which is optionally identically or differently substituted one to 3 times by hydroxyl, cyano, fluorine, chlorine, nitro, azido, trifluoromethyl or phenyl which, for its part, can be identically or differently substituted up to 2 times by nitro, fluorine, chlorine, hydroxyl, methyl, ethyl, methoxy or ethoxy, or
R³ represents radicals of the formulae 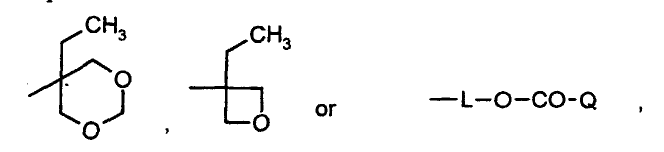 in which
L represents a straight-chain or branched alkanediyl group having up to 4 carbon atoms,
Q represents alkyl having up to 3 carbon atoms, which is optionally substituted by carboxyl, or
represents a radical of the formula 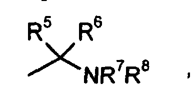 in which
R⁵ denotes hydrogen,
R⁶ denotes cyclopentyl, cyclohexyl or hydrogen, or denotes straight-chain or branched alkyl having up to 4 carbon atoms,
where the alkyl can optionally be substituted by cyano, methylthio, hydroxyl, mercapto, guanidyl, amino, carboxyl or H₂N-CO-,
or the alkyl is substituted by cyclohexyl, naphthyl or phenyl which, for its part, can be substituted by fluorine, chlorine or alkoxy having up to 4 carbon atoms,
or the alkyl is substituted by indolyl, imidazolyl, triazolyl, pyridyl or pyrazolyl, where the corresponding -NH functions are optionally substituted by methyl or protected by benzyloxymethylene or tert-butyloxy-carbonyl (BOC),
R⁷ and R⁸ are identical or different and denote hydrogen or tert-butyloxycarbonyl,
R⁴ represents hydrogen or a radical of the formula 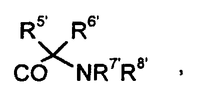 in which
R^{5'}, R^{6'}, R^{7'} and R^{8'} have the meaning of R⁵, R⁶, R⁷ and R⁸ indicated above and are identical to or different from these,
and their stereoisomers, stereoisomer mixtures and salts,
with the exception of N-[4-[[[5-(dimethylamino)-1-naphthalenyl)sulfonyl]-amino]phenyl]acetamide.

7. Sulphonamides of the general formula (I) according to any one of Claims 1 to 6, in which
R³ represents straight-chain or branched alkenyl having up to 4 carbon atoms, or
represents straight-chain or branched alkyl having up to 5 carbon atoms, in which the alkyl carries an amino group which can be substituted by tert-butyloxycarbonyl or benzyloxycarbonyl, or the alkyl is identically or differently substituted one to 3 times by hydroxyl, cyano, fluorine, chlorine, nitro, azido, trifluoromethyl or phenyl which, for its part, can be identically or differently substituted up to 2 times by nitro, fluorine, chlorine, hydroxyl, methyl; ethyl, methoxy or ethoxy, or
R³ represents radicals of the formulae 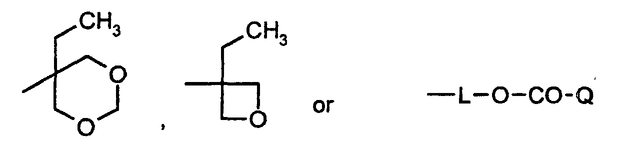
in which L or Q are as defined above,
and their stereoisomers, stereoisomeric mixtures and salts.

8. Sulphonamides of the general formula (I) according to any one of Claims 1 to 7, in which
R¹ and R² represent straight-chain or branched alkyl having up to 4 carbon atoms,
A, D, E and G represent hydrogen,
R³ represents straight-chain or branched alkyl having up to 5 carbon atoms, which is substituted by hydroxyl, or
R³ represents a radical of the formula
-L-O-CO-Q
in which
L represents a straight-chain or branched alkanediyl group having up to 4 carbon atoms,
Q represents a radical of the formula 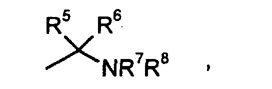 in which
R⁵ and R⁶ denote hydrogen, and
R⁷ and R⁸ denote hydrogen, and
R⁴ represents hydrogen
and their stereoisomers, stereoisomeric mixtures and salts.

9. Sulphonamides of the general formula (I) according to any of Claims 1 to 8, which are selected from the group of the following compounds : 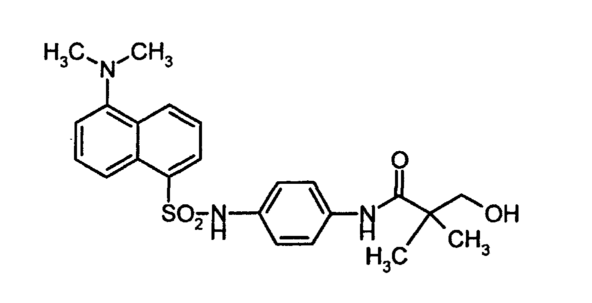 and

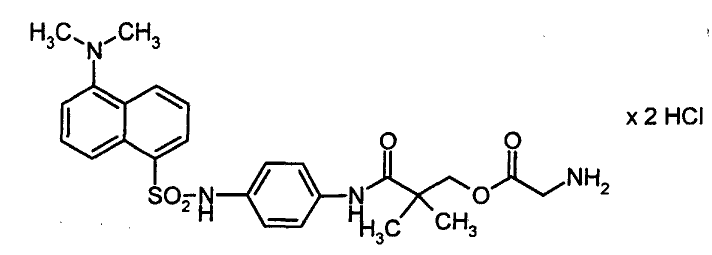

10. Process for the preparation of compounds of the general formula (I), **characterized in that**
[A] compounds of the general formula (II) 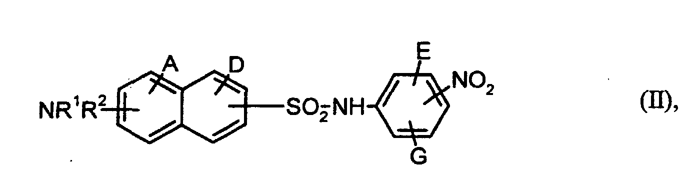 in which
A, D, E, G, R¹ and R² have the meaning indicated above,
are first converted by catalytic hydrogenation on palladium/C or by reduction with SnCl₂ in inert solvents into the compounds of the general formula (III) 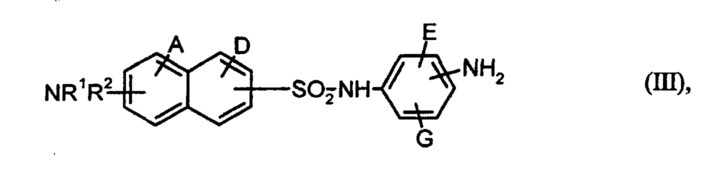 in which
A, D, E, G, R¹ and R² have the meaning indicated above,
and these are finally reacted with compounds of the general formula (IV)
T-CO-R³ (IV)
in which
R³ has the meaning indicated above
and
T represents hydroxyl or halogen, preferably chlorine,
in inert solvents, if appropriate in the presence of a base and/or of an auxiliary,
or
[B] compounds of the general formula (V) 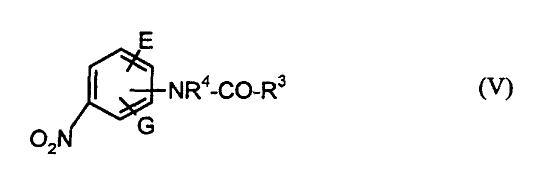 in which
E, G, R³ and R⁴ have the meaning indicated above,
are first converted as described under [A] by hydrogenation on Pd/C or by reduction with SnCl₂ in inert solvents into the compounds of the general formula (VI) 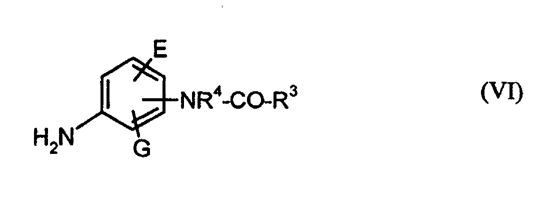 in which
E, G, R³ and R⁴ have the meaning indicated above,
and these are finally reacted with compounds of the general formula (VII) 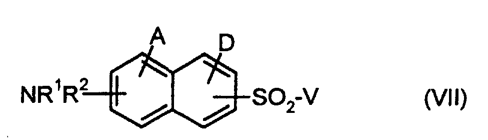 in which
A, D, R¹ and R² have the meaning indicated above,
and
V represents halogen, preferably chlorine,
in inert solvents, if appropriate in the presence of a base and/or of an auxiliary,
or
[C] if R³ and/or R³' represent a radical of the formula -L-O-CO-Q, compounds of the general formula (Ia) 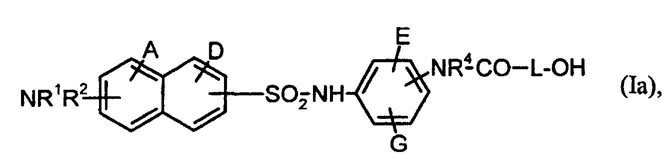 in which
R¹, R², R⁴, A, D, E, G and L have the meaning indicated above
are reacted with amino acid residues of the general formula (VIII)
HO-CO-Q' (VIII)
in which
Q' has the meaning of Q indicated above, where one of the terminal radicals on the nitrogen, mentioned there, represents one of the abovementioned protective groups, preferably tert-butyloxycarbonyl,
if appropriate with activation of the carboxylic acid according to customary methods, in inert solvents and in the presence of a base and of an auxiliary,
and finally the protective group is removed according to the methods customary in peptide chemistry,
and in the case in which X, R⁴ ≠ H is reacted with 2 or more equivalents of the compounds of the general formula (VIII),
if appropriate the stereoisomers are separated according to methods known per se and if appropriate the free bases are converted into the salts or the salts are converted into the free bases.

11. Sulphonamides of the general formula (I) according to any one of Claims 1 to 9 for the prophylaxis or treatment of diseases.

12. Use of sulphonamides of the general formula (I) according to any one of Claims 1 to 9 for the production of medicaments.

13. Use according to Claim 12, in which the medicaments are for the control of viral disorders.

14. Use according to Claim 12 or 13, in which the medicaments are for the control of cytomegalovirus.

15. Medicaments comprising sulphonamides of the general formula (I) according to any one of Claims 1 to 9.

## Revendications

1. Sulfonamides de formule générale (I) 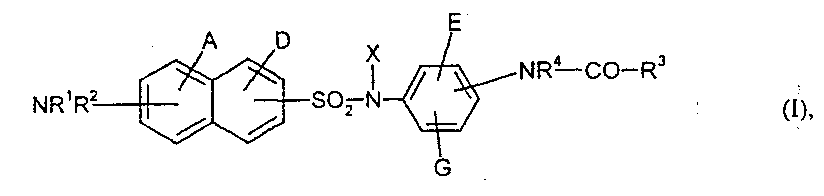 dans laquelle
R¹ et R² sont identiques ou différents et représentent l'hydrogène, un reste formyle, un reste phényle ou benzyle pouvant éventuellement être substitué par un à trois atomes d'halogène, ou un reste alkyle ou acyle linéaire ou ramifié ayant jusqu'à 6 atomes de carbone, le reste alkyle ou acyle pouvant éventuellement être substitué par un à trois substituants choisis entre halogène et hydroxy,
A, D, E et G sont identiques ou différents et représentent l'hydrogène, un halogène, un reste nitro, cyano, hydroxy, carboxyle, trifluorométhyle, trifluorométhoxy ou un reste alkyle, alkoxy ou alkoxycarbonyle linéaire ou ramifié ayant chacun jusqu'à 5 atomes de carbone,
R³ est un reste alcényle linéaire ou ramifié ayant jusqu'à 6 atomes de carbone, ou bien
un reste alkyle linéaire ou ramifié ayant jusqu'à 8 atomes de carbone, qui porte éventuellement un groupe amino qui peut être substitué par un groupe alkyle ayant jusqu'à 4 atomes de carbone ou par un groupe protégeant la fonction amino, ou bien le reste alkyle est substitué le cas échéant une à trois fois identiques ou différentes par un radical hydroxy, cyano, halogéno, azido, nitro, trifluorométhyle, carboxyle ou par un radical phényle qui peut lui-même porter jusqu'à deux substituants, identiques ou différents, nitro, halogéno, hydroxy, ou bien alkyle ou alkoxy linéaire ou ramifié ayant jusqu'à 4 atomes de carbone,
ou bien
R³ représente des restes de formules 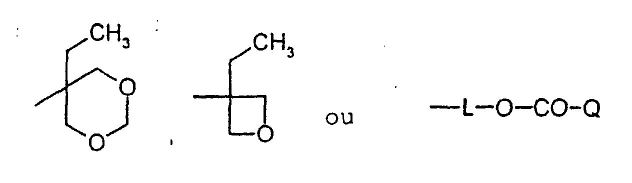 dans lesquelles
L est un groupe alcanediyle linéaire ou ramifié ayant jusqu'à 6 atomes de carbone,
Q est un reste alkyle ayant jusqu'à 6 atomes de carbone, qui est éventuellement substitué par un radical carboxyle, ou bien
des restes de formules 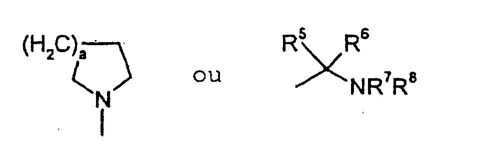 dans lesquelles
a représente le nombre 1 ou 2,
R⁵ représente l'hydrogène,
R⁶ représente un reste cycloalkyle ayant 3 à 8 atomes de carbone ou un reste aryle ayant 6 à 10 atomes de carbone ou l'hydrogène, ou bien
un reste alkyle linéaire ou ramifié ayant jusqu'à 8 atomes de carbone,
le reste alkyle étant éventuellement substitué par un radical cyano, méthylthio, hydroxy, mercapto, guanidyle ou par un groupe de formule -NR⁹R¹⁰ ou -R¹¹-OC-, dans lequel
R⁹ et R¹⁰ représentent indépendamment l'un de l'autre l'hydrogène, un reste alkyle linéaire ou ramifié ayant jusqu'à 8 atomes de carbone ou un reste phényle,
et
R¹¹ est un reste hydroxy, benzyloxy, alkoxy ayant jusqu'à 6 atomes de carbone ou le groupe -NR⁹R¹⁰ indiqué ci-dessus,
ou bien le reste alkyle est éventuellement substitué par un groupe cycloalkyle ayant 3 à 8 atomes de carbone ou par un groupe aryle ayant 6 à 10 atomes de carbone, qui est substitué quant à lui par un radical hydroxy, halogéno, nitro, alkoxy ayant jusqu'à 8 atomes de carbone ou par le groupe NR⁹R¹⁰,
dans lequel
R⁹ et R¹⁰ ont la définition indiquée ci-dessus,
ou bien le reste alkyle est éventuellement substitué par un hétérocycle pentagonal ou hexagonal contenant de l'azote ou par un reste indolyle, les fonctions -NH- correspondantes étant éventuellement substituées par un radical alkyle ayant jusqu'à 6 atomes de carbone ou par un groupe protégeant la fonction amino,
R⁷ et R⁸ sont identiques ou différents et représentent l'hydrogène ou un groupe protégeant la fonction amino,
R⁴ représente l'hydrogène ou un reste de formule 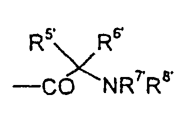 dans laquelle
R^{5'}, R^{6'}, R^{7'} et R^{8'} ont la définition indiquée ci-dessus pour R⁵, R⁶, R⁷ et R⁸ et y sont identiques ou en sont différents,
X a la définition indiquée ci-dessus pour R⁴ et peut être identique à cette définition ou peut en être différent,
et leurs stéréo-isomères, leurs mélanges de stéréo-isomères et leurs sels,
à l'exception du N-[4-[[[5-(diméthylamino)-1-naphtalényl]-sulfonyl]amino]phényl]-acétamide.

2. Sulfonamides de formule générale (I) suivant la revendication 1, formule dans laquelle
R¹ et R² sont identiques ou différents et représentent l'hydrogène, un reste phényle ou un reste alkyle ou acyle linéaire ou ramifié ayant chacun jusqu'à 6 atomes de carbone,
A, D, E et G sont identiques ou différents et représentent l'hydrogène, un halogène, un reste nitro, cyano, hydroxy, carboxyle, trifluorométhyle, trifluorométhoxy ou un reste alkyle, alkoxy ou alkoxycarbonyle linéaire ou ramifié ayant chacun jusqu'à 5 atomes de carbone,
R³ est un reste alcényle linéaire ou ramifié ayant jusqu'à 6 atomes de carbone, ou bien
un reste alkyle linéaire ou ramifié ayant jusqu'à 8 atomes de carbone, qui porte éventuellement un groupe amino qui peut être substitué par un radical alkyle ayant jusqu'à 4 atomes de carbone ou par un groupe protégeant la fonction amino, ou bien le reste alkyle est éventuellement substitué une à trois fois identiques ou différentes par un radical hydroxy, cyano, halogéno, azido, nitro, trifluorométhyle, carboxyle ou par un groupe phényle qui peut lui-même être substitué jusqu'à deux fois identiques ou différentes par un radical nitro, halogéno, hydroxy, ou bien par un radical alkyle ou alkoxy linéaire ou ramifié ayant jusqu'à 4 atomes de carbone,
ou bien
R³ représente des restes de formules 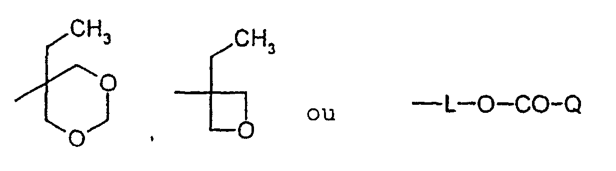 dans lesquelles
L est un groupe alcanediyle linéaire ou ramifié ayant jusqu'à 6 atomes de carbone,
Q est un reste alkyle ayant jusqu'à 6 atomes de carbone, qui est éventuellement substitué par un radical carboxyle, ou bien
des restes de formules 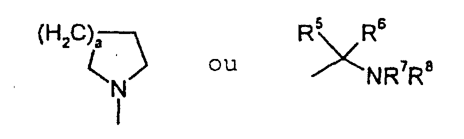 dans lesquelles
a représente le nombre 1 ou 2,
R⁵ représente l'hydrogène,
R⁶ représente un reste cycloalkyle ayant 3 à 8 atomes de carbone ou un reste aryle ayant 6 à 10 atomes de carbone ou l'hydrogène, ou bien
un reste alkyle linéaire ou ramifié ayant jusqu'à 8 atomes de carbone,
le reste alkyle pouvant éventuellement être substitué par un radical cyano, méthylthio, hydroxy, mercapto, guanidyle
ou par un groupe de formule -NR⁹R¹⁰ ou -R¹¹-OC-,
dans laquelle
R⁹ et R¹⁰ représentent indépendamment l'un de l'autre l'hydrogène, un reste alkyle linéaire ou ramifié ayant jusqu'à 8 atomes de carbone ou un reste phényle,
et
R¹¹ est un reste hydroxy, benzyloxy, alkoxy ayant jusqu'à 6 atomes de carbone ou le groupe -NR⁹R¹⁰ indiqué ci-dessus,
ou bien le reste alkyle porte éventuellement un substituant cycloalkyle ayant 3 à 8 atomes de carbone ou aryle ayant 6 à 10 atomes de carbone, qui peut lui-même être substitué par un radical hydroxy, halogéno, nitro, alkoxy ayant jusqu'à 8 atomes de carbone ou par le groupe -NR⁹R¹⁰,
dans lequel
R⁹ et R¹⁰ ont la définition indiquée ci-dessus,
ou bien le reste alkyle est éventuellement substitué par un hétérocycle azoté pentagonal ou hexagonal ou par un reste indolyle, les fonctions -NH- correspondantes étant éventuellement substituées par un radical alkyle ayant jusqu'à 6 atomes de carbone ou par un groupe protégeant la fonction amino,
R⁷ et R⁸ sont identiques ou différents et représentent l'hydrogène ou un groupe protégeant la fonction amino, R⁴ représente l'hydrogène ou un reste de formule 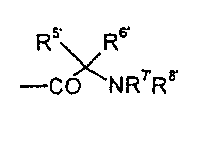 dans laquelle
R⁵'_{,} R⁶', R⁷' et R⁸' ont la définition indiquée ci-dessus pour R⁵, R⁶, R⁷ et R⁸ et y sont identiques ou en sont différents, et
X représente l'hydrogène,
et leurs stéréo-isomères, leurs mélanges de stéréo-isomères et leurs sels,
à l'exception du N- [4- [[[5- <diméthylamino> -1-naphtalényl]sulfonyl]amino]phényl]-acétamide.

3. Sulfonamides de formule générale (I) suivant la revendication 1 ou 2, formule dans laquelle
R³ est un reste alcényle linéaire ou ramifié ayant jusqu'à 6 atomes de carbone, ou bien
un reste alkyle linéaire ou ramifié ayant jusqu'à 8 atomes de carbone, ce reste alkyle portant un groupe amino qui peut être substitué par un radical alkyle ayant jusqu'à 4 atomes de carbone ou par un groupe protégeant la fonction amino, ou bien le reste alkyle porte 1 à 3 substituants, identiques ou différents, hydroxy, cyano, halogéno, azido, nitro, trifluorométhyle, carboxyle ou phényle qui peut lui-même porter 1 ou 2 substituants, identiques ou différents, nitro, halogéno, hydroxy, ou bien alkyle ou alkoxy linéaire ou ramifié ayant jusqu'à 4 atomes de carbone,
ou bien
R³ représente des restes de formules 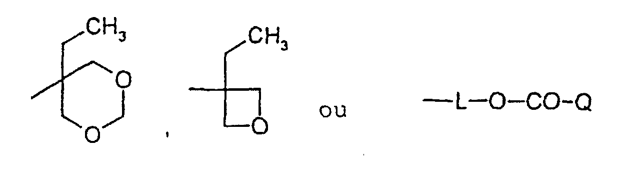 dans lesquelles L et Q sont tels que définis ci-dessus.

4. Sulfonamides de formule générale (I) suivant la revendication 1 ou 2, formule dans laquelle
R¹ et R² sont identiques ou différents et représentent l'hydrogène, un reste phényle ou un reste alkyle ou acyle linéaire ou ramifié ayant chacun jusqu' à 5 atomes de carbone,
A, D, E et G sont identiques ou différents et représentent l'hydrogène, le fluor, le chlore, le brome, un reste nitro, cyano, hydroxy ou un reste alkyle, alkoxy ou alkoxycarbonyle linéaire ou ramifié ayant chacun jusqu'à 3 atomes de carbone,
R³ est un reste alcényle linéaire ou ramifié ayant jusqu'à 5 atomes de carbone, ou bien
un reste alkyle linéaire ou ramifié ayant jusqu'à 7 atomes de carbone, qui porte éventuellement un groupe amino qui peut être substitué par un reste alkyle ayant jusqu'à 3 atomes de carbone tertiobutoxycarbonyle ou benzyloxycarbonyle, ou bien le reste alkyle est éventuellement substitué une à trois fois identiques ou différents par un radical hydroxy, cyano, fluoro, chloro, azido, nitro trifluorométhyle ou un radical phényle qui peut lui-même porter jusqu'à deux radicaux, identiques ou différents, nitro, fluoro, chloro, hydroxy, ou bien alkyle ou alkoxy linéaire où ramifié ayant jusqu'à 3 atomes de carbone,
ou bien
R³ représente les restes de formules 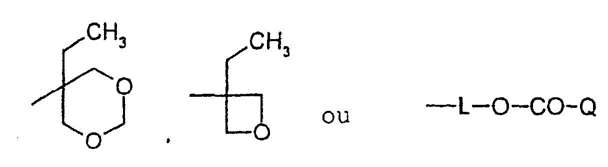 dans lesquelles
L est un groupe alcanediyle linéaire ou ramifié ayant jusqu'à 4 atomes de carbone,
Q est un reste alkyle ayant jusqu'à 4 atomes de carbone, qui est éventuellement substitué par un radical carboxyle, ou bien
des restes de formules 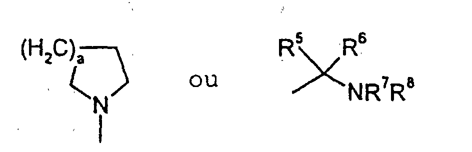 dans lesquelles
a représente le nombre 1 ou 2,
R⁵ est l'hydrogène,
R⁶ est un reste cyclopentyle, cyclohexyle, phényle ou l'hydrogène, ou bien
un reste alkyle linéaire ou ramifié ayant jusqu'à 6 atomes de carbone,
le reste alkyle pouvant éventuellement être substitué par un radical cyano, méthylthio, hydroxy, mercapto, guanidyle, amino, carboxy ou H₂N-CO-,
ou bien le reste alkyle est substitué par un radical cyclohexyle, naphtyle ou phényle qui peut lui-même être substitué par du fluor, un radical hydroxy, nitro ou un radical alkoxy ayant jusqu'à 4 atomes de carbone,
ou bien le reste alkyle est substitué par un radical indolyle, imidazolyle, pyridyle, triazolyle ou pyrazolyle, les fonctions -NH- correspondantes étant éventuellement substituées par un reste alkyle ayant jusqu'à 4 atomes de carbone ou par un reste tertiobutyloxycarbonyle ou benzyloxycarbonyle,
R⁷ et R⁸ sont identiques ou différents et représentent l'hydrogène, un reste tertiobutyloxycarbonyle ou benzyloxycarbonyle,
R⁴ est l'hydrogène ou un reste de formule 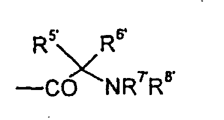 dans laquelle
R⁵', R⁶', R⁷' et R⁸' ont la définition indiquée ci-dessus pour R⁵, R⁶, R⁷ et R⁸ et y sont identiques ou en sont différents,
et leurs stéréo-isomères, leurs mélanges de stéréo-isomères et leurs sels,
à l'exception du N-(4- [[[5-(diméthylamino)-1-naphtalényl]sulfonyl]amino]phényl]-acétamide.

5. Sulfonamides de formule générale (I) suivant la revendication 4, formule dans laquelle
R³ est un reste alcényle linéaire ou ramifié ayant jusqu'à 5 atomes de carbone, ou bien
un reste alkyle linéaire ou ramifié ayant jusqu'à 7 atomes de carbone, ce reste alkyle portant un groupe amino qui peut être substitué par un radical alkyle ayant jusqu'à 3 atomes de carbone, tertiobutyloxycarbonyle ou benzyloxycarbonyle, ou bien le reste alkyle porte 1 à 3 substituants, identiques ou différents, hydroxy, cyano, fluoro, chloro, azido, nitro, trifluorométhyle ou phényle qui peut lui-même porter 1 ou 2 substituants, identiques ou différents, nitro, fluoro, chloro, hydroxy, ou bien alkyle ou alkoxy linéaire ou ramifié ayant jusqu'à 3 atomes de carbone, ou bien R³
représente des restes de formules 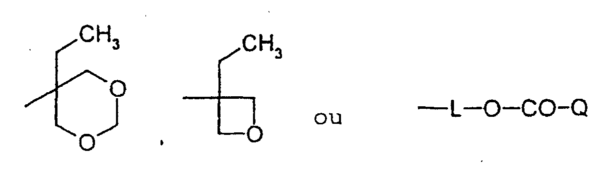 dans lesquelles L et Q sont tels que définis ci-dessus.

6. Sulfonamides de formule générale (I) suivant la revendication 1, 2 ou 4, formule dans laquelle
R¹ et R² sont identiques ou différents et représentent l'hydrogène, un reste phényle ou un reste alkyle ou acyle linéaire ou ramifié ayant chacun jusqu'à 4 atomes de carbone,
A, D, E et G sont identiques ou différents et représentent l'hydrogène, le fluor, le chlore, le brome, un reste hydroxy, méthyle ou méthoxy,
R³ est un reste alcényle linéaire ou ramifié ayant jusqu'à 4 atomes de carbone, ou bien
un reste alkyle linéaire ou ramifié ayant jusqu'à 5 atomes de carbone, qui porte éventuellement un groupe amino qui peut être substitué par un radical tertiobutyloxycarbonyle ou benzyloxycarbonyle, ou qui est éventuellement substitué une à trois fois identiques ou différentes par un radical hydroxy, cyano; fluoro, chloro, nitro, azido, trifluorométhyle ou phényle qui peut lui-même être substitué jusqu'à deux fois identiques ou différentes par un radical nitro, fluoro, chloro, hydroxy, méthyle, éthyle, méthoxy ou éthoxy, ou bien
R³ représente des restes de formules 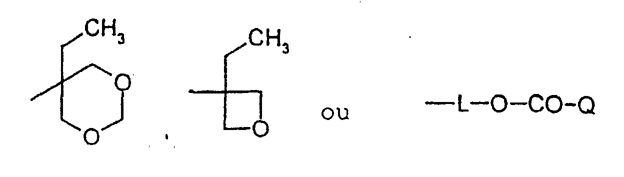 dans lesquelles
L est un groupe alcanediyle linéaire ou ramifié ayant jusqu'à 4 atomes de carbone,
Q est un reste alkyle ayant jusqu'à 3 atomes de carbone, qui est éventuellement substitué par un radical carboxyle, ou bien
un reste de formule 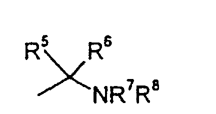 dans laquelle
R⁵ représente l'hydrogène,
R⁶ est un reste cyclopentyle, cyclohexyle ou l'hydrogène
ou bien
un reste alkyle linéaire ou ramifié ayant jusqu'à 4 atomes de carbone,
le reste alkyle pouvant éventuellement être substitué par un radical cyano, méthylthio, hydroxy, mercapto, guanidyle, amino, carboxy ou H₂N-CO-,
ou bien le reste alkyle est substitué par un radical cyclohexyle, naphtyle ou phényle qui peut lui-même être substitué par du fluor, du chlore ou un radical alkoxy ayant jusqu'à 4 atomes de carbone,
ou bien le reste alkyle est substitué par un radical indolyle, imidazolyle, triazolyle, pyridyle ou pyrazolyle, les fonctions -NH- correspondantes étant éventuellement substituées par un radical méthyle ou protégées par un reste benzyloxyméthylène ou tertiobutyloxycarbonyle (BOC),
R⁷ et R⁸ sont identiques ou différents et représentent l'hydrogène ou un reste tertiobutyloxycarbonyle,
R⁴ est l'hydrogène ou un reste de formule 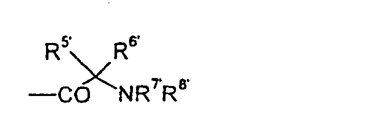 dans laquelle
R^{5'}, R^{6'}, R^{7'} et R^{8'} ont la définition indiquée ci-dessus pour R⁵, R⁶, R⁷ et R⁸ et y sont identiques ou en sont différents,
et leurs stéréo-isomères, leurs mélanges de stéréo-isomères et leurs sels,
à l'exception du N-[4-[[[5-(diméthylamino)-1-naphtalényl]sulfonyl]amino]phényl]-acétamide.

7. Sulfonamides de formule générale (I) suivant l'une quelconque des revendications 1 à 6, formule dans laquelle
R³ est un reste alcényle linéaire ou ramifié ayant jusqu'à 4 atomes de carbone, ou bien
un reste alkyle linéaire ou ramifié ayant jusqu'à 5 atomes de carbone, le reste alkyle portant un groupe amino qui peut être substitué par un radical tertiobutyloxycarbonyle ou benzyloxycarbonyle, ou bien le reste alkyle porte 1 à 3 substituants, identiques ou différents, hydroxy, cyano, fluoro, chloro, nitro, azido, trifluorométhyle ou phényle qui peut lui-même porter jusqu'à 2 substituants, identiques ou différents, nitro, fluoro, chloro, hydroxy, méthyle, éthyle, méthoxy ou éthoxy, ou bien
R³ représente des restes de formules 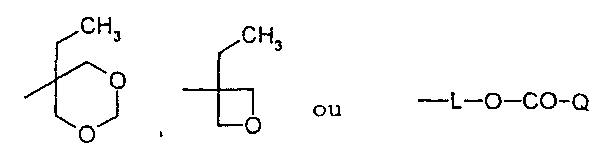
dans lesquelles L ou Q a la définition indiquée ci-dessus,
et leurs stéréo-isomères, leurs mélanges de stéréo-isomères et leurs sels.

8. Sulfonamides de formule générale (I) suivant l'une quelconque des revendications 1 à 7, formule dans laquelle
R¹ et R² représentent un reste alkyle linéaire ou ramifié ayant jusqu'à 4 atomes de carbone,
A, D, E et G représentent l'hydrogène,
R³ est un reste alkyle linéaire ou ramifié ayant jusqu'à 5 atomes de carbone, qui est substitué par un radical hydroxy, ou bien
R³ est un reste alkyle de formule
-L-O-CO-Q
dans laquelle
L est un groupe alcanediyle linéaire ou ramifié ayant jusqu'à 4 atomes de carbone,
Q est un reste de formule 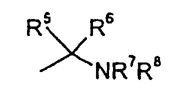
dans laquelle
R⁵ et R⁶ représentent l'hydrogène, et
R⁷ et R⁸ représentent l'hydrogène, et
R⁴ est l'hydrogène,
et leurs stéréo-isomères, leurs mélanges de stéréo-isomères et leurs sels.

9. Sulfonamides de formule générale (I) suivant l'une quelconque des revendications 1 à 8, qui sont choisis dans le groupe des composés suivants :

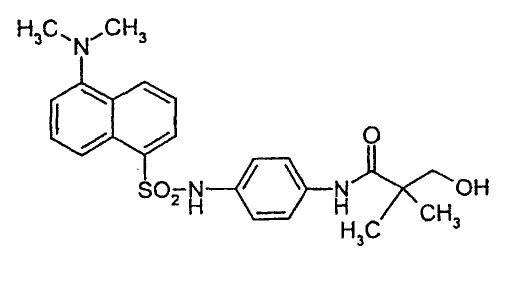

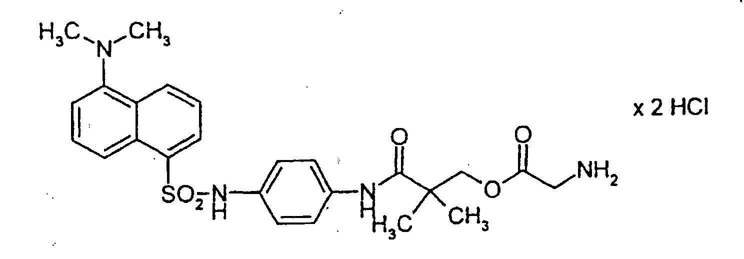

10. Procédé de production de composés de formule générale (I) **caractérisé en ce que** :
[A] des composés de formule générale (II) 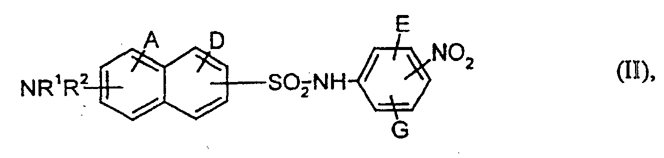 dans laquelle
A, D, E, G, R¹ et R² ont la définition indiquée ci-dessus, sont tout d'abord transformés par hydrogénation catalytique sur du palladium fixé sur du carbone ou par réduction avec SnCl₂ dans des solvants inertes, en les composés de formule générale (III) 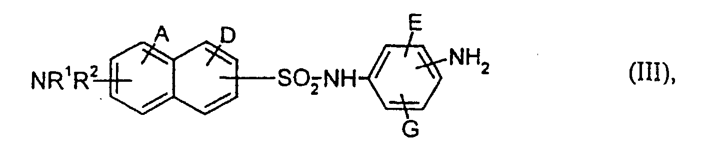 dans laquelle
A, D, E, G, R¹ et R² ont la définition indiquée ci-dessus, qui sont ensuite amenés à réagir avec des composés de formule générale (IV)
T-CO-R¹ (IV)
dans laquelle
R³ a la définition indiquée ci-dessus,
et
T est un reste hydroxy, un halogène, de préférence le chlore,
dans des solvants inertes, éventuellement en présence d'une base et/ou d'une substance auxiliaire,
ou bien
[B] des composés de formule générale (V) 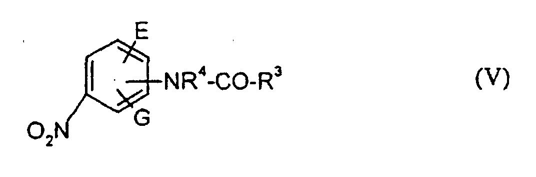 dans laquelle
E, G, R³ et R⁴ ont la définition indiquée ci-dessus, sont tout d'abord transformés de la manière décrite en [A] par hydrogénation sur du palladium fixé sur du carbone ou par réduction avec SnCl₂ dans des solvants inertes, en les composés de formule générale (VI) 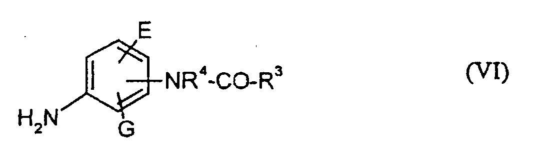 dans laquelle
E, G, R³ et R⁴ ont la définition indiquée ci-dessus, qui sont ensuite amenés à réagir avec des composés de formule générale (VII) 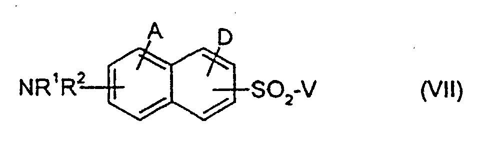
dans laquelle
A, et D, R¹ et R² ont la définition indiquée ci-dessus,
V représente un halogène, de préférence le chlore,
dans des solvants inertes, éventuellement en présence d'une base et/ou d'une substance auxiliaire,
ou bien
[C] au cas où R³ et/ou R⁴ représentent un reste de formule -L-O-CO-Q, des composés de formule générale (Ia) 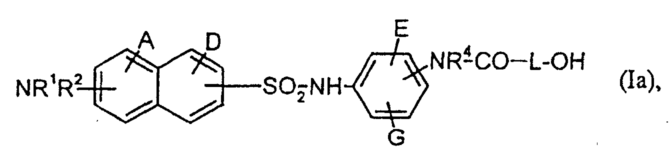
dans laquelle
R¹, R², R⁴, A, D, E, G et L ont la définition indiquée ci-dessus,
sont amenés à réagir avec des esters d'aminoacides de formule générale (VIII)
HO-CO-Q' (VIII)
dans laquelle
Q' a la définition indiquée ci-dessus pour Q, l'un des restes terminaux sur l'atome d'azote indiqués dans cette définition étant l'un des groupes protecteurs énumérés ci-dessus, de préférence le groupe tertio-butyloxycarbonyle,
le cas échéant avec activation de l'acide carboxylique selon des modes opératoires classiques, dans des solvants inertes et en présence d'une base et d'une substance auxiliaire,
après quoi le groupe protecteur est éliminé selon les modes opératoires d'emploi classique dans la chimie des peptides, et dans le cas X, R⁴ ≠ H, ils sont amenés à réagir avec deux ou plus de deux équivalents des composés de formule générale (VIII),
les stéréo-isomères sont éventuellement séparés selon des modes opératoires connus et les bases libres sont
transformées le cas échéant en sels, et les sels en bases libres.

11. Sulfonamides de formule générale (I) suivant l'une quelconque des revendications 1 à 9, destinés à la prophylaxie ou au traitement de maladies.

12. Utilisation de sulfonamides de formule générale (I) suivant l'une quelconque des revendications 1 à 9 pour la préparation de médicaments.

13. Utilisation suivant la revendication 12, dans laquelle les médicaments sont destinés à combattre des maladies virales.

14. Utilisation suivant la revendication 12 ou 13, dans laquelle les médicaments sont destinés à combattre le cytomégalovirus.

15. Médicament contenant des sulfonamides de formule générale (I) suivant l'une quelconque des revendications 1 à 9.
